(19) Europäisches Patentamt / European Patent Office / Office européen des brevets

(11) EP 2 315 760 B1

(12) # EUROPÄISCHE PATENTSCHRIFT

(45) Veröffentlichungstag und Bekanntmachung des Hinweises auf die Patenterteilung:
**06.03.2013 Patentblatt 2013/10**

(21) Anmeldenummer: **09780999.0**

(22) Anmeldetag: **23.07.2009**

(51) Int Cl.:
***C07D 401/06*** (2006.01) ***C07D 403/06*** (2006.01)
***A01N 43/60*** (2006.01)

(86) Internationale Anmeldenummer:
**PCT/EP2009/059518**

(87) Internationale Veröffentlichungsnummer:
**WO 2010/012649 (04.02.2010 Gazette 2010/05)**

(54) **PIPERAZINVERBINDUNGEN MIT HERBIZIDER WIRKUNG**

PIPERAZINE COMPOUNDS WITH HERBICIDAL EFFECT

COMPOSÉS PIPÉRAZINES AYANT UNE ACTIVITÉ HERBICIDE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **29.07.2008 EP 08161347**
**24.09.2008 EP 08164985**
**02.10.2008 EP 08165693**

(43) Veröffentlichungstag der Anmeldung:
**04.05.2011 Patentblatt 2011/18**

(73) Patentinhaber: **BASF SE**
**67056 Ludwigshafen (DE)**

(72) Erfinder:
- **PARRA RAPADO, Liliana**
  **77654 Offenburg (DE)**
- **HUPE, Eike**
  **68161 Mannheim (DE)**
- **WITSCHEL, Matthias**
  **67098 Bad Dürkheim (DE)**
- **SEITZ, Thomas**
  **68519 Viernheim (DE)**
- **SIMON, Anja**
  **69469 Weinheim (DE)**
- **REINHARD, Robert**
  **67117 Limburgerhof (DE)**
- **SIEVERNICH, Bernd**
  **67454 Haßloch (DE)**
- **GROßMANN, Klaus**
  **67141 Neuhofen (DE)**
- **EHRHARDT, Thomas**
  **67346 Speyer (DE)**
- **NEWTON, Trevor William**
  **67435 Neustadt (DE)**
- **STELZER, Frank**
  **68309 Mannheim (DE)**
- **QU, Tao**
  **67063 Ludwigshafen (DE)**
- **MOBERG, William Karl**
  **67433 Neustadt (DE)**
- **SONG, Dschun**
  **68167 Mannheim (DE)**
- **RACK, Michael**
  **69214 Eppelheim (DE)**
- **FRASSETTO, Timo**
  **68165 Mannheim (DE)**
- **KREUZ, Klaus**
  **79211 Denzlingen (DE)**
- **MAJOR, Julia**
  **68167 Mannheim (DE)**

(56) Entgegenhaltungen:
**WO-A-2007/077201 WO-A-2007/077247**

Bemerkungen:
Die Akte enthält technische Angaben, die nach dem Eingang der Anmeldung eingereicht wurden und die nicht in dieser Patentschrift enthalten sind.

EP 2 315 760 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Piperazinverbindungen der Formel I.1

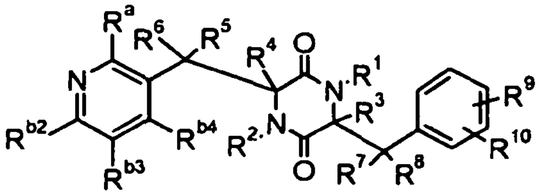

I.1

worin die Variablen folgende Bedeutung haben

$R^a$ Halogen, CN, $NO_2$, $C_1$-$C_4$-Alkyl, $C_3$-$C_6$-Cycloalkyl, $C_1$-$C_4$-Haloalkyl, $C_1$-$C_4$-Alkoxy, $C_1$-$C_4$-Thioalkyl, $C_1$-$C_4$-Haloalkoxy, $C_1$-$C_4$-Halogenthioalkyl, $S(O)_nR^y$, $C_2$-$C_6$-Alkenyl, $C_3$-$C_6$-Cycloalkenyl, $C_3$-$C_6$-Alkenyloxy, $C_3$-$C_6$-Thioalkenyl, $C_2$-$C_6$-Akinyl, $C_3$-$C_6$-Alkinyloxy, $C_3$-$C_6$-Thioalkinyl, $NR^AR^B$, Tri-$C_1$-$C_4$-alkylsilyl, Z-C(=O)-$R^{a1}$, Z-C(=S)-$R^{a1}$, Z-C(=N-$OR^A$)-$R^{a1}$, Z-C[=N(O)$R^A$]-$R^{a1}$, Z-P(=O)$(R^{a1})_2$, über C oder N gebundener 3- bis 7-gliedriger monocyclischer oder 9- oder 1 0-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen $R^{aa}$ und/oder $R^{a1}$ substituiert und/oder an einen einen weiteren gesättigten, ungesättigten oder aromatischen carbo- oder heterocyclischen Ring anneliert sein kann,

$R^y$ $C_1$-$C_4$-Akyl und $C_1$-$C_4$-Haloalkyl bedeutet und n für 0, 1 oder 2 steht;

$R^A$,$R^B$ unabhängig voneinander Wasserstoff, $C_1$-$C_6$-Alkyl, $C_3$-$C_6$-Alkenyl, $C_3$-$C_6$-Alkinyl, $C_3$-$C_6$-Cycloalkyl, $C_1$-$C_6$-Alkylcarbonyl, $C_3$-$C_6$-Cycloalkylcarbonyl, $C_3$-$C_6$-Alkenylcarbonyl, $C_3$-$C_6$-Cycloalkenylcarbonyl und $C_3$-$C_6$-Akinyl-carbonyl;

Z eine kovalente Bindung oder $C_1$-$C_4$-Alkylen;

$R^{a1}$ Wasserstoff, OH, $C_1$-$C_8$-Alkyl, $C_1$-$C_4$-Haloalkyl, $C_3$-$C_6$-Cycloalkyl, $C_2$-$C_8$-Alkenyl, $C_5$-$C_6$-Cycloalkenyl, $C_2$-$C_8$-Alkinyl, $C_1$-$C_6$-Alkoxy, $C_1$-$C_4$-Haloalkoxy, $C_3$-$C_8$-Alkenyloxy, $C_3$-$C_8$-Alkinyloxy, $NH_2$, $C_1$-$C_6$-Alkylamino, [Di-($C_1$-$C_{6)}$-alkyl]amino, $C_1$-$C_6$-Akoxyamino, $C_1$-$C_6$-Alkylsulfonylamino, $C_1$-$C_6$-Alkylaminosulfonylamino, [Di-($C_1$-$C_6$)-alkylamino]sulfonylamino, $C_3$-$C_6$-Alkenylamino, $C_3$-$C_6$-Alkinylamino, N-($C_2$-$C_6$-Akenyl)-($C_1$-$C_6$-alkyl)-amino, N-($C_2$-$C_6$-Alkinyl)-N-($C_1$-$C_6$-alkyl)-amino, N-($C_1$-$C_6$-Alkoxy)-N-($C_1$-$C_6$-alkyl)-amino, N-($C_2$-$C_6$-Alkenyl)-N-($C_1$-$C_6$-alkoxy)-amino, N-($C_2$-$C_6$-Alkinyl)-N-($C_1$-$C_6$-alkoxy)-amino, $C_1$-$C_6$-Alkylsulfonyl, Tri-$C_1$-$C_4$-alkylsilyl, Phenyl, Phenoxy, Phenylamino und 5- oder 6-gliedriger monocyclischer oder 9- oder 10-gliedriger bicyclischer Heterocyclus, enthaltend 1, 2, 3 oder 4 Heteroatome ausgewählt aus O, N und S, wobei die cyclischen Gruppen unsubstituiert oder durch 1, 2, 3 oder 4 Gruppen $R^{aa}$ substituiert sind, bedeutet;

$R^{aa}$ Halogen, CN, $NO_2$, $C_1$-$C_4$-Alkyl, $C_1$-$C_4$-Haloalkyl, $C_1$-$C_4$-Alkoxy, $C_1$-$C_4$-Haloalkoxy, Z-C(=O)-$R^{a1}$, Z-C(=S)-$R^{a1}$, Z-C(=N-$OR^A$)-$R^{a1}$, Z-C[=N(O)-$R^A$]-$R^{a1}$, Oxo (=O) und Tri-$C_1$-$C_4$-alkylsilyl;

wobei in Gruppen $R^a$ und deren Untersubstituenten die Kohlenstoffketten und/oder die cyclischen Gruppen 1, 2, 3 oder 4 Substituenten $R^{aa}$ und/oder $R^{a1}$ tragen können;

$R^{b2}$, $R^{b3}$ und $R^{b4}$ jeweils für Wasserstoff stehen;

$R^1$ Wasserstoff, $C_1$-$C_6$-Alkyl, $C_1$-$C_4$-Haloalkyl, $C_3$-$C_4$-Alkenyl, $C_3$-$C_4$-Alkinyl und C(=O)$R^{11}$,

$R^{11}$ Wasserstoff, $C_1$-$C_4$-Alkyl, $C_1$-$C_4$-Haloalkyl, $C_1$-$C_4$-Akoxy und $C_1$-$C_4$-Haloalkoxy;

$R^2$ $C_1$-$C_4$-Alkyl, $C_3$-$C_4$-Alkenyl und $C_3$-$C_4$-Alkinyl;

$R^3$ OH, $NH_2$, $C_1$-$C_4$-Alkyl, $C_3$-$C_6$-Cycloalkyl, $C_3$-$C_6$-Alkenyl, $C_3$-$C_6$-Alkinyl, $C_1$-$C_4$-Hydroxyalkyl, $C_1$-$C_4$-Cyanoalkyl, $C_1$-$C_4$-Haloalkyl, $C_1$-$C_4$-Alkoxy-$C_1$-$C_4$-alkyl und C(=O)$R^{11}$;

$R^4$ und $R^5$ gemeinsam für eine kovalente Bindung oder für Wasserstoff und $R^6$, $R^7$ und $R^8$ für Wasserstoff stehen

$R^9$,$R^{10}$ unabhängig voneinander Wasserstoff, Halogen, OH, Haloalkyl, $NR^AR^B$, $NR^AC(O)R^{91}$, CN, $NO_2$, $C_1$-$C_4$-Akyl, $C_1$-$C_4$-Haloalkyl, $C_2$-$C_4$-Alkenyl, $C_3$-$C_6$-Alkinyl, $C_1$-$C_4$-Alkoxy, $C_1$-$C_4$-Haloalkoxy, O-C(O)$R^{91}$, Phenoxy und Benzyloxy, wobei in Gruppen $R^9$ und $R^{10}$ die Kohlenstoffketten und/oder die cyclischen Gruppen 1, 2, 3 oder 4 Substituenten $R^{aa}$ tragen können;

$R^{91}$ $C_1$-$C_4$-Alkyl oder $NR^AR^B$;

sowie deren landwirtschaftlich geeignete Salze.

Außerdem betrifft die Erfindung Verfahren und Zwischenprodukte zur Herstellung der Piperazinverbindungen der Formel I.1 und deren landwirtschaftlich brauchbaren Salze, sie enthaltende Mittel und deren Verwendung als Herbizide, d.h. zur Bekämpfung von Schadpflanzen, sowie ein Verfahren zur Bekämpfung von unerwünschtem Pflanzenwuchs, bei dem man eine herbizid wirksame Menge mindestens einer Piperazinverbindung der Formel I.1 oder eines landwirtschaftlich brauchbaren Salzes von I.1 auf Pflanzen, deren Samen und/oder deren Lebensraum einwirken läßt.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden

Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Bei den von dem Pflanzenpathogen S. scabies produzierten Thaxtominen A und B (King R. R. et al., J. Agric. Food Chem. (1992) 40, 834-837) handelt es sich um Naturstoffe mit einem zentralen Piperazin-2,5-dion-Ring, der in der 3-Position einen 4-Nitro-indol-3-ylmethyl-Rest und in der 2-Position, einen gegebenenfalls durch OH substituierten Benzyl-Rest trägt. Aufgrund ihrer pflanzenschädigenden Wirkung wurde auch die Verwendungsmöglichkeit dieser Verbindungsklasse als Herbizide untersucht (King R. R. et al., J. Agric. Food Chem. (2001) 49, 2298-2301).

In WO 2007/077201 und WO 2007/077247 werden herbizide 2,5-Diketopiperazine beschrieben, die in den 3- und 6-positionen über Methylen- bzw. Methingruppen verknüpfte Phenyl-, bzw. Hetarylgruppen aufweisen.

Eine Aufgabe der vorliegenden Erfindung ist die Bereitstellung von Verbindungen mit herbizider Wirkung. Insbesondere sollen Verbindungen zur Verfügung gestellt werden, die eine hohe herbizide Wirkung, insbesondere bereits bei niedrigen Aufwandmengen, aufweisen und deren Verträglichkeit gegenüber Kulturpflanzen für eine kommerzielle Verwertung hinreichend ist.

Diese und weitere Aufgaben werden durch die eingangs definierten Verbindungen der Formel I.1 und durch ihre landwirtschaftlich geeigneten Salze gelöst.

Die erfindungsgemäßen Verbindungen können analog der in WO 2007/077201 und WO 2007/077247 beschriebenen Syntheserouten nach Standardverfahren der organischen Chemie hergestellt werden, beispielsweise einem Verfahren (im Folgenden Verfahren A) welches die folgenden Schritte umfasst:

Verfahren A

Verbindungen der Formel I mit $R^1 \neq$ Wasserstoff können bevorzugt dadurch hergestellt werden, dass man eine Piperazinverbindung der Formel I, worin $R^1$ für Wasserstoff steht, mit einem Alkylierungsmittel oder Acylierungsmittel, das den von Wasserstoff verschiedene Gruppe $R^1$ enthält, umsetzt (Verfahren A). Derartige Umsetzungen können analog bekannter Verfahren erfolgen, beispielsweise nach den von I.O. Donkor et al., Bioorg. Med. Chem. Lett. 11 (19) (2001), 2647-2649, B.B. Snider et al., Tetrahedron 57 (16) (2001), 3301-3307, I. Yasuhiro et al., J. Am. Chem. Soc. 124 (47) (2002), 14017-14019, oder M. Falomi et al., Europ. J. Org. Chem. (8) (2000), 1669-1675 beschriebenen Methoden

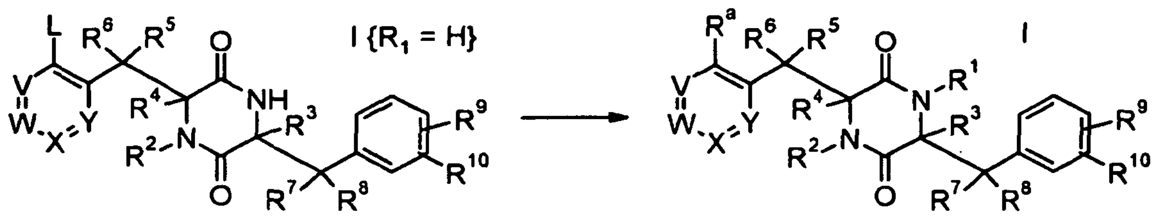

Gemäß Verfahren A wird eine Piperazinverbindung der Formel I mit $R^1$ = Wasserstoff mit einem geeigneten Alkylierungsmittel, im Folgenden Verbindung $X^1$-$R^1$, oder Acylierungsmittel, im Folgenden Verbindung $X^2$-$R^1$, umgesetzt, wobei man eine Piperazinverbindung der Formel I mit $R^1$ * Wasserstoff erhält.

In den Alkylierungsmitteln $X^1$-$R^1$ kann $X^1$ Halogen oder O-$SO_2$-$R^m$ mit $R^m$ in der Bedeutung von $C_1$-$C_4$-Alkyl oder Aryl, welche gegebenenfalls durch Halogen, $C_1$-$C_4$-Alkyl oder Halo-$C_1$-$C_4$-alkyl substituiert sind, bedeuten. In Acylierungsmitteln $X^2$-$R^1$ kann $X^2$ Halogen, insbesondere Cl bedeuten. Dabei ist $R^1 \neq$ Wasserstoff und hat die oben angegebene Bedeutung und steht insbesondere für $C_1$-$C_6$-Alkyl, $C_3$-$C_6$-Cycloalkyl, $C_3$-$C_6$-Alkenyl, $C_3$-$C_6$-Cycloalkenyl, $C_3$-$C_6$-Alkinyl, $C_3$-$C_6$-Cycloalkinyl, Phenyl-$C_1$-$C_6$-alkyl, Heterocyclyl, Heterocyclyl-$C_1$-$C_6$-alkyl; Phenyl-[$C_1$-$C_6$-alkoxycarbonyl]-$C_1$-$C_6$-alkyl oder Phenylheterocyclyl-$C_1$-$C_6$-alkyl; oder $COR^{11}$ oder $SO_2R^{25}$, wobei die genannten aliphatischen, cyclischen oder aromatischen Teile von $R^1$ partiell oder vollständig halogeniert sein können und/oder eine bis drei der folgenden Gruppen tragen können: Cyano, Hydroxy, $C_1$-$C_4$-Alkyl, $C_1$-$C_4$-Halogenalkyl, $C_3$-$C_6$-Cycloalkyl, $C_1$-$C_4$-Alkoxy, $C_1$-$C_4$-Alkylthio, [Di-($C_1$-$C_4$)-alkyl]-amino, $C_1$-$C_4$-Akylcarbonyl, Hydroxycarbonyl, $C_1$-$C_4$-Alkoxycarbonyl, Aminocarbonyl, $C_1$-$C_4$-Alkylaminocarbonyl, [Di-($C_1$-$C_4$)-alkyl]aminacarbonyl oder $C_1$-$C_4$-Alkylcarbonyloxy.

Die Umsetzung erfolgt üblicherweise bei Temperaturen im Bereich von -78°C bis zum Siedepunkt der Reaktionsmischung, bevorzugt von -50°C bis 65°C, insbesondere bevorzugt von -30°C bis 65°C. In der Regel wird die Umsetzung in einem Lösungsmittel vorgenommen, bevorzugt in einem inerten organischen Lösungsmittel.

Geeignete inerte organische Lösungsmittel umfassen aliphatische Kohlenwasserstoffe wie Pentan, Hexan, Cyclohexan und Gemische von $C_5$-$C_8$-Alkanen, aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol, tert.-Butylalkohol Wasser sowie Dimethylsulfoxid, Dimethylformamid und Dimethylacetamid sowie Morpholin und N-Methylmorpholin. Es können auch Gemische der genannten Lösungsmittel verwendet werden.

Bevorzugtwird die Reaktion in einem Tetrahydrofuran - Wasser Gemisch durchgeführt, beispielsweise mit einem Mischungsverhältnis von 1 : 10 bis 10 : 1 (Volumenteile). In einer anderen bevorzugten Ausgestaltung kommen Toluol, Dichlormethan, Tetrahydrofuran oder Dimethylformamid oder deren Mischungen in Betracht. Besonders bevorzugt wird die Reaktion in Tetrahydrofuran durchgeführt.

Besonders bevorzugt wird die Verbindung I mit $R^1$ = H mit dem Alkylierungs- bzw. Acylierungsmittel in Gegenwart einer Base durchgeführt.

Als Basen kommen allgemein anorganische Verbindungen wie Alkalimetall- und Erdalkalimetallhydroxide wie Lithiumhydroxid, Natriumhydroxid, Kaliumhydroxid oder Calciumhydroxid, wässrige Lösung von Ammoniak, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, beispielsweise Lithiumdiisopropylamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butylalkoholat, Kalium-tert.-Pentylakoholat und Dimethoxymagnesium, außerdem organische Basen, z.B. tertiäre Amine wie Trimethylamin, Triethylamin, Diisopropylethylamin 2-Hydroxypyridin und N-Methylpiperidin, Pyridin, substituierte Pyridine wie Collidin, Lutidin und 4-Dimethylaminopyridin sowie bicyclische Amine in Betracht. Es kann auch eine Mischung verschiedener Basen verwendet werden.

Die Reaktion von II kann in Gegenwart von Basen, bevorzugt in Gegenwart der Basen Kalium- tert.- Butylalkoholat, 2-Hydroxypyridin oder einer wässrigen Lösung von Ammoniak oder einer Mischung dieser Basen durchgeführt werden. Bevorzugt wird nur eine dieser Basen verwendet. Besonders bevorzugt wird die Reaktion in Gegenwart einer wässrigen Lösung von Ammoniak, die beispielsweise von 10 bis 50 w/v %ig sein kann, durchgeführt.

Die Basen werden im allgemeinen äquimolar eingesetzt. Sie können auch im Überschuß oder selbst als Lösungsmittel verwendet werden. Die Base kann in äquimolarer Menge oder im wesentlichen äquimolarer Menge zugesetzt werden. Natriumhydrid kann als Base verwendet werden.

Die Reaktionsgemische erhalten nach einem der genannten Verfahren können z.B. in üblicher Weise aufgearbeitet werden. Das kann z.B. durch Mischen mit Wasser, Trennung der Phasen und gegebenenfalls chromatographische Reinigung der Rohprodukte erfolgen. Die Zwischen- und Endprodukte fallen z.T. in Form zäher Öle an, die in der Regel unter vermindertem Druck und bei mäßig erhöhter Temperatur von flüchtigen Anteilen befreit oder gereinigt werden können. Sofern die Zwischen- und Endprodukte als Feststoffe erhalten werden, kann die Reinigung auch durch Umkristallisieren oder Digerieren erfolgen.

Verfahren B

B.1

Analog zu der voranstehend geschilderten Weise können Verbindungen I, worin $R^2$ für Wasserstoff steht, mit Alkylierungsmitteln $R^2$-$X^1$ oder Acyclierungsmitteln $R^2$-$X^2$ umgesetzt werden, wobei man Verbindungen der Formel I mit $R^2$ * Wasserstoff erhält (Verfahren B). Die Reaktionsbedingungen entsprechen denen des Verfahrens A. Bevorzugte Basen sind Natriumhydrid (NaH), Lithiumdiisopropylamid (LDA) und Lithiumhexamethyldisilazid (LiHMDS).

B.2

Analog zu der voranstehend geschilderten Weise können Verbindungen I, worin $R^3$ für Wasserstoff steht, mit Alkylierungsmitteln $R^3$-$X^1$ oder Acyclierungsmitteln $R^3$-$X^2$ umgesetzt werden, wobei man Verbindungen der Formel I mit $R^3$ * Wasserstoff erhält. Die Reaktionsbedingungen entsprechen denen des Verfahrens A.

Sofern die Gruppe $R^1$ in Formel I oder II für Wasserstoff steht, wird durch Alkylierung die Gruppe $R^1$ eingeführt. Sofern die Gruppe $R^1$ in Formel I oder II für eine Schutzgruppe steht, wird diese zunächst entfernt, wobei man eine Verbindung erhält, worin $R^1$ für Wasserstoff steht, in die durch Alkylierung die Gruppe $R^1$ eingeführt wird. Sofern $R^2$ in Formel I oder II für Wasserstoff steht, kann durch einen Alkylierungs- bzw. Acylierungsschritt die Gruppe $R^2$ eingeführt werden. Wenn $R^1$ und $R^2$ identisch sind, können die Alkylierungs- bzw. Acylierungsschritte gleichzeitig oder sukzessive in beliebiger Reihenfolge durchgeführt werden. Wenn die Gruppen $R^1$, $R^2$ und $R^3$ identisch sind, kann man die Einführung der Gruppe $R^3$ gleichzeitig mit der Einführung der Gruppen $R^1$ und/oder $R^2$ oder im Anschluss daran durchführen.

Die Einführung der Gruppen $R^1$, $R^2$ und/oder $R^3$ kann alternativ auch bei weiteren Vorstufen der Verbindungen I oder II erfolgen. So können z.B. Verbindungen IV, VI, VIII, IX, XI und XII, in denen $R^1$, $R^2$ und/oder $R^3$ für Wasserstoff stehen, den zuvor beschriebenen Umsetzungen unterworfen werden.

Verfahren C

Die Verbindungen der Formel I können gemäß dem im folgenden Schema skizzierten Verfahren durch Umwandlung des Substituenten Ra hergestellt werden, beispielsweise analog J. Tsuji, Top. Organomet. Chem. (14) (2005), 332 pp., oder J. Tsuji, Organic Synthesis with Palladium Compounds. (1980), 207 pp.

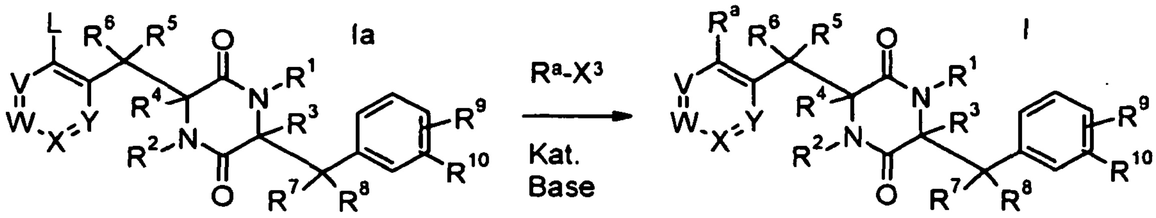


So können beispielweise Verbindungen der Formel I, in denen $R^a$ für CN, gegebenenfalls substituiertes Phenyl oder für eine gegebenenfalls substituierte heterocyclische Gruppe steht, ausgehend von Verbindungen I, worin $R^a$ für Halogen wie Cl, Br oder I steht, durch Umwandlung des Substituenten $R^a$ hergestellt werden

Hierzu wird eine Piperazinverbindung der Formel Ia, die anstelle des Substituenten $R^a$ eine geeignete Abgangsgruppe L aufweist, durch Umsetzung mit einem Kupplungspartner, der eine Gruppe $R^a$ enthält (Verbindung $R^a$-$X^3$), in ein anderes Piperazinderivat der Formel I übenführt.

Die Umsetzung erfolgt üblicherweise in Gegenwart eines Katalysators, bevorzugt in Gegenwart eines Übergangsmetallkatalysators. In der Regel findet die Reaktion in Gegenwart einer Base statt.

Diese Reaktionssequenz ist im Folgenden am Beispiel des Substituenten $R^a$ dargestellt und kann selbstredend in analoger Weise für die Umwandlung der Substituenten $R^b$ herangezogen werden.

Als Abgangsgruppe L kommen z.B. Halogen oder $S(O)_nR^k$, mit n = 0, 1, 2, 3, wie z.B. Triflat und $R^k$ in der Bedeutung von $C_1$-$C_6$-Alkyl, Halo-$C_1$-$C_6$-alkyl oder gegebenenfalls halogeniertem oder mit $C_1$-$C_4$-Alkyl substituiertem Aryl in Betracht.

Als Kupplungspartner $X^3$-$R^a$ kommen insbesondere solche Verbindungen in Betracht, worin $X^3$ im Falle von $R^a$ in der Bedeutung von $C_1$-$C_6$-Alkyl, $C_2$-$C_6$-Akenyl, Aryl oder Heteroaryl für eine der folgenden Gruppen steht:

- Zn-$R^l$ mit $R^l$ in der Bedeutung von Halogen, $C_1$-$C_6$-Alkyl, $C_2$-$C_6$-Alkenyl, Aryl oder Heteroaryl ;
- $B(OR^m)_2$, mit $R^m$ in der Bedeutung von H oder $C_1$-$C_6$-Alkyl, wobei zwei Alkylsubstituenten zusammen eine $C_2$-$C_4$-Alkylenkette bilden können; oder
- $SnR^n{}_3$, mit $R^n$ in der Bedeutung von $C_1$-$C_6$-Alkyl, Aryl oder Alkoxyalkenyl bedeutet; und

Sofern $R^a$ für $C_2$-$C_6$-Akinyl steht, kann $X^3$ auch Wasserstoff bedeuten.

Diese Umsetzung erfolgt üblicherweise bei Temperaturen im Bereich von -78°C und dem Siedepunkt der Reaktionsmischung, bevorzugt von -30°C bis 65°C, insbesondere bevorzugt bei Temperaturen von 30°C bis 65°C. In der Regel wird die Umsetzung in einem inerten organischen Lösungsmittel in Gegenwart einer Base durchgeführt.

Geeignete Lösungsmittel sind die unter Verfahren A zitierten Verbindungen. In einer Ausführungsform wird Tetrahydrofuran mit einer katalytischen Menge Wasser verwendet werden; in einer anderen Ausführungsform wird nur Tetrahydrofuran eingesetzt.

Geeignete Basen sind die unter Verfahren A zitierten Verbindungen. Die Basen werden im allgemeinen äquimolar eingesetzt. Sie können auch im Überschuß oder selbst als Lösungsmittel verwendet werden.

Bevorzugt wird die Base in äquimolarer Menge zugesetzt. In einer weiteren bevorzugten Ausführungsform werden Triethylamin oder Cäsiumcarbonat als Base verwendet, besonders bevorzugt Cäsiumcarbonat.

Als Katalysatoren sind prinzipiell Verbindungen der Übergangsmetalle Ni, Fe, Pd, oder Cu geeignet. Es ist möglich organische oder anorganische Verbindungen einzusetzen. Es kommen Übergangsmetallkomplexe mit verschiedenen Liganden in Frage (vgl. Accts. Chem. Res. 2008, 41 (11), 1439-1564, Sonderheft; Angew. Chem. Int. Ed. Engl., 2009, 48, 4114-4133). Beispielhaft seien genannt: $Pd(PPh_3)_2Cl_2$, $Pd(OAc)_2$, $PdCl_2$, oder $Na_2PdCl_4$. Ph steht hierbei für Phenyl.

Zur Herstellung der Verbindung I, worin $R^a$ für CN steht, kann man die Verbindung Ia, worin L für Chlor, Brom oder Iod steht, auch mit Kupfercyanid analog bekannter Verfahren umsetzen (vgl. Organikum, 21. Auflage, 2001, Wiley, S. 404; Tetrahedron Lett. 42, 2001, S.7473; Org. Lett. 5, 2003, 1785).

Diese Umsetzungen erfolgt üblicherweise bei Temperaturen im Bereich von 100°C bis zum Siedepunkt der Reaktionsmischung, bevorzugt von 100°C bis 250°C. In der Regel wird die Umsetzung in einem inerten organischen Lösungsmittel durchgeführt. Geeignete Lösungsmittel sind insbesondere aprotisch polare Lösungsmittel, z.B. Dimethylformamid, N-Methylpyrrolidon, N,N'-Dimethylimidazolidin-2-on und Dimethylacetamid.

Die Umwandlung der Gruppe $R^a$ kann alternativ auch bei den Vorstufen der Verbindung I erfolgen. So können z.B. Verbindungen II in denen $R^a$ für ein Halogenatom wie Cl, Br oder I steht, der zuvor beschriebenen Umsetzung unterworfen werden.

Verfahren D

Die Verbindungen der Formel I können gemäß der im folgenden gezeigten Synthese durch Kupplung von Piperazinverbindungen der Formel IV mit Verbindungen V hergestellt werden. Die Kupplung von IV mit V gelingt analog bekannter Verfahren, beispielsweise nach G. Porzi, et al., Tetrahedron 9 (19), (1998), 3411-3420, oder C. I. Harding et al., Tetrahedron 60 (35), (2004), 7679-7692.

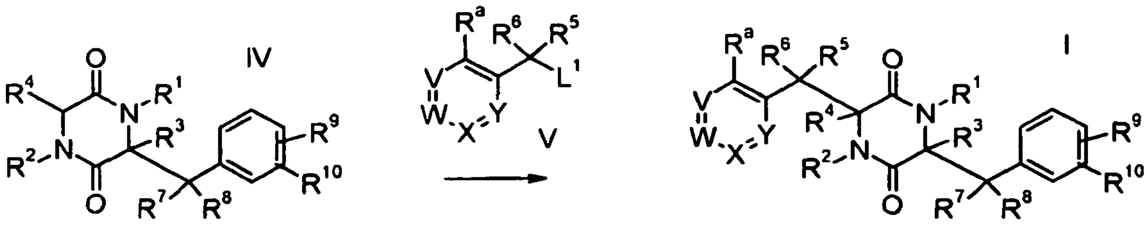


In dem Schema haben V, W, X, Y und $R^1$-$R^{10}$ die angebenen Bedeutungen. $L^1$ steht für eine geeignete Abgangsgruppe, wie Halogen oder $OSO_2R^m$, mit Rm in der Bedeutung von $C_1$-$C_4$-Alkyl, Aryl, oder ein- bis dreifach durch $C_1$-$C_4$-Alkyl substituiertes Aryl.

In der Regel erfolgt die Reaktion bei Temperaturen im Bereich von -78°C bis zum Siedepunkt der Reaktionsmischung, bevorzugt im Bereich von -78°C bis 40°C, insbesondere bevorzugt im Bereich von -78°C bis 30°C.

In der Regel wird die Reaktion in einem inerten organischen Lösungsmittel in Gegenwart einer Base durchgeführt. Geeignete Lösungsmittel sind die unter Verfahren A Zitierten, bevorzugt Tetrahydrofuran.

Geeignete Basen sind die unter Verfahren A zitierten Verbindungen. In einer weiteren bevorzugten Ausführungsform wird Lithiumdiisopropylamid, besonders bevorzugt in im wesentlichen äquimolarer Menge, insbesondere äquimolar als Base verwendet.

Verbindungen der Formel V sind zum Teil kommerziell erhältlich oder durch literaturbeschriebene Transformationen der entsprechenden kommerziell erhältlichen Vorprodukte herstellbar. Die Aufarbeitung kann analog Verfahren A erfolgen.

Die für die Herstellung der Verbindungen der Formel I benötigen Vorstufen und Zwischenprodukte sind teilweise kommerziell erhältlich, aus der Literatur bekannt oder können nach literaturbekannten Verfahren hergestellt werden.

Die Dipeptid-Verbindungen der Formel II können beispielsweise aus N-geschützten Dipeptiden der Formel VI analog bekannter Verfahren hergestellt werden, beispielsweise nach Glenn L. Stahl et al., J. Org. Chem. 43(11), (1978), 2285-6 oder A. K. Ghosh et al., Org. Lett. 3(4), (2001), 635-638.

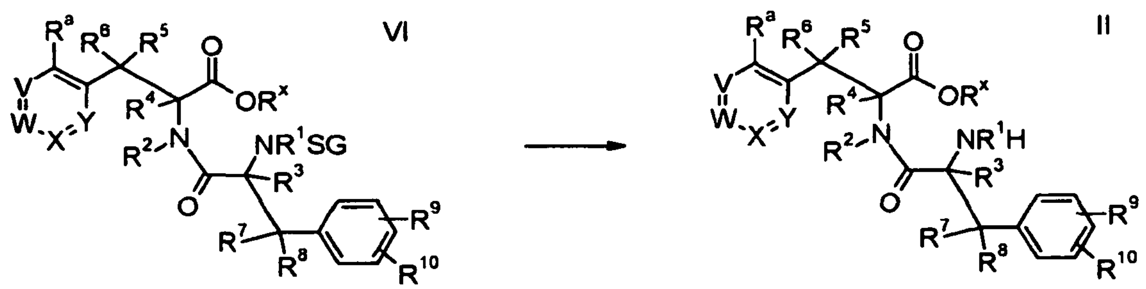


In Formeln II und VI haben die Variablen die angegebene Bedeutung, SG bedeutet eine Stickstoff-Schutzgruppe wie Boc (= tert-Butoxycarbonyl) und $OR^x$ steht für eine über ein Sauerstoffatom gebundene Abgangsgruppe. Selbstverständlich gelten die bevorzugten Bedeutungen für die Verbindungen der Formel I sinngemäß jeweils für die Verbindungen der Formel II oder IV. Bezüglich der Abgangsgruppe $OR^x$ gilt das zuvor für Formel II gesagte.

So kann beispielsweise ein Dipeptid der Formel VI, worin SG für Boc steht und $OR^x$ eine geeignete Abgangsgruppe, bei der $R^x$ z.B. $C_1$-$C_6$-Alkyl, insbesondere Methyl, Ethyl oder Benzyl ist, in Gegenwart einer Säure zu einer Verbindung der Formel II umgesetzt werden.

Die Umsetzung erfolgt üblicherweise bei Temperaturen im Bereich von-30°C und dem Siedepunkt der Reaktionsmischung, bevorzugt von 0°C bis 50°C, insbesondere bevorzugt von 20°C bis 35°C.

Die Reaktion kann in einem Lösungsmittel stattfinden, inbesondere in einem inerten organischen Lösungsmittel. Als Lösungsmittel kommen die bei der basischen Cyclisierung Zitierten in Betracht, insbesondere Tetrahydrofuran oder Dichlormethan oder deren Mischungen, bevorzugt in Dichlormethan.

Als Säuren kommen grundsätzlich sowohl Brönstedt- als auch Lewis-Säuren in Betracht. Insbesondere können anorganische Säuren, z.B. Halogenwasserstoffsäuren wie

Fluorwasserstoffsäure, Salzsäure, Bromwasserstoffsäure, anorganische Oxosäuren wie Schwefelsäure und Perchlorsäure, weiterhin anorganische Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren, Beispielsweise Carbonsäuren und Hydroxycarbonsäuren wie Ameisensäure, Essigsäure, Propionsäure, Oxalsäure, Zitronensäure und Trifluoressigsäure, sowie organische Sulfonsäuren wie Toluolsulfonsäure, Benzolsulfonsäure, Camphersulfonsäure und dergleichen, Verwendung finden. Selbstverständlich kann auch eine Mischung verschiedener Säuren eingesetzt werden.

Die Reaktion kann in Gegenwart von organischen Säuren durchgeführt werden, beispielsweise in Gegenwart starker organischer Säuren, wie Ameisensäure, Essigsäure oder Trifluoressigsäure oder deren Mischungen. In einer bevorzugten Ausführungsform wird die Reaktion in Gegenwart von Trifluoressigsäure durchgeführt. Die Aufarbeitung kann analog Verfahren A erfolgen.

Dipeptide der Formel VI können analog bekannter Verfahren hergestellt werden, beispielsweise nach Wilford L. Mendelson et al., Int. J.Peptide & Protein Research 35(3), (1990), 249-57. Ein typischer Zugang ist die Amidierung einer Boc-geschützen Aminosäure VIII mit einem Aminosäureester der Formel VII gemäß folgendem Schema:

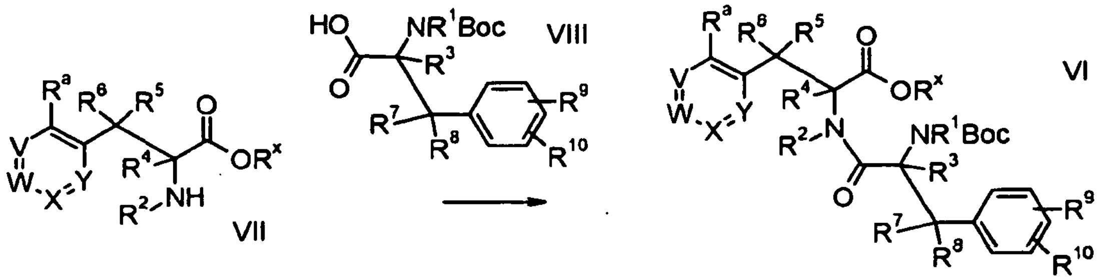

In diesem Schema haben die Variablen die genannten Bedeutungen. Anstelle von Boc können auch andere Amino-Schutzgruppen eingesetzt werden.

In der Regel erfolgt die Umsetzung von VII mit VIII bei Temperaturen in einem Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgeführt werden. Geeignet sind die bei Verfahren A, im Zusammenhang mit der basischen Cyclisierung genannten Lösungsmittel.

Im allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodiimid (DCC), Diisopropylcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isopropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Nach einer Ausführungsform werden als Aktivierungsreagenzien EDAC oder DCC bevorzugt.

Vorzugsweise erfolgt die Umsetzung von VII mit VIII in Gegenwart einer Base. Geeignete Basen sind die unter Verfahren A zitierten Verbindungen. In einer Ausführungsform werden als Base Triethylamin oder N-Ethyldiisopropylamin oder deren Mischungen, besonders bevorzugt N-Ethyldiisopropylamin verwendet. Die Aufarbeitung kann analog Verfahren A erfolgen.

Die Verbindungen der Formel VII können ihrerseits durch Entschützen entsprechender geschützten Aminosäureverbindungen IX analog bekannter Verfahren hergestellt werden, beispielsweise nach Glenn L. Stahl et al., J. Org. Chem. 43(11), (1978), 2285-6. oder A. K. Ghosh et al., Org. Lett. 3(4), (2001), 635-638. Die Herstellung von VII aus einer Boc-geschützten Aminosäureverbindung IX ist im folgenden Schema dargestellt. Anstelle der Boc-Gruppe können auch andere Amino-Schutzgruppen eingesetzt werden.

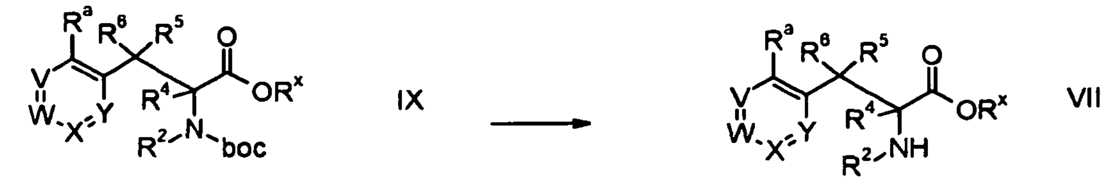

IX VII

Die Umsetzung einer Verbindung der Formel IX zur Verbindung VII erfolgt typischerweise in Gegenwart einer Säure bei Temperaturen in einem Berich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel.

Als Lösungsmittel kommen die unter der basischen Cyclisierung Zitierten in Betracht, insbesondere Tetrahydrofuran oder Dichlormethan oder deren Mischungen, bevorzugt in Dichlormethan.

Als Säuren und saure Katalysatoren kommen grundsätzlich sowohl Brönstedt- als auch Lewis-Säuren, insbesondere die weiter oben genannten, in Betracht.

Die Reaktion kann in Gegenwart von organischen Säuren durchgeführt werden, beispielsweise in Gegenwart von starken organischen Säuren, wie Ameisensäure, Essigsäure oder Trifluoressigsäure oder deren Mischungen, bevorzugt in Gegenwart von Trifluoressigsäure. Die Aufarbeitung kann analog Verfahren A erfolgen.

Die Verbindungen der Formel IX können entsprechend der im folgenden Schema dargestellten Umsetzung hergestellt werden. Die Umsetzung von Verbindung V mit der Aminosäureverbindung X kann in Analogie zu Literatur bekannten Verfahren erfolgen, beispielsweise nach I. Ojima et al., J. Am. Chem. Soc., 109(21), (1987), 6537-6538 oder J. M. McIntosh et al., Tetrahedron 48(30), (1992), 6219-6224.

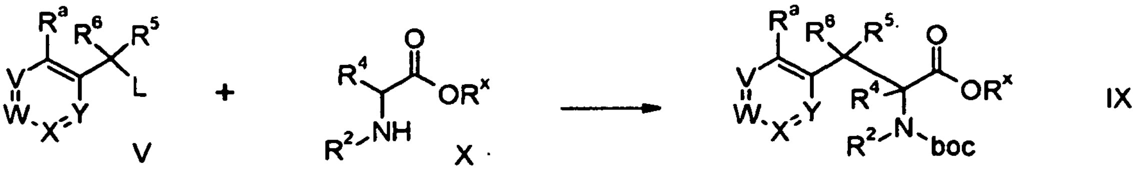

V X IX

In diesem Schema haben die Variablen die genannten Bedeutungen. L steht für eine Abgangsgruppe, z.B eine der bei Verfahren F genannten Abgangsgruppen. Anstelle von Boc können auch andere Amino-Schutzgruppen eingesetzt werden.

Die Umsetzung von V mit X von erfolgt in der Regel in Gegenwart von Base. Geeignete Basen sind die unter Verfahren A zitierten Verbindungen. In einer weiteren bevorzugten Ausführungsform wird Lithiumdiisopropylamid, besonders bevorzugt in im wesentlichen äquimolarer Menge, insbesondere äquimolar als Base verwendet.

Üblicherweise wird die Reaktion bei Temperaturen im Bereich von - 78°C und dem Siedepunkt der Reaktionsmischung, bevorzugt von - 78°C und dem Siedepunkt, insbesondere bevorzugt von - 78°C bis 30°C durchgeführt.

Die Reaktion kann in einem Lösungsmittel durchgeführt werden, bevorzugt in einem inerten organischen Lösungsmittel. Als Lösungsmittel kommen prinzipiell die unter der basischen Cyclisierung genannten Lösungsmittel in Betracht, insbesondere Dichlormethan oder Tetrahydrofuran oder deren Mischungen, bevorzugt in Tetrahydrofuran. Die Aufarbeitung kann analog Verfahren A erfolgen.

Verbindungen der Formel V sind zum Teil kommerziell erhältlich oder durch literaturbeschriebene Transformationen der entsprechenden kommerziell erhältlichen Vorprodukte herstellbar.

Aminosäurederivate der Formel VIII, X oder das unten beschriebene Derivat XV sind ebenfalls zum Teil kommerziell erhältlich oder durch literaturbeschriebene Transformationen der entsprechenden kommerziell erhältlichen Vorprodukten herstellbar.

Die Verbindungen der Formel IV mit $R^1 \neq$ Wasserstoff können dadurch hergestellt werden, dass man eine Piperazinverbindung der Formel IV, worin $R^1$ für Wasserstoff steht, mit einem Alkylierungsmittel oder Acylierungsmittel, das den von Wasserstoff verschiedenen Rest $R^1$ enthält, umsetzt. In analoger Weise können Verbindungen IV mit $R^2$ * Wasserstoff dadurch hergestellt werden, dass man eine Piperazinverbindung der Formel IV, worin $R^2$ für Wasserstoff steht, mit einem Alkylierungsmittel oder Acylierungsmittel, das den von Wasserstoff verschiedenen Rest $R^2$ enthält, umsetzt. Derartige Umsetzungen können analog bekannter Verfahren erfolgen, beispielsweise nach den von I.O. Donkor et al., Bioorg. Med. Chem. Lett. 11 (19) (2001), 2647-2649, B.B. Snider et al., Tetrahedron 57 (16) (2001), 3301-3307, I. Yasuhiro et al., J. Am. Chem. Soc. 124(47) (2002), 14017-14019, oder M. Falorni et al., Europ. J. Org. Chem. (8) (2000), 1669-1675 beschriebenen Methoden.

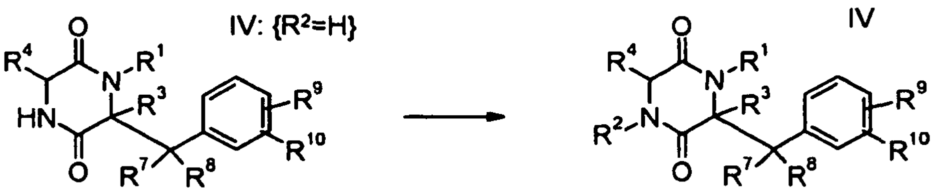

Bezüglich der Alkylierungsmittel bzw. Acyclierungsmittel gilt das bei den Verfahren B und C Gesagte in gleicher Weise. Bezüglich der Reaktionsbedingungen dieser Umsetzungen gilt ebenfalls das zuvor bei den Verfahren B und C Gesagte.

Die Verbindungen der Formel IV können auch durch intramolekulare Cyclisierung von Verbindungen der Formel XIII analog zu weiteren bekannten Verfahren hergestellt werden, beispielsweise nach T. Kawasaki et al., Org. Lett. 2 (19) (2000), 3027-3029.

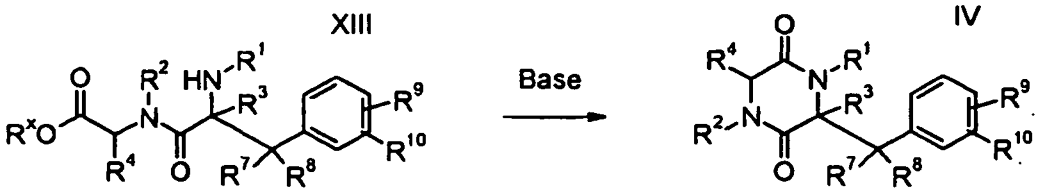


Dabei ist $OR^x$ eine geeignete Abgangsgruppe, $R^x$ ist hierbei z.B. $C_1$-$C_6$-Alkyl, insbesondere Methyl, Ethyl oder Benzyl.

In Formel XIII haben die Variablen die für Formel II angegebene Bedeutung. Die Gruppe $OR^x$ steht für eine geeignete, über Sauerstoff gebundene Abgangsgruppe. $R^x$ ist hierbei z.B. $C_1$-$C_6$-Alkyl, insbesondere Methyl, Ethyl oder Phenyl-$C_1$-$C_6$-alkyl, z.B. Benzyl.

Die Cyclisierung der Verbindungen der Formel XIII, kann in Gegenwart einer Base erfolgen. Die Reaktion erfolgt dann in der Regel bei Temperaturen im Bereich von 0°C und dem Siedepunkt der Reaktionsmischung, bevorzugt von 10°C bis 50°C, insbesondere bevorzugt von 15°C bis 35°C. Die Umsetzung kann in einem Lösungsmittel durchgeführt werden, bevorzugt in einem inerten organischen Lösungsmittel.

Als Lösungsmittel kommen prinzipiell die unter der thermischen Cyclisierung zitierten Verbindungen, in Betracht, insbesondere ein Tetrahydrofuran - Wasser Gemisch mit einem Mischungsverhältnis von 1 : 10 bis 10 : 1.

Geeignete Basen sind die bei der basischen Cyclisierung gemäß Verfahren A genannten Basen, insbesondere Kalium- tert.- Butylalkoholat, 2-Hydroxypyridin oder eine wässrige Lösung von Ammoniak oder einer Mischung dieser Basen. Bevorzugt wird nur eine dieser Basen verwendet. In einer besonders bevorzugten Ausführungsform wird die Reaktion in Gegenwart einer wässrigen Lösung von Ammoniak, die beispielsweise von 10 bis 50 v/v %ig sein kann, durchgeführt.

Die Verbindungen der Formel XIII können ihrerseits nach der im folgenden Schema dargestellten Synthese analog zu bekannten Verfahren hergestellt werden, beispielsweise nach Wilford L. Mendelson et al., Int. J.Peptide & Protein Research 35(3), (1990), 249-57, Glenn L. Stahl et al., J. Org. Chem. 43(11), (1978), 2285-6. oder A. K. Ghosh et al., Org. Lett. 3(4), (2001), 635-638.

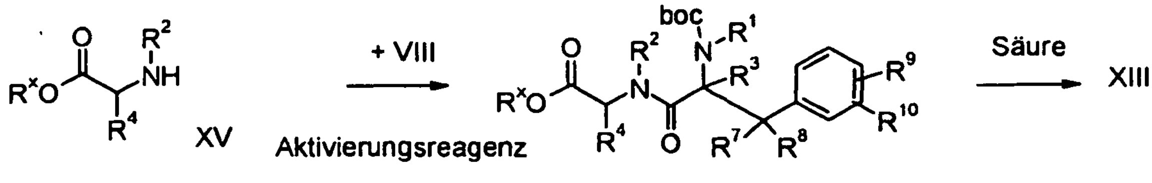


In dem Schema haben die Variablen $R^x$, $R^1$-$R^4$ und $R^7$-$R^{10}$ die für Formel II bzw. XIII angegebenen Bedeutungen. Die Synthese umfasst in einem ersten Schritt die Kupplung von Aminosäureverbindungen XV mit Boc-geschützten Aminosäuren VIII in Gegenwart eines Aktivierungsreagenz.

Die Umsetzung einer Verbindung der Formel XV mit einer Verbindung der Formel VIII erfolgt üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel. Wegen weiterer Details wird diesbezüglich auf die Herstellung von Verbindung VI durch Amidierung der Aminosäureverbindung VIII mit der Verbindung VII verwiesen.

Im allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodiimid (DCC), Diisopropylcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isopropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCl) oder Sulfonylchloride wie Methansulfonylchlorid, Toluolsulfonylchlorid oder Benzolsulfonylchlorid. Nach einer Ausführungsform werden als Aktivierungsreagenzien EDAC oder DCC bevorzugt.

Vorzugsweise erfolgt die Umsetzung von XV mit VIII in Gegenwart einer Base. Geeignete Basen sind die unter Verfahren A Zitierten. In einer Ausführungsform werden als Base Triethylamin oder N-Ethyldiisopropylamin oder deren Mischungen, besonders bevorzugt N-Ethyldiisopropylamin verwendet. Die Aufarbeitung kann analog Verfahren A erfolgen.

Das Entschützen der Verbindung XIV zur Verbindung XIII erfolgt typischerweise durch Behandlung mit einer Säure. Die Umsetzung erfolgt üblicherweise bei Temperaturen im Bereich von -30°C und dem Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgefürt werden.

Als Lösungsmittel kommen prinzipiell die unter Verfahren A im Zusammenhang mit der basischen Cyclisierung genannten Lösungsmittel in Betracht, insbesondere Tetrahydrofuran oder Dichlormethan oder deren Mischungen, bevorzugt in Dichlormethan.

Als Säuren finden die bei Verfahren A genannten Säuren Verwendung. Wegen weitere Details wird auch auf die Entschützung von VI zur Verbindung II verwiesen. Die dort genannten Reaktionsbedingungen eignen sich auch zum Entschützen von Verbindung XIV. Die Reaktion kann in Gegenwart von organischen Säuren, insbesondere starker organischer Säuren durchgeführt werden, beispielsweise in Gegenwart von Ameisensäure, Essigsäure oder Trifluoressigsäure oder deren Mischungen, bevorzugt in Gegenwart von Trifluoressigsäure. Die Aufarbeitung kann analog Verfahren A erfolgen.

Die Verbindungen der Formel I, in der $R^4$ und $R^5$ gemeinsam eine kovalente Bindung darstellen (Formel I.A),

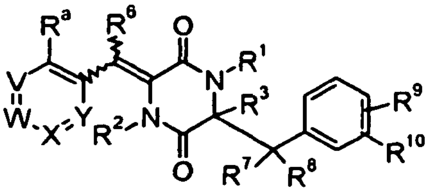

I.A

können nach Standardmethoden der Synthese organischer Verbindungen auf verschiedene Art und Weise herstellt werden, bevorzugt über die im Folgenden dargestellten Synthesen:

Verfahren E

Verbindung der Formel XIV, worin $R^6$ für H steht (Formel XIVa), können auch dadurch hergestellt werden, dass man in einer Aldolreaktion den Aldehyd Va mit dem Piperazin IV koppelt, wie im folgenden Schema skizziert ist:

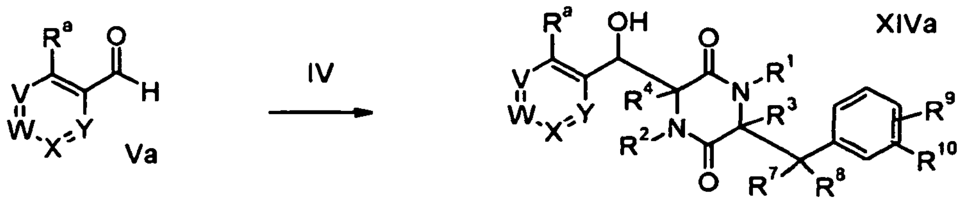


In den Formeln haben die Variablen die angegebene Bedeutung. In Formel XIVa können die Gruppen $R^1$ und $R^2$ unabhängig voneinander auch für Alkylcarbonyl, wie z.B. Acetyl, stehen. Die Umsetzung erfolgt in der Regel analog der für die Umsetzung von IIa zu XIV beschriebenen Bedingungen.

Die Aldolreakion kann auch direkt zum entsprechenden Aldolkondensationsprodukt, d.h. zu Verbindungen der Formel I.A, worin $R^6$ für H steht, führen. Dies ist insbesondere dann der Fall, wenn die Umsetzung bei höheren Temperaturen und unter längeren Reaktionszeiten verläuft.

Der Aldehyd Va ist entweder kommerziell erhältlich oder kann gemäß bekannten Verfahren zur Herstellung

von Aldehyden synthetisiert werden. Solche Aldolkondensationen können analog zu den in J. Org. Chem. 2000, 65 (24), 8402-8405 beschriebenen Verfahren durchgeführt werden.

Verfahren F

Das Verfahren A ist vorteilhaft zur Herstellung von Verbindungen I.A mit $R^1 \neq$ Wasserstoff geeignet. Die in Verfahren A genannten Bedingungen und Bevorzugungen betreffen analog auch die Herstellung der Verbindungen I.A.

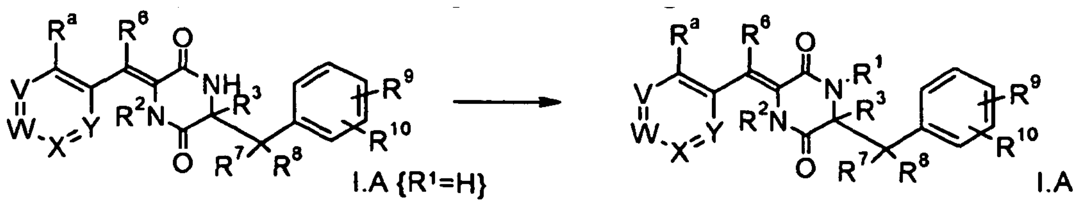

I.A {R¹=H} I.A

Vorteilhaft geeignete Lösungsmittel sind die unter Verfahren A Zitierten, unter anderem Toluol, Dichlormethan, Tetrahydrofuran oder Dimethylformamid oder deren Mischungen, bevorzugt Tetrahydrofuran.

In einer bevorzugten Ausführungsform wird die Verbindung I mit $R^1$ = H mit dem Alkylierungs- bzw. Acylierungsmittel in Gegenwart einer Base durchgeführt. Geeignete Basen sind die unter Verfahren A zitierten Verbindungen. Die Basen werden im allgemeinen äquimolar eingesetzt. Sie können auch im Überschuss oder selbst als Lösungsmittel verwendet werden. In einer bevorzugten Ausführungsform wird die Base in äquimolarer Menge oder im wesentlichen äquimolarer Menge zugesetzt. in einer weiteren bevorzugten Ausführungsform wird Natriumhydrid als Base verwendet. Die Aufarbeitung kann analog Verfahren A erfolgen.

Die Alkylierung bzw. Acylierung der Gruppe $NR^1$, und/oder $NR^2$ worin $R^1$, bzw. $R^2$ für H steht, kann alternativ auch in den Vorstufen erfolgen. So können z.B. Verbindungen II, IV, VI, VII, VIII, IX, X, XIII, XIV, XV oder XVI, in denen $R^1$ und/oder $R^2$ für H steht, wie zuvor beschrieben N-alkyliert oder N-acyliert werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder in der zu bekämpfenden Schadpflanze erfolgen.

Die für die Substituenten der erfindungsgemäßen Verbindungen genannten organischen Molekülteile stellen Sammelbegriffe für individuelle Aufzählungen der einzelnen Gruppenmitglieder dar. Sämtliche Kohlenwasserstoffketten, wie Alkyl, Halo(gen)alkyl, Alkenyl, Alkinyl, sowie die Alkylteile und Alkenylteile in Alkoxy, Halo(gen)alkoxy, Alkylamino, Dialkylamino, N-Alkylsulfonylamino, Alkenyloxy, Alkinyloxy, Alkoxyamino, Alkylaminosulfonylamino, Dialkylaminosulfonylamino, Alkenylamino, Alkinylamino, N-(Alkenyl)-N-(alkyl)-amino, N-(Alkinyl)-N-(alkyl)-amino, N-(Alkoxy)-N-(alkyl)-amino, N-(Alkenyl)-N-(alkoxy)-amino oder N-(Alkinyl)-N-(alkoxy)-amino können geradkettig oder verzweigt sein.

Das Präfix $C_n$-$C_m$- gibt die jeweilige Kohlenstoffzahl der Kohlenwasserstoffeinheit an. Sofern nicht anders angegeben tragen halogenierte Substituenten vorzugsweise ein bis fünf gleiche oder verschiedene Halogenatome, insbesondere Fluoratome oder Chloratome.

Die Bedeutung Halogen steht jeweils für Fluor, Chlor, Brom oder lod.

Ferner bedeuten beispielsweise:

Alkyl sowie die Alkylteile beispielsweise in Alkoxy, Alkylamino, Dialkylamino, N-Alkylsulfonylamino, Alkylaminosulfonylamino, Dialkylaminosulfonylamino, N-(Alkenyl)-N-(alkyl)-amino, N-(Alkinyl)-N-(alkyl)-amino, N-(Alkoxy)-N-(alkyl)-amino,: gesättigte, geradkettige oder verzweigte Kohlenwasserstoffreste mit einem oder mehr C-Atomen, z.B. 1 bis 2, 1 bis 4, oder 1 bis 6 Kohlenstoffatomen, z.B. $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl. In einer erfindungsgemäßen Ausführungsform steht Alkyl für kleine Alkylgruppen wie $C_1$-$C_4$-Alkyl. In einer anderen erfindungsgemäßen Ausführungsform steht Alkyl für größere Alkylgruppen wie $C_5$-$C_6$-Alkyl.

Halogenalkyl (auch als Haloalkyl bezeichnet): einen Alkylrest wie vorstehend genannt, dessen Wasserstoffatome partiell oder vollständig durch Halogenatome wie Fluor, Chlor, Brom und/oder Iod substituiert sind, z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl.

Cycloalkyl sowie die Cycloalkylteile beispielsweise in Cycloalkoxy oder Cycloalkylcarbonyl: monocyclische, gesättigte Kohlenwasserstoffgruppen mit drei oder mehr C-Atomen, z.B. 3 bis 6 Kohlenstoffringgliedem, wie Cyclopropyl, Cyclobutyl, Cyclopentyl und Cyclohexyl.

Alkenyl sowie Alkenylteile beispielsweise in Alkenylamino, Alkenyloxy, N-(Alkenyl)-N-(alkyl)-amino, N-(Alkenyl)-N-(alkoxy)-amino: einfach ungesättigte, geradkettige oder verzweigte Kohlenwasserstoffreste mit zwei oder mehr C-Atomen, z. B. 2 bis 4, 2 bis 6 oder 3 bis 6 Kohlenstoffatomen und einer Doppelbindung in einer beliebigen Position, z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

Cycloalkenyl: monocyclische, einfach ungesättigte Kohlenwasserstoffgruppen mit 3 bis 6, vorzugsweise 5 bis 6 Kohlenstoffringgliedem, wie Cyclopenten-1-yl, Cyclopenten-3-yl, Cyclohexen-1-yl, Cyclohexen-3-yl, Cyclohexen-4-yl.

Alkinyl sowie Alkinylteile beispielsweise in Alkinyloxy, Alkinylamino, N-(Alkinyl)-N-(alkyl)-amino oder N-(Alkinyl)-N-(alkoxy)-amino: geradkettige oder verzweigte Kohlenwasserstoffgruppen mit zwei oder mehr C-Atomen, z. B. 2 bis 4, 2 bis 6, oder 3 bis 6 Kohlenstoffatomen und einer Dreifachbindung in beliebiger Position, z. B. $C_2$-$C_6$-Akinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Alkoxy: Alkyl, wie vorstehend definiert, das über ein O-Atom gebunden ist: z. B. Methoxy, Ethoxy, n-Propoxy, 1-Methylethoxy, Butoxy, 1-Methylpropoxy, 2-Methylpropoxy oder 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

5- oder 6-gliedriger Heterocyclus: eine cyclische Gruppe, die 5 oder 6 Ringatome aufweist wobei 1, 2, 3 oder 4 Ringatome Heteroatome sind, die ausgewählt sind aus O, S und N, wobei die cyclische Gruppe gesättigt, partiell ungesättigt oder aromatisch ist. Beispiele für heterocyclische Gruppen sind:

C-gebundene, 5-gliedrige, gesättigte Ringe wie Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl,

1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;

C-gebundene, 6-gliedrige, gesättigte Ringe wie: Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-l ,3-oxazin-6-yl, TeVahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl;

N-gebundene, 5-gliedrige, gesättigte Ringe wie: Tetrahydropyrrol-1-yl, Tetrahydropyrazof-1-yl, Tetrahydroisoxazot-2-yl, Tetrahydroisothiazol-2-yl, Tetrahydroimidazol-1-yl, Tetrahydrooxazol-3-yl, Tetrahydrothiazol-3-yl;

N-gebundene, 6-gliedrige, gesättigte Ringe wie: Piperidin-1-yl, Hexahydropyrimidin-1-yl, Hexahydropyrazin-1-yl, Hexahydro-pyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl;

C-gebundene, 5-gliedrige, partiell ungesättigte Ringe wie: 2,3-Dihydrofuran-2-yl, 2,3-Dihydrofuran-3-yl, 2,5-Dihydrofuran-2-yl, 2,5-Di-hydrofuran-3-yl, 4,5-Dihydrofuran-2-yl, 4,5-Dihydrofuran-3-yl, 2,3-Dihydro-thien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1 H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydro-isoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, $\Delta^3$-1,2-Dithiol-3-yl, $\Delta^3$-1,2-Dithiol-4-yl, $\Delta^3$-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydro-oxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl;

C-gebundene, 6-gliedrige, partiell ungesättigte Ringe wie: 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydrothiopyran-6-yl, 2H-3,4-Dihydrothiopyran-5-yl, 2H-3,4-Dihydrothiopyran-4-yl, 2H-3,4-Dihydrothiopyran-3-yl, 2H-3,4-Dihydrothiopyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetra-hydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydro-pyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetra-hydropyridazin-5-yl, 1,2,5,6-Tetrahydropyridazin-6-yl, 1,2,3,6-Tetrahydro-pyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydro-pyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl , 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl;

N-gebundene, 5-gliedrige, partiell ungesättigte Ringe wie: 2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-$\Delta^4$-Oxadiazolin-2-yl, 1,2,4-$\Delta^{2}$-Oxadiazolin-4-yl, 1,2,4-$\Delta^3$-Oxadiazolin-2-yl, 1,3,4-$\Delta^{2}$-Oxadiazolin-4-yl, 1,2,4-$\Delta^2$ -Thiadiazolin-2-yl, 1,2,4-$\Delta^3$-Thiadiazolin-2-yl, 1,2,4-$\Delta^2$-Thiadiazolin-4-yl, 1,3,4-$\Delta^2$-Thiadiazolin-4-yl, 1,2,3-$\Delta^2$-Triazolin-1-yl, 1,2,4-$\Delta^2$-Triazolin-1-yl, 1,2,4-$\Delta^2$-Triazolin-4-yl, 1,2,4-$\Delta^3$-Triazolin-1-yl, 1,2,4-$\Delta^1$-Triazolin-4-yl;

N-gebundene, 6-gliedrige, partiell ungesättigte Ringe wie: 1,2,3,4-Tetrahydropyridin-1-yl, 1,2,5,6-Tetrahydropyridin-1-yl, 1,4-Dihydro-pyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihydro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;

C-gebundene, 5-gliedrige, heteroaromatische Ringe wie: 2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3- Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, [1H]-Tetrazol-5-yl und [2H]-Tetrazol-5-yl;

C-gebundene, 6-gliedrige, heteroaromatische Ringe wie : Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl und 1,2,4-Triazin-6-yl;

N-gebundene, 5-gliedrige, heteroaromatische Ringe wie: Pyrrol-1-yl, Pyrazol-1-yl, Imidazol-1-yl, 1,2,3-Triazol-1-yl, 1,2,4-Triazol-1-yl, [1H]-Tetrazol-1-yl und [2H]-Tetrazol-2-yl.

Die vorgenannten Heterocyclen können in der angegebenen Weise substituiert sein. In den vorgenannten Heterocyclen kann ein Schwefelatom zu S=O oder $S(=O)_2$ oxidiert sein.

Die Verbindungen der Formel I.1 weisen am Kohlenstoffatom, welches die Gruppe $R^3$ und/oder $R^4$ trägt, ein Chiralitätszentrum auf. Zudem können sie, je nach Substitutionsmuster, ein oder mehrere weitere Chiralitätszentren enthalten. Die erfindungsgemäßen Verbindungen können daher als reine Enantiomere oder Diastereomere oder als Enantiomeren- oder Diastereomerengemische vorliegen. Gegenstand der Erfindung sind sowohl die reinen Enantiomeren oder Diastereomeren als auch deren Gemische.

Die Verbindungen der Formel I.1 können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen 1.1 nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium verwendet werden, wobei hier gewünschtenfalls ein bis vier Wasserstoffatome durch $C_1$-$C_4$-Alkyl, Hydroxy-$C_1$-$C_4$-alkyl, $C_1$-$C_4$-Alkoxy-$C_1$-$C_4$-alkyl, Hydroxy-$C_1$-$C_4$-alkoxy-$C_1$-$C_4$-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri($C_1$-$C_4$-alkyl)sulfonium oder Sulfoxoniumionen, vorzugsweise Tri($C_1$-$C_4$-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von $C_1$-$C_4$-Alkansäuren, vorzugsweise Formiat, Acetat, Propionat oder Butyrat.

Die Zwischenprodukte entsprechen in Bezug auf die Variablen denen der Gruppen der Formel I.1.

Die Verbindungen der Formel I, worin V für N und W, X sowie Y für C-$R^b$ stehen entsprechen den Verbindungen der Formel I.1

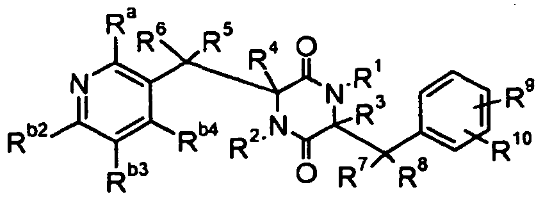

I.1

worin die Gruppen $R^{b2}$, $R^{b3}$ und $R^{b4}$ jeweils einer Gruppe $R^b$ entsprechen, worin $R^b$ für H steht.

In einer ersten bevorzugten Ausführungsform der Erfindung steht $R^a$ für CN oder $NO_2$.

$R^a$ steht insbesondere für CN, $NO_2$ oder für eine 5- oder 6-gliedrige heteroaromatische Gruppe, wie zuvor definiert, die vorzugsweise entweder 1, 2 oder 3 Stickstoffatome oder 1 Sauerstoff oder 1 Schwefelatom und gegebenenfalls 1 oder 2 Stickstoffatome als Ringlieder aufweist und die unsubstituiert ist oder 1 oder 2 aus $R^{aa}$ und/oder $R^{a1}$ ausgewählte Substituenten aufweisen kann.

In einer weiteren bevorzugten Ausführungsform der Erfindung steht $R^a$ für einen 5-oder 6-gliedrigen Heterocyclus wie zuvor definiert, die vorzugsweise entweder 1, 2, 3 oder 4 Stickstoffatome oder 1 Sauerstoff oder 1 Schwefelatom und gegebenenfalls 1 oder 2 Stickstoffatome als Ringlieder aufweist und die unsubstituiert ist oder 1 oder 2 aus $R^{aa}$ ausgewählte Substituenten aufweisen kann. Bevorzugt sind über N gebundene gesättigte oder teilweise ungesättigte Gruppen, wie z.B.:

Heteroaromatische Gruppen: Pyridazin-3-yl, Pyridazin-4-yl, Pyrimidin-2-yl, Pyrimidin-4-yl, Pyrimidin-5-yl, Pyrazin-2-yl, 2-Furyl, 3-Furyl, 2-Thienyl, 3-Thienyl, Pyrazol-1-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-1-yl, Imidazol-2-yl, Imidazol-4-yl, Oxazol-2-yl, Oxazol-4-yl, Oxazol-5-yl, Thiazol-2-yl, Thiazol-4-yl und Thiazol-5-yl;

In einer anderen Ausgestaltung steht $R^a$ für eine über C-gebundene heteroaromatische Gruppe wie Pyrazol-3-yl, Imidazol-5-yl, Oxazol-2-yl, Thiazol-2-yl, Thiazol-4-yl, Thiazol-5-yl, Pyridin-2-yl, Pyridin-3-yl, Pyridin-4-yl, Pyrimidin-2-yl, Pyrimidin-4-yl, Pyrimidin-5-yl, Pyridazin-4-yl, Pyrazin-2-yl, [1H]-Tetrazol-5-yl und [2H]-Tetrazol-5-yl, wobei die hier exemplarisch genannten Heterocyclen 1 oder 2 aus $R^{aa}$ ausgewählte Substituenten aufweisen können. Bevorzugte Gruppen $R^{aa}$ sind insbesondere F, Cl, CN, $NO_2$, $CH_3$, Ethyl, $OCH_3$, $OC_2H_5$, $OCHF_2$, $OCF_3$ und $CF_3$.

Ebenfalls bevorzugt sind Verbindungen der Formel I.1 und deren Salze, worin $R^a$ für Halogen, insbesondere für Cl oder Br, steht.

In einer weiteren bevorzugten Ausgestaltung steht $R^a$ für $NR^AR^B$, worin $R^A$ und $R^B$ unabhängig voneinander für Wasserstoff, Alkyl, Haloalkyl, Alkenyl, Alkinyl oder Alkoxyalkyl oder Cyanoalkyl stehen.

In einer weiteren bevorzugten Ausgestaltung steht $R^a$ für $C(R^{aa})C(O)R^{a1}$, worin $R^{aa}$ insbesondere für CN oder eine Gruppe $C(O)R^{a1}$ steht und $R^{a1}$ bevorzugt $C_1$-$C_6$-Alkoxy bedeutet.

Sofern $R^a$ Cycloalkyl bedeutet, sind bevorzugte Gruppen Cyclohexyl und, insbesondere, Cyclopropyl.

In einer weiteren bevorzugten Ausgestaltung steht $R^a$ für $C_1$-$C_4$-Alkyl, das durch $C_1$-$C_6$-Alkoxy, $C_3$-$C_8$-Alkenyloxy oder $C_3$-$C_8$-Alkinyloxy substituiert sein kann.

In einer weiteren bevorzugten Ausgestaltung steht $R^a$ für $C_1$-$C_4$-Alkyl, $C_2$-$C_6$-Alkenyl, oder $C_2$-$C_6$-Alkinyl, welche das durch Halogen, CN, $NO_2$ oder $NR^AR^B$ substituiert sein können.

Die Gruppe $R^b$ steht für H. $R^1$ steht vorzugsweise für H, $C_1$-$C_6$-Alkyl, $C_3$-$C_4$-Alkenyl, $C_3$-$C_4$-Alkinyl, besonders bevorzugt für H, $CH_3$, $C_2H_5$, n-Propyl, Allyl, n-Butyl, insbesondere bevorzugt für $CH_3$.

Ebenfalls bevorzugt sind Verbindungen der Formel I.1, worin $R^1$ eine Gruppe $C(=O)R^{11}$ bedeutet, worin $R^{11}$ eine der zuvor genannten Bedeutungen aufweist und insbesondere für H, $C_1$-$C_4$-Alkyl, bevorzugt $CH_3$ oder $C_2H_5$, oder für $C_1$-$C_4$-Haloalkyl, bevorzugt $C_1$-$C_2$-Fluoralkyl wie $CF_3$ steht.

In einer weiteren Ausführung der Erfindung steht $R^1$ für $C_1$-$C_4$-Haloalkyl.

In einer weiteren Ausführung der Erfindung steht $R^1$ für $C_3$-$C_4$-Alkenyl oder $C_3$-$C_4$-Alkinyl, wie $CH_2CH{=}CHCH_3$, $CH_2CH_2CH{=}CH_2$, $CH_2C(CH_3){=}CH_2$, $CH_2C{\equiv}CCH_3$ oder $CH_2CH_2C{\equiv}CH$.

$R^2$ steht vorzugsweise für $CH_3$.

$R^3$ steht vorzugsweise für $C_1$-$C_3$-Alkyl, $C_1$-$C_2$-Fluoralkyl oder $C_2$-$C_3$-Alkenyl, insbesondere für $CH_3$, $C_2H_5$, n-Propyl, $CF_3$, $CH{=}CH_2$ oder 2-Propen-1-yl und bevorzugt für $CH_3$ oder $C_2H_5$.

Unter den Verbindungen der Formel 1.1, in denen $R^9$ für eine von H verschiedene Gruppe steht, sind solche Verbindungen bevorzugt, worin $R^9$ in para-Position zur Gruppen $CR^7R^8$ angeordnet ist.

Unter den Verbindungen der Formel I.1, in denen $R^9$ für eine von H verschiedene Gruppe steht, sind solche Verbindungen bevorzugt, worin $R^9$ in meta-Position zu der Verknüpfungsstelle steht und bevorzugt für Halogen, insbesondere für F oder Cl steht. In einer anderen, ebenfalls bevorzugten Ausführungsform steht $R^9$ für H.

$R^{10}$ steht vorzugsweise für H oder Halogen, wie Cl oder F, insbesondere F. In einer bevorzugten Ausgestaltung steht $R^{10}$ in ortho- oder para-Stellung. Besonders bevorzugt steht $R^{10}$ für H.

In der Gruppe $C(O)R^{11}$ steht $R^{11}$ vorzugsweise für H, $C_1$-$C_4$-Alkyl oder $C_1$-$C_4$-Haloalkyl.

Von den erfindungsgemäßen Verbindungen und deren Salzen sind die Verbindungen der Formel I.A sowie deren landwirtschaftlich geeigneten Salze bevorzugt:

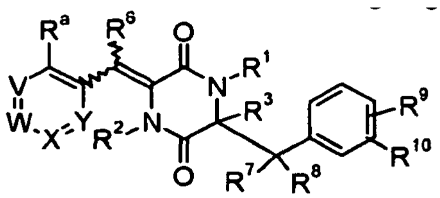

I.A

worin die Variablen eine der angegebenen Bedeutungen, insbesondere die als bevorzugt angegebenen Bedeutungen haben.

In Formel I.1 und insbesondere in Formel I.A und den davon abgeleiteten Unterformeln haben die Gruppen $R^1$, $R^2$, $R^3$, $R^6$, $R^7$, $R^8$, $R^9$, $R^{10}$, $R^a$ und $R^b$ unabhängig voneinander, vorzugsweise jedoch in Kombination, die nachfolgenden Bedeutungen:

$R^1$ H, $CH_3$, $C_2H_5$, $CH_2CH_2CH_3$, $CH_2CH_2CH_2CH_3$, $CH_2C{\equiv}CH$, $CH_2CH{=}CH_2$, insbesondere $CH_3$;
R2 $CH_3$;
$R^3$ $C_1$-$C_4$-Alkyl, OH, $CH_2OH$, $NH_2$, $C(O)R^{11}$, wobei R" für $C_1$-$C_4$-Alkoxy steht, insbesondere für $CH_3$ oder $C_2H_5$;
$R^6$ H;
$R^7$,$R^8$H;
$R^9$ H, Halogen, OH, $C_1$-$C_4$-Alkyl, $C_1$-$C_4$-Alkylcarbonyloxy, insbesondere H oder 3-Halogen, OH, $CH_3$, $OCOCH_3$, speziell H oder 3-F;
$R^{10}$ H oder F;
$R^a$ Halogen, CN, $NO_2$, $C_1$-$C_4$-Alkyl, $C_1$-$C_4$-Alkoxy, $C_1$-$C_4$-Thioalkyl, $NR^AR^B$, Haloalkyl, Haloalkoxy, insbesondere Cl, CN, $NO_2$, $CH_3$, $OCH_3$, $OC_2H_5$, $SCH_3$, und $NR^AR^B$, worin $R^A$ und $R^B$ gemeinsam mit dem N-Atom einen sechsgliedrigen gesättigten Heterocyclus bilden, wie beispielsweise N-Morpholinyl; und
$R^b$ H.

In besonders bevorzugten Ausgestaltungen weisen die Verbindungen I.A die bevorzugten Merkmale der Formel I.1 auf. Sie werden dementsprechend als Formeln I.1A bezeichnet

Eine weitere Ausführungsform der erfindungsgemäßen Verbindungen betrifft solche, wobei $R^4$ und $R^5$ für H stehen. Solche Verbindungen entsprechen der Formel I.B

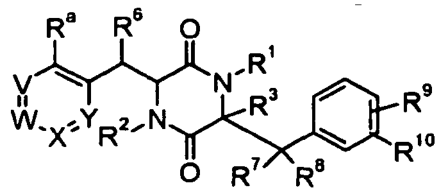
I.B

In besonders bevorzugten Ausgestaltungen weisen die Verbindungen I.B die bevorzugten Merkmale der Formel I.1 auf. Sie werden dementsprechend als Formel I.1B bezeichnet.

Die erfindungsgemäßen Verbindungen weisen am Kohlenstoffatom, welches die Gruppe $R^3$ trägt, ein Chiralitätszentrum auf. Eine bevorzugte Ausführungsform der Erfindung betrifft die reinen Enantiomere der im Folgenden angegebenen Formel I-S,

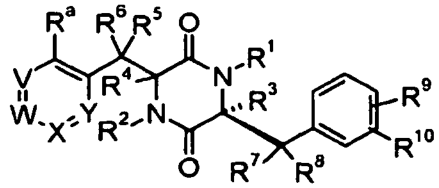
I-S

worin die Variablen eine der zuvor angegebenen Bedeutungen, insbesondere eine der als bevorzugt oder als besonders bevorzugt angegebenen Bedeutungen aufweisen, sowie Enantiomerenmischungen, die einen Enantiomerenüberschuss bezüglich des Enantiomers der Formel I-S aufweisen.

In besonders bevorzugten Ausgestaltungen weisen die Verbindungen I-S die bevorzugten Merkmale der Formeln I.1 auf. Sie werden dementsprechend als Formel I.1-S bezeichnet.

Sofern $R^4$ nicht eine Bindung mit $R^5$ darstellt, weisen die erfindungsgemäßen Verbindungen auch an dem Kohlenstoffatom, welches die Gruppe $R^4$ trägt, ein Chiralitätszentrum auf. Die S-Konfiguration an dieser Position ist für die Verbindungen der Formel I.1, insbesondere derjenigen der Formel I-S, bevorzugt.

Enantiomerenüberschuss bedeutet bevorzugt einen ee-Wert (enantiomeric excess) von wenigstens 70 %, insbesondere wenigstens 80 % und bevorzugt wenigstens 90 %. Ebenso bevorzugt sind die landwirtschaftlich geeigneten Salze der Enantiomere I-S und Enantiomerenmischungen der Salze, die einen Enantiomerenüberschuss bezüglich des Enantiomers der Formel I-S aufweisen.

Eine andere, ebenfalls bevorzugte Ausführungsform betrifft die Racemate von I und deren Salze.

Eine besonders bevorzugte Ausführungsform betrifft die reinen Enantiomere der im Folgenden angegebenen Formel I.A-S, worin die Variablen eine der zuvor angegebenen Bedeutungen, insbesondere eine der als bevorzugt oder als besonders bevorzugt angegebenen Bedeutungen aufweisen, sowie Enantiomerenmischungen, die einen Enantiomerenüberschuss bezüglich des Enantiomers der Formel I.A-S aufweisen.

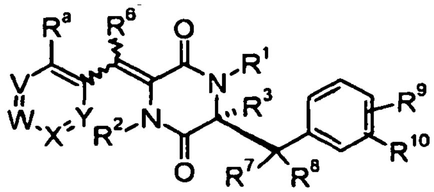
I.A-S

Ebenso bevorzugt sind die landwirtschaftlich geeigneten Salze der Enantiomere I.A S und Enantiomerenmischungen der Salze, die einen Enantiomerenüberschuss bezüglich des Enantiomers der Formel I.A-S aufweisen.

Eine andere besonders bevorzugte Ausführungsform der Erfindung betrifft die Racemate von I.A und deren Salze.

Aus den Verbindungen der Formeln I.A und den davon abgeleiteten Unterformeln sind solche Verbindungen bevorzugt, worin die exo-Doppelbindung am Piperazinring die (Z)-Konfiguration aufweist. Ebenso bevorzugt Gemische des (E)-Isomers mit dem (Z)-Isomer, worin das Z-Isomer im Überschuss vorliegt, insbesondere Isomerengemische mit

einem E/Z-Verhältnis, von nicht mehr als 1:2 insbesondere nicht mehr als 1:5.

Insbesondere sind im Hinblick auf ihre Verwendung die in den folgenden Tabellen zusammengestellten Verbindungen der Formel I.1, welche den Formeln I.1A', bzw. I.1B' entsprechen, bevorzugt.

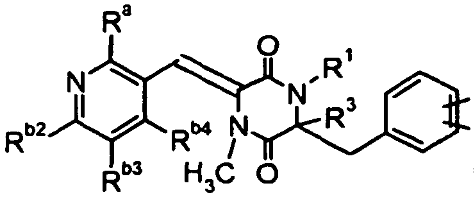
I.1A'

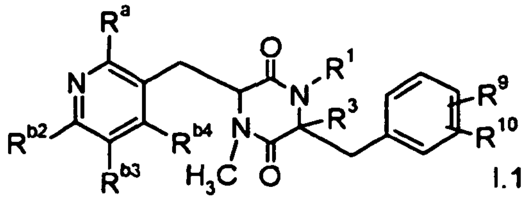
I.1B'

Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

Tabelle 1

Verbindungen der Formel I.1, in denen $R^a$ CN, $R^{b2}$, $R^{b3}$ und $R^{b4}$ H bedeuten und die Kombination von $R^1$, $R^3$, $R^9$ und $R^{10}$ für eine Verbindung jeweils einer Zeile der Tabelle A entspricht

Tabelle 2

Verbindungen der Formel I.1, in denen $R^a$ Cl, $R^{b2}$, $R^{b3}$ und $R^{b4}$ H bedeuten und die Kombination von $R^1$, $R^3$, $R^9$ und $R^{10}$ für eine Verbindung jeweils einer Zeile der Tabelle A entspricht

Tabelle 3

Verbindungen der Formel I.1, in denen $R^a$ $NO_2$, $R^{b2}$, $R^{b3}$ und $R^{b4}$ H bedeuten und die Kombination von $R^1$, $R^3$, $R^9$ und $R^{10}$ für eine Verbindung jeweils einer Zeile der Tabelle A entspricht

Tabelle 4

Verbindungen der Formel I.1, in denen $R^a$ $CH_3$, $R^{b2}$ und $R^{b3}$ H und $R^{b4}$ H bedeuten und die Kombination von $R^1$, $R^3$, $R^9$ und $R^{10}$ für eine Verbindung jeweils einer Zeile der Tabelle A entspricht

Tabelle 5

Verbindungen der Formel I.1, in denen $R^a$ $CF_3$, $R^{b2}$ und $R^{b3}$ H und $R^{b4}$ H bedeuten und die Kombination von $R^1$, $R^3$, $R^9$ und $R^{10}$ für eine Verbindung jeweils einer Zeile der Tabelle A entspricht

Tabelle 6

Verbindungen der Formel I.1, in denen $R^a$ $OCH_3$, $R^{b2}$ und $R^{b3}$ H und $R^{b4}$ H bedeuten und die Kombination von $R^1$, $R^3$, $R^9$ und $R^{10}$ für eine Verbindung jeweils einer Zeile der Tabelle A entspricht

Tabelle 7

Verbindungen der Formel I.1, in denen $R^a$ $OC_2H_5$, $R^{b2}$ und $R^{b3}$ H und $R^{b4}$ H bedeuten und die Kombination von $R^1$, $R^3$, $R^9$ und $R^{10}$ für eine Verbindung jeweils einer Zeile der Tabelle A entspricht

Tabelle 8

Verbindungen der Formel I.1, in denen $R^a$ Morpholin-1-yl, $R^{b2}$ und $R^{b3}$ H und $R^{b4}$ H bedeuten und die Kombination von $R^1$, $R^3$, $R^9$ und $R^{10}$ für eine Verbindung jeweils einer Zeile der Tabelle A entspricht

Tabelle A Verbindungen der Formel I.1, welche den Formeln I.1A', bzw. I.1B' entsprechen

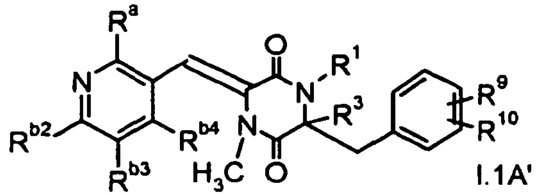

I.1A'

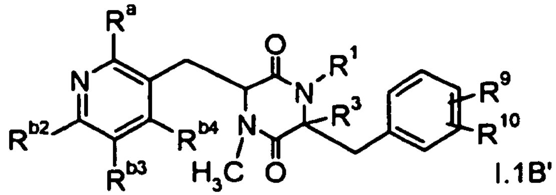

I.1B'

| Nr. | Formel | $R^1$ | $R^3$ | $R^9$ | $R^{10}$ |
|---|---|---|---|---|---|
| A-1 | I.1A' | H | $CH_3$ | H | H |
| A-2 | I.1A' | $CH_3$ | $CH_3$ | H | H |
| A-3 | I.1A' | $CH_2CH_3$ | $CH_3$ | H | H |
| A-4 | I.1A' | $CH_2CH_2CH_3$ | $CH_3$ | H | H |
| A-5 | I.1A' | $CH_2CH_2CH_2CH_3$ | $CH_3$ | H | H |
| A-6 | I.1A' | $CH_2CH=CH_2$ | $CH_3$ | H | H |
| A-7 | I.1A' | $CH_2C\equiv CH$ | $CH_3$ | H | H |
| A-8 | I.1A' | H | $CH_2CH_3$ | H | H |
| A-9 | I.1A' | $CH_3$ | $CH_2CH_3$ | H | H |
| A-10 | I.1A' | $CH_2CH_3$ | $CH_2CH_3$ | H | H |
| A-11 | I.1A' | $CH_2CH_2CH_3$ | $CH_2CH_3$ | H | H |
| A-12 | I.1A' | $CH_2CH_2CH_2CH_3$ | $CH_2CH_3$ | H | H |
| A-13 | I.1A' | $CH_2CH=CH_2$ | $CH_2CH_3$ | H | H |
| A-14 | I.1A' | $CH_2C\equiv CH$ | $CH_2CH_3$ | H | H |
| A-15 | I.1A' | H | OH | H | H |
| A-16 | I.1A' | $CH_3$ | OH | H | H |
| A-17 | I.1A' | $CH_2CH_3$ | OH | H | H |
| A-18 | I.1A' | $CH_2CH_2CH_3$ | OH | H | H |
| A-19 | I.1A' | $CH_2CH_2CH_2CH_3$ | OH | H | H |

(fortgesetzt)

| Nr. | Formel | $R^1$ | $R^3$ | $R^9$ | $R^{10}$ |
|---|---|---|---|---|---|
| A-20 | I.1A' | $CH_2CH=CH_2$ | OH | H | H |
| A-21 | I.1A' | $CH_2C\equiv CH$ | OH | H | H |
| A-22 | I.1A' | H | $CH_2OH$ | H | H |
| A-23 | I.1A' | $CH_3$ | $CH_2OH$ | H | H |
| A-24 | I.1A' | $CH_2CH_3$ | $CH_2OH$ | H | H |
| A-25 | I.1A' | $CH_2CH_2CH_3$ | $CH_2OH$ | H | H |
| A-26 | I.1A' | $CH_2CH_2CH_2CH_3$ | $CH_2OH$ | H | H |
| A-27 | I.1A' | $CH_2CH=CH_2$ | $CH_2OH$ | H | H |
| A-28 | I.1A' | $CH_2C\equiv CH$ | $CH_2OH$ | H | H |
| A-29 | I.1A' | H | $NH_2$ | H | H |
| A-30 | I.1A' | $CH_3$ | $NH_2$ | H | H |
| A-31 | I.1A' | $CH_2CH_3$ | $NH_2$ | H | H |
| A-32 | I.1A' | $CH_2CH_2CH_3$ | $NH_2$ | H | H |
| A-33 | I.1A' | $CH_2CH_2CH_2CH_3$ | $NH_2$ | H | H |
| A-34 | I.1A' | $CH_2CH=CH_2$ | $NH_2$ | H | H |
| A-35 | I.1A' | $CH_2C\equiv CH$ | $NH_2$ | H | H |
| A-36 | I.1A' | H | $COOCH_3$ | H | H |
| A-37 | I.1A' | $CH_3$ | $COOCH_3$ | H | H |
| A-38 | I.1A' | $CH_2CH_3$ | $COOCH_3$ | H | H |
| A-39 | I.1A' | $CH_2CH_2CH_3$ | $COOCH_3$ | H | H |
| A-40 | I.1A' | $CH_2CH_2CH_2CH_3$ | $COOCH_3$ | H | H |
| A-41 | I.1A' | $CH_2CH=CH_2$ | $COOCH_3$ | H | H |
| A-42 | I.1A' | $CH_2C\equiv CH$ | $COOCH_3$ | H | H |
| A-43 | I.1A' | H | $CH_3$ | 2-F | H |
| A-44 | I.1A' | $CH_3$ | $CH_3$ | 2-F | H |

(fortgesetzt)

| Nr. | Formel | $R^1$ | $R^3$ | $R^9$ | $R^{10}$ |
|---|---|---|---|---|---|
| A-45 | I.1A' | $CH_2CH_3$ | $CH_3$ | 2-F | H |
| A-46 | I.1A' | $CH_2CH_2CH_3$ | $CH_3$ | 2-F | H |
| A-47 | I.1A' | $CH_2CH_2CH_2CH_3$ | $CH_3$ | 2-F | H |
| A-48 | I.1A' | $CH_2CH=CH_2$ | $CH_3$ | 2-F | H |
| A-49 | I.1A' | $CH_2C\equiv CH$ | $CH_3$ | 2-F | H |
| A-50 | I.1A' | H | $CH_2CH_3$ | 2-F | H |
| A-51 | I.1A' | $CH_3$ | $CH_2CH_3$ | 2-F | H |
| A-52 | I.1A' | $CH_2CH_3$ | $CH_2CH_3$ | 2-F | H |
| A-53 | I.1A' | $CH_2CH_2CH_3$ | $CH_2CH_3$ | 2-F | H |
| A-54 | I.1A' | $CH_2CH_2CH_2CH_3$ | $CH_2CH_3$ | 2-F | H |
| A-55 | I.1A' | $CH_2CH=CH_2$ | $CH_2CH_3$ | 2-F | H |
| A-56 | I.1A' | $CH_2C\equiv CH$ | $CH_2CH_3$ | 2-F | H |
| A-57 | I.1A' | H | OH | 2-F | H |
| A-58 | I.1A' | $CH_3$ | OH | 2-F | H |
| A-59 | I.1A' | $CH_2CH_3$ | OH | 2-F | H |
| A-60 | I.1A' | $CH_2CH_2CH_3$ | OH | 2-F | H |
| A-61 | I.1A' | $CH_2CH_2CH_2CH_3$ | OH | 2-F | H |
| A-62 | I.1A' | $CH_2CH=CH_2$ | OH | 2-F | H |
| A-63 | I.1A' | $CH_2C\equiv CH$ | OH | 2-F | H |
| A-64 | I.1A' | H | $CH_2OH$ | 2-F | H |
| A-65 | I.1A' | $CH_3$ | $CH_2OH$ | 2-F | H |
| A-66 | I.1A' | $CH_2CH_3$ | $CH_2OH$ | 2-F | H |
| A-67 | I.1A' | $CH_2CH_2CH_3$ | $CH_2OH$ | 2-F | H |
| A-68 | I.1A' | $CH_2CH_2CH_2CH_3$ | $CH_2OH$ | 2-F | H |
| A-69 | I.1A' | $CH_2CH=CH_2$ | $CH_2OH$ | 2-F | H |

(fortgesetzt)

| Nr. | Formel | $R^1$ | $R^3$ | $R^9$ | $R^{10}$ |
|---|---|---|---|---|---|
| A-70 | I.1A' | $CH_2C{\equiv}CH$ | $CH_2OH$ | 2-F | H |
| A-71 | I.1A' | H | $NH_2$ | 2-F | H |
| A-72 | I.1A' | $CH_3$ | $NH_2$ | 2-F | H |
| A-73 | I.1A' | $CH_2CH_3$ | $NH_2$ | 2-F | H |
| A-74 | I.1A' | $CH_2CH_2CH_3$ | $NH_2$ | 2-F | H |
| A-75 | I.1A' | $CH_2CH_2CH_2CH_3$ | $NH_2$ | 2-F | H |
| A-76 | I.1A' | $CH_2CH{=}CH_2$ | $NH_2$ | 2-F | H |
| A-77 | I.1A' | $CH_2C{\equiv}CH$ | $NH_2$ | 2-F | H |
| A-78 | I.1A' | H | $COOCH_3$ | 2-F | H |
| A-79 | I.1A' | $CH_3$ | $COOCH_3$ | 2-F | H |
| A-80 | I.1A' | $CH_2CH_3$ | $COOCH_3$ | 2-F | H |
| A-81 | I.1A' | $CH_2CH_2CH_3$ | $COOCH_3$ | 2-F | H |
| A-82 | I.1A' | $CH_2CH_2CH_2CH_3$ | $COOCH_3$ | 2-F | H |
| A-83 | I.1A' | $CH_2CH{=}CH_2$ | $COOCH_3$ | 2-F | H |
| A-84 | I.1A' | $CH_2C{\equiv}CH$ | $COOCH_3$ | 2-F | H |
| A-85 | I.1A' | H | $CH_3$ | 3-F | H |
| A-86 | I.1A' | $CH_3$ | $CH_3$ | 3-F | H |
| A-87 | I.1A' | $CH_2CH_3$ | $CH_3$ | 3-F | H |
| A-88 | I.1A' | $CH_2CH_2CH_3$ | $CH_3$ | 3-F | H |
| A-89 | I.1A' | $CH_2CH_2CH_2CH_3$ | $CH_3$ | 3-F | H |
| A-90 | I.1A' | $CH_2CH{=}CH_2$ | $CH_3$ | 3-F | H |
| A-91 | I.1A' | $CH_2C{\equiv}CH$ | $CH_3$ | 3-F | H |
| A-92 | I.1A' | H | $CH_2CH_3$ | 3-F | H |
| A-93 | I.1A' | $CH_3$ | $CH_2CH_3$ | 3-F | H |
| A-94 | I.1A' | $CH_2CH_3$ | $CH_2CH_3$ | 3-F | H |

(fortgesetzt)

| Nr. | Formel | $R^1$ | $R^3$ | $R^9$ | $R^{10}$ |
|---|---|---|---|---|---|
| A-95 | I.1A' | $CH_2CH_2CH_3$ | $CH_2CH_3$ | 3-F | H |
| A-96 | I.1A' | $CH_2CH_2CH_2CH_3$ | $CH_2CH_3$ | 3-F | H |
| A-97 | I.1A' | $CH_2CH=CH_2$ | $CH_2CH_3$ | 3-F | H |
| A-98 | I.1A' | $CH_2C\equiv CH$ | $CH_2CH_3$ | 3-F | H |
| A-99 | I.1A' | H | OH | 3-F | H |
| A-100 | I.1A' | $CH_3$ | OH | 3-F | H |
| A-101 | I.1A' | $CH_2CH_3$ | OH | 3-F | H |
| A-102 | I.1A' | $CH_2CH_2CH_3$ | OH | 3-F | H |
| A-103 | I.1A' | $CH_2CH_2CH_2CH_3$ | OH | 3-F | H |
| A-104 | I.1A' | $CH_2CH=CH_2$ | OH | 3-F | H |
| A-105 | I.1A' | $CH_2C\equiv CH$ | OH | 3-F | H |
| A-106 | I.1A' | H | $CH_2OH$ | 3-F | H |
| A-107 | I.1A' | $CH_3$ | $CH_2OH$ | 3-F | H |
| A-108 | I.1A' | $CH_2CH_3$ | $CH_2OH$ | 3-F | H |
| A-109 | I.1A' | $CH_2CH_2CH_3$ | $CH_2OH$ | 3-F | H |
| A-110 | I.1A' | $CH_2CH_2CH_2CH_3$ | $CH_2OH$ | 3-F | H |
| A-111 | I.1A' | $CH_2CH=CH_2$ | $CH_2OH$ | 3-F | H |
| A-112 | I.1A' | $CH_2C\equiv CH$ | $CH_2OH$ | 3-F | H |
| A-113 | I.1A' | H | $NH_2$ | 3-F | H |
| A-114 | I.1A' | $CH_3$ | $NH_2$ | 3-F | H |
| A-115 | I.1A' | $CH_2CH_3$ | $NH_2$ | 3-F | H |
| A-116 | I.1A' | $CH_2CH_2CH_3$ | $NH_2$ | 3-F | H |
| A-117 | I.1A' | $CH_2CH_2CH_2CH_3$ | $NH_2$ | 3-F | H |
| A-118 | I.1A' | $CH_2CH=CH_2$ | $NH_2$ | 3-F | H |
| A-119 | I.1A' | $CH_2C\equiv CH$ | $NH_2$ | 3-F | H |

(fortgesetzt)

| Nr. | Formel | $R^1$ | $R^3$ | $R^9$ | $R^{10}$ |
|---|---|---|---|---|---|
| A-120 | I.1A’ | H | $COOCH_3$ | 3-F | H |
| A-121 | I.1A’ | $CH_3$ | $COOCH_3$ | 3-F | H |
| A-122 | I.1A’ | $CH_2CH_3$ | $COOCH_3$ | 3-F | H |
| A-123 | I.1A’ | $CH_2CH_2CH_3$ | $COOCH_3$ | 3-F | H |
| A-124 | I.1A’ | $CH_2CH_2CH_2CH_3$ | $COOCH_3$ | 3-F | H |
| A-125 | I.1A’ | $CH_2CH=CH_2$ | $COOCH_3$ | 3-F | H |
| A-126 | I.1A’ | $CH_2C\equiv CH$ | $COOCH_3$ | 3-F | H |
| A-127 | I.1A’ | H | $CH_3$ | 4-F | H |
| A-128 | I.1A’ | $CH_3$ | $CH_3$ | 4-F | H |
| A-129 | I.1A’ | $CH_2CH_3$ | $CH_3$ | 4-F | H |
| A-130 | I.1A’ | $CH_2CH_2CH_3$ | $CH_3$ | 4-F | H |
| A-131 | I.1A’ | $CH_2CH_2CH_2CH_3$ | $CH_3$ | 4-F | H |
| A-132 | I.1A’ | $CH_2CH=CH_2$ | $CH_3$ | 4-F | H |
| A-133 | I.1A’ | $CH_2C\equiv CH$ | $CH_3$ | 4-F | H |
| A-134 | I.1A’ | H | $CH_2CH_3$ | 4-F | H |
| A-135 | I.1A’ | $CH_3$ | $CH_2CH_3$ | 4-F | H |
| A-136 | I.1A’ | $CH_2CH_3$ | $CH_2CH_3$ | 4-F | H |
| A-137 | I.1A’ | $CH_2CH_2CH_3$ | $CH_2CH_3$ | 4-F | H |
| A-138 | I.1A’ | $CH_2CH_2CH_2CH_3$ | $CH_2CH_3$ | 4-F | H |
| A-139 | I.1A’ | $CH_2CH=CH_2$ | $CH_2CH_3$ | 4-F | H |
| A-140 | I.1A’ | $CH_2C\equiv CH$ | $CH_2CH_3$ | 4-F | H |
| A-141 | I.1A’ | H | OH | 4-F | H |
| A-142 | I.1A’ | $CH_3$ | OH | 4-F | H |
| A-143 | I.1A’ | $CH_2CH_3$ | OH | 4-F | H |
| A-144 | I.1A’ | $CH_2CH_2CH_3$ | OH | 4-F | H |

(fortgesetzt)

| Nr. | Formel | $R^1$ | $R^3$ | $R^9$ | $R^{10}$ |
|---|---|---|---|---|---|
| A-145 | I.1A' | $CH_2CH_2CH_2CH_3$ | OH | 4-F | H |
| A-146 | I.1A' | $CH_2CH=CH_2$ | OH | 4-F | H |
| A-147 | I.1A' | $CH_2C\equiv CH$ | OH | 4-F | H |
| A-148 | I.1A' | H | $CH_2OH$ | 4-F | H |
| A-149 | I.1A' | $CH_3$ | $CH_2OH$ | 4-F | H |
| A-150 | I.1A' | $CH_2CH_3$ | $CH_2OH$ | 4-F | H |
| A-151 | I.1A' | $CH_2CH_2CH_3$ | $CH_2OH$ | 4-F | H |
| A-152 | I.1A' | $CH_2CH_2CH_2CH_3$ | $CH_2OH$ | 4-F | H |
| A-153 | I.1A' | $CH_2CH=CH_2$ | $CH_2OH$ | 4-F | H |
| A-154 | I.1A' | $CH_2C\equiv CH$ | $CH_2OH$ | 4-F | H |
| A-155 | I.1A' | H | $NH_2$ | 4-F | H |
| A-156 | I.1A' | $CH_3$ | $NH_2$ | 4-F | H |
| A-157 | I.1A' | $CH_2CH_3$ | $NH_2$ | 4-F | H |
| A-158 | I.1A' | $CH_2CH_2CH_3$ | $NH_2$ | 4-F | H |
| A-159 | I.1A' | $CH_2CH_2CH_2CH_3$ | $NH_2$ | 4-F | H |
| A-160 | I.1A' | $CH_2CH=CH_2$ | $NH_2$ | 4-F | H |
| A-161 | I.1A' | $CH_2C\equiv CH$ | $NH_2$ | 4-F | H |
| A-162 | I.1A' | H | $COOCH_3$ | 4-F | H |
| A-163 | I.1A' | $CH_3$ | $COOCH_3$ | 4-F | H |
| A-164 | I.1A' | $CH_2CH_3$ | $COOCH_3$ | 4-F | H |
| A-165 | I.1A' | $CH_2CH_2CH_3$ | $COOCH_3$ | 4-F | H |
| A-166 | I.1A' | $CH_2CH_2CH_2CH_3$ | $COOCH_3$ | 4-F | H |
| A-167 | I.1A' | $CH_2CH=CH_2$ | $COOCH_3$ | 4-F | H |
| A-168 | I.1A' | $CH_2C\equiv CH$ | $COOCH_3$ | 4-F | H |
| A-169 | I.1A' | H | $CH_3$ | 3-F | 2-F |

(fortgesetzt)

| Nr. | Formel | $R^1$ | $R^3$ | $R^9$ | $R^{10}$ |
|---|---|---|---|---|---|
| A-170 | I.1A' | $CH_3$ | $CH_3$ | 3-F | 2-F |
| A-171 | I.1A' | $CH_2CH_3$ | $CH_3$ | 3-F | 2-F |
| A-172 | I.1A' | $CH_2CH_2CH_3$ | $CH_3$ | 3-F | 2-F |
| A-173 | I.1A' | $CH_2CH_2CH_2CH_3$ | $CH_3$ | 3-F | 2-F |
| A-174 | I.1A' | $CH_2CH=CH_2$ | $CH_3$ | 3-F | 2-F |
| A-175 | I.1A' | $CH_2C\equiv CH$ | $CH_3$ | 3-F | 2-F |
| A-176 | I.1A' | H | $CH_2CH_3$ | 3-F | 2-F |
| A-177 | I.1A' | $CH_3$ | $CH_2CH_3$ | 3-F | 2-F |
| A-178 | I.1A' | $CH_2CH_3$ | $CH_2CH_3$ | 3-F | 2-F |
| A-179 | I.1A' | $CH_2CH_2CH_3$ | $CH_2CH_3$ | 3-F | 2-F |
| A-180 | I.1A' | $CH_2CH_2CH_2CH_3$ | $CH_2CH_3$ | 3-F | 2-F |
| A-181 | I.1A' | $CH_2CH=CH_2$ | $CH_2CH_3$ | 3-F | 2-F |
| A-182 | I.1A' | $CH_2C\equiv CH$ | $CH_2CH_3$ | 3-F | 2-F |
| A-183 | I.1A' | H | OH | 3-F | 2-F |
| A-184 | I.1A' | $CH_3$ | OH | 3-F | 2-F |
| A-185 | I.1A' | $CH_2CH_3$ | OH | 3-F | 2-F |
| A-186 | I.1A' | $CH_2CH_2CH_3$ | OH | 3-F | 2-F |
| A-187 | I.1A' | $CH_2CH_2CH_2CH_3$ | OH | 3-F | 2-F |
| A-188 | I.1A' | $CH_2CH=CH_2$ | OH | 3-F | 2-F |
| A-189 | I.1A' | $CH_2C\equiv CH$ | OH | 3-F | 2-F |
| A-190 | I.1A' | H | $CH_2OH$ | 3-F | 2-F |
| A-191 | I.1A' | $CH_3$ | $CH_2OH$ | 3-F | 2-F |
| A-192 | I.1A' | $CH_2CH_3$ | $CH_2OH$ | 3-F | 2-F |
| A-193 | I.1A' | $CH_2CH_2CH_3$ | $CH_2OH$ | 3-F | 2-F |
| A-194 | I.1A' | $CH_2CH_2CH_2CH_3$ | $CH_2OH$ | 3-F | 2-F |

(fortgesetzt)

| Nr. | Formel | $R^1$ | $R^3$ | $R^9$ | $R^{10}$ |
|---|---|---|---|---|---|
| A-195 | I.1A' | $CH_2CH=CH_2$ | $CH_2OH$ | 3-F | 2-F |
| A-196 | I.1A' | $CH_2C\equiv CH$ | $CH_2OH$ | 3-F | 2-F |
| A-197 | I.1A' | H | $NH_2$ | 3-F | 2-F |
| A-198 | I.1A' | $CH_3$ | $NH_2$ | 3-F | 2-F |
| A-199 | I.1A' | $CH_2CH_3$ | $NH_2$ | 3-F | 2-F |
| A-200 | I.1A' | $CH_2CH_2CH_3$ | $NH_2$ | 3-F | 2-F |
| A-201 | I.1A' | $CH_2CH_2CH_2CH_3$ | $NH_2$ | 3-F | 2-F |
| A-202 | I.1A' | $CH_2CH=CH_2$ | $NH_2$ | 3-F | 2-F |
| A-203 | I.1A' | $CH_2C\equiv CH$ | $NH_2$ | 3-F | 2-F |
| A-204 | I.1A' | H | $COOCH_3$ | 3-F | 2-F |
| A-205 | I.1A' | $CH_3$ | $COOCH_3$ | 3-F | 2-F |
| A-206 | I.1A' | $CH_2CH_3$ | $COOCH_3$ | 3-F | 2-F |
| A-207 | I.1A' | $CH_2CH_2CH_3$ | $COOCH_3$ | 3-F | 2-F |
| A-208 | I.1A' | $CH_2CH_2CH_2CH_3$ | $COOCH_3$ | 3-F | 2-F |
| A-209 | I.1A' | $CH_2CH=CH_2$ | $COOCH_3$ | 3-F | 2-F |
| A-210 | I.1A' | $CH_2C\equiv CH$ | $COOCH_3$ | 3-F | 2-F |
| A-211 | I.1A' | H | $CH_3$ | 3-F | 4-F |
| A-212 | I.1A' | $CH_3$ | $CH_3$ | 3-F | 4-F |
| A-213 | I.1A' | $CH_2CH_3$ | $CH_3$ | 3-F | 4-F |
| A-214 | I.1A' | $CH_2CH_2CH_3$ | $CH_3$ | 3-F | 4-F |
| A-215 | I.1A' | $CH_2CH_2CH_2CH_3$ | $CH_3$ | 3-F | 4-F |
| A-216 | I.1A' | $CH_2CH=CH_2$ | $CH_3$ | 3-F | 4-F |
| A-217 | I.1A' | $CH_2C\equiv CH$ | $CH_3$ | 3-F | 4-F |
| A-218 | I.1A' | H | $CH_2CH_3$ | 3-F | 4-F |
| A-219 | I.1A' | $CH_3$ | $CH_2CH_3$ | 3-F | 4-F |

(fortgesetzt)

| Nr. | Formel | $R^1$ | $R^3$ | $R^9$ | $R^{10}$ |
|---|---|---|---|---|---|
| A-220 | I.1A' | $CH_2CH_3$ | $CH_2CH_3$ | 3-F | 4-F |
| A-221 | I.1A' | $CH_2CH_2CH_3$ | $CH_2CH_3$ | 3-F | 4-F |
| A-222 | I.1A' | $CH_2CH_2CH_2CH_3$ | $CH_2CH_3$ | 3-F | 4-F |
| A-223 | I.1A' | $CH_2CH{=}CH_2$ | $CH_2CH_3$ | 3-F | 4-F |
| A-224 | I.1A' | $CH_2C{\equiv}CH$ | $CH_2CH_3$ | 3-F | 4-F |
| A-225 | I.1A' | H | OH | 3-F | 4-F |
| A-226 | I.1A' | $CH_3$ | OH | 3-F | 4-F |
| A-227 | I.1A' | $CH_2CH_3$ | OH | 3-F | 4-F |
| A-228 | I.1A' | $CH_2CH_2CH_3$ | OH | 3-F | 4-F |
| A-229 | I.1A' | $CH_2CH_2CH_2CH_3$ | OH | 3-F | 4-F |
| A-230 | I.1A' | $CH_2CH{=}CH_2$ | OH | 3-F | 4-F |
| A-231 | I.1A' | $CH_2C{\equiv}CH$ | OH | 3-F | 4-F |
| A-232 | I.1A' | H | $CH_2OH$ | 3-F | 4-F |
| A-233 | I.1A' | $CH_3$ | $CH_2OH$ | 3-F | 4-F |
| A-234 | I.1A' | $CH_2CH_3$ | $CH_2OH$ | 3-F | 4-F |
| A-235 | I.1A' | $CH_2CH_2CH_3$ | $CH_2OH$ | 3-F | 4-F |
| A-236 | I.1A' | $CH_2CH_2CH_2CH_3$ | $CH_2OH$ | 3-F | 4-F |
| A-237 | I.1A' | $CH_2CH{=}CH_2$ | $CH_2OH$ | 3-F | 4-F |
| A-238 | I.1A' | $CH_2C{\equiv}CH$ | $CH_2OH$ | 3-F | 4-F |
| A-239 | I.1A' | H | $NH_2$ | 3-F | 4-F |
| A-240 | I.1A' | $CH_3$ | $NH_2$ | 3-F | 4-F |
| A-241 | I.1A' | $CH_2CH_3$ | $NH_2$ | 3-F | 4-F |
| A-242 | I.1A' | $CH_2CH_2CH_3$ | $NH_2$ | 3-F | 4-F |
| A-243 | I.1A' | $CH_2CH_2CH_2CH_3$ | $NH_2$ | 3-F | 4-F |
| A-244 | I.1A' | $CH_2CH{=}CH_2$ | $NH_2$ | 3-F | 4-F |

(fortgesetzt)

| Nr. | Formel | $R^1$ | $R^3$ | $R^9$ | $R^{10}$ |
|---|---|---|---|---|---|
| A-245 | I.1A' | $CH_2C{\equiv}CH$ | $NH_2$ | 3-F | 4-F |
| A-246 | I.1A' | H | $COOCH_3$ | 3-F | 4-F |
| A-247 | I.1A' | $CH_3$ | $COOCH_3$ | 3-F | 4-F |
| A-248 | I.1A' | $CH_2CH_3$ | $COOCH_3$ | 3-F | 4-F |
| A-249 | I.1A' | $CH_2CH_2CH_3$ | $COOCH_3$ | 3-F | 4-F |
| A-250 | I.1A' | $CH_2CH_2CH_2CH_3$ | $COOCH_3$ | 3-F | 4-F |
| A-251 | I.1A' | $CH_2CH{=}CH_2$ | $COOCH_3$ | 3-F | 4-F |
| A-252 | I.1A' | $CH_2C{\equiv}CH$ | $COOCH_3$ | 3-F | 4-F |
| A-253 | I.1A' | H | $CH_3$ | 3-$CH_3$ | H |
| A-254 | I.1A' | $CH_3$ | $CH_3$ | 3-$CH_3$ | H |
| A-255 | I.1A' | $CH_2CH_3$ | $CH_3$ | 3-$CH_3$ | H |
| A-256 | I.1A' | $CH_2CH_2CH_3$ | $CH_3$ | 3-$CH_3$ | H |
| A-257 | I.1A' | $CH_2CH_2CH_2CH_3$ | $CH_3$ | 3-$CH_3$ | H |
| A-258 | I.1A' | $CH_2CH{=}CH_2$ | $CH_3$ | 3-$CH_3$ | H |
| A-259 | I.1A' | $CH_2C{\equiv}CH$ | $CH_3$ | 3-$CH_3$ | H |
| A-260 | I.1A' | H | $CH_2CH_3$ | 3-$CH_3$ | H |
| A-261 | I.1A' | $CH_3$ | $CH_2CH_3$ | 3-$CH_3$ | H |
| A-262 | I.1A' | $CH_2CH_3$ | $CH_2CH_3$ | 3-$CH_3$ | H |
| A-263 | I.1A' | $CH_2CH_2CH_3$ | $CH_2CH_3$ | 3-$CH_3$ | H |
| A-264 | I.1A' | $CH_2CH_2CH_2CH_3$ | $CH_2CH_3$ | 3-$CH_3$ | H |
| A-265 | I.1A' | $CH_2CH{=}CH_2$ | $CH_2CH_3$ | 3-$CH_3$ | H |
| A-266 | I.1A' | $CH_2C{\equiv}CH$ | $CH_2CH_3$ | 3-$CH_3$ | H |
| A-267 | I.1A' | H | OH | 3-$CH_3$ | H |
| A-268 | I.1A' | $CH_3$ | OH | 3-$CH_3$ | H |
| A-269 | I.1A' | $CH_2CH_3$ | OH | 3-$CH_3$ | H |

(fortgesetzt)

| Nr. | Formel | $R^1$ | $R^3$ | $R^9$ | $R^{10}$ |
|---|---|---|---|---|---|
| A-270 | I.1A' | $CH_2CH_2CH_3$ | OH | 3-$CH_3$ | H |
| A-271 | I.1A' | $CH_2CH_2CH_2CH_3$ | OH | 3-$CH_3$ | H |
| A-272 | I.1A' | $CH_2CH{=}CH_2$ | OH | 3-$CH_3$ | H |
| A-273 | I.1A' | $CH_2C{\equiv}CH$ | OH | 3-$CH_3$ | H |
| A-274 | I.1A' | H | $CH_2OH$ | 3-$CH_3$ | H |
| A-275 | I.1A' | $CH_3$ | $CH_2OH$ | 3-$CH_3$ | H |
| A-276 | I.1A' | $CH_2CH_3$ | $CH_2OH$ | 3-$CH_3$ | H |
| A-277 | I.1A' | $CH_2CH_2CH_3$ | $CH_2OH$ | 3-$CH_3$ | H |
| A-278 | I.1A' | $CH_2CH_2CH_2CH_3$ | $CH_2OH$ | 3-$CH_3$ | H |
| A-279 | I.1A' | $CH_2CH{=}CH_2$ | $CH_2OH$ | 3-$CH_3$ | H |
| A-280 | I.1A' | $CH_2C{\equiv}CH$ | $CH_2OH$ | 3-$CH_3$ | H |
| A-281 | I.1A' | H | $NH_2$ | 3-$CH_3$ | H |
| A-282 | I.1A' | $CH_3$ | $NH_2$ | 3-$CH_3$ | H |
| A-283 | I.1A' | $CH_2CH_3$ | $NH_2$ | 3-$CH_3$ | H |
| A-284 | I.1A' | $CH_2CH_2CH_3$ | $NH_2$ | 3-$CH_3$ | H |
| A-285 | I.1A' | $CH_2CH_2CH_2CH_3$ | $NH_2$ | 3-$CH_3$ | H |
| A-286 | I.1A' | $CH_2CH{=}CH_2$ | $NH_2$ | 3-$CH_3$ | H |
| A-287 | I.1A' | $CH_2C{\equiv}CH$ | $NH_2$ | 3-$CH_3$ | H |
| A-288 | I.1A' | H | $COOCH_3$ | 3-$CH_3$ | H |
| A-289 | I.1A' | $CH_3$ | $COOCH_3$ | 3-$CH_3$ | H |
| A-290 | I.1A' | $CH_2CH_3$ | $COOCH_3$ | 3-$CH_3$ | H |
| A-291 | I.1A' | $CH_2CH_2CH_3$ | $COOCH_3$ | 3-$CH_3$ | H |
| A-292 | I.1A' | $CH_2CH_2CH_2CH_3$ | $COOCH_3$ | 3-$CH_3$ | H |
| A-293 | I.1A' | $CH_2CH{=}CH_2$ | $COOCH_3$ | 3-$CH_3$ | H |
| A-294 | I.1A' | $CH_2C{\equiv}CH$ | $COOCH_3$ | 3-$CH_3$ | H |

(fortgesetzt)

| Nr. | Formel | $R^1$ | $R^3$ | $R^9$ | $R^{10}$ |
|---|---|---|---|---|---|
| A-295 | I.1A’ | H | $CH_3$ | 3-OH | H |
| A-296 | I.1A’ | $CH_3$ | $CH_3$ | 3-OH | H |
| A-297 | I.1A’ | $CH_2CH_3$ | $CH_3$ | 3-OH | H |
| A-298 | I.1A’ | $CH_2CH_2CH_3$ | $CH_3$ | 3-OH | H |
| A-299 | I.1A’ | $CH_2CH_2CH_2CH_3$ | $CH_3$ | 3-OH | H |
| A-300 | I.1A’ | $CH_2CH=CH_2$ | $CH_3$ | 3-OH | H |
| A-301 | I.1A’ | $CH_2C\equiv CH$ | $CH_3$ | 3-OH | H |
| A-302 | I.1A’ | H | $CH_2CH_3$ | 3-OH | H |
| A-303 | I.1A’ | $CH_3$ | $CH_2CH_3$ | 3-OH | H |
| A-304 | I.1A’ | $CH_2CH_3$ | $CH_2CH_3$ | 3-OH | H |
| A-305 | I.1A’ | $CH_2CH_2CH_3$ | $CH_2CH_3$ | 3-OH | H |
| A-306 | I.1A’ | $CH_2CH_2CH_2CH_3$ | $CH_2CH_3$ | 3-OH | H |
| A-307 | I.1A’ | $CH_2CH=CH_2$ | $CH_2CH_3$ | 3-OH | H |
| A-308 | I.1A’ | $CH_2C\equiv CH$ | $CH_2CH_3$ | 3-OH | H |
| A-309 | I.1A’ | H | OH | 3-OH | H |
| A-310 | I.1A’ | $CH_3$ | OH | 3-OH | H |
| A-311 | I.1A’ | $CH_2CH_3$ | OH | 3-OH | H |
| A-312 | I.1A’ | $CH_2CH_2CH_3$ | OH | 3-OH | H |
| A-313 | I.1A’ | $CH_2CH_2CH_2CH_3$ | OH | 3-OH | H |
| A-314 | I.1A’ | $CH_2CH=CH_2$ | OH | 3-OH | H |
| A-315 | I.1A’ | $CH_2C\equiv CH$ | OH | 3-OH | H |
| A-316 | I.1A’ | H | $CH_2OH$ | 3-OH | H |
| A-317 | I.1A’ | $CH_3$ | $CH_2OH$ | 3-OH | H |
| A-318 | I.1A’ | $CH_2CH_3$ | $CH_2OH$ | 3-OH | H |
| A-319 | I.1A’ | $CH_2CH_2CH_3$ | $CH_2OH$ | 3-OH | H |

(fortgesetzt)

| Nr. | Formel | $R^1$ | $R^3$ | $R^9$ | $R^{10}$ |
|---|---|---|---|---|---|
| A-320 | I.1A' | $CH_2CH_2CH_2CH_3$ | $CH_2OH$ | 3-OH | H |
| A-321 | I.1A' | $CH_2CH=CH_2$ | $CH_2OH$ | 3-OH | H |
| A-322 | I.1A' | $CH_2C\equiv CH$ | $CH_2OH$ | 3-OH | H |
| A-323 | I.1A' | H | $NH_2$ | 3-OH | H |
| A-324 | I.1A' | $CH_3$ | $NH_2$ | 3-OH | H |
| A-325 | I.1A' | $CH_2CH_3$ | $NH_2$ | 3-OH | H |
| A-326 | I.1A' | $CH_2CH_2CH_3$ | $NH_2$ | 3-OH | H |
| A-327 | I.1A' | $CH_2CH_2CH_2CH_3$ | $NH_2$ | 3-OH | H |
| A-328 | I.1A' | $CH_2CH=CH_2$ | $NH_2$ | 3-OH | H |
| A-329 | I.1A' | $CH_2C\equiv CH$ | $NH_2$ | 3-OH | H |
| A-330 | I.1A' | H | $COOCH_3$ | 3-OH | H |
| A-331 | I.1A' | $CH_3$ | $COOCH_3$ | 3-OH | H |
| A-332 | I.1A' | $CH_2CH_3$ | $COOCH_3$ | 3-OH | H |
| A-333 | I.1A' | $CH_2CH_2CH_3$ | $COOCH_3$ | 3-OH | H |
| A-334 | I.1A' | $CH_2CH_2CH_2CH_3$ | $COOCH_3$ | 3-OH | H |
| A-335 | I.1A' | $CH_2CH=CH_2$ | $COOCH_3$ | 3-OH | H |
| A-336 | I.1A' | $CH_2C\equiv CH$ | $COOCH_3$ | 3-OH | H |
| A-337 | I.1A' | H | $CH_3$ | 3-OC(O)$CH_3$ | H |
| A-338 | I.1A' | $CH_3$ | $CH_3$ | 3-OC(O)$CH_3$ | H |
| A-339 | I.1A' | $CH_2CH_3$ | $CH_3$ | 3-OC(O)$CH_3$ | H |
| A-340 | I.1A' | $CH_2CH_2CH_3$ | $CH_3$ | 3-OC(O)$CH_3$ | H |
| A-341 | I.1A' | $CH_2CH_2CH_2CH_3$ | $CH_3$ | 3-OC(O)$CH_3$ | H |
| A-342 | I.1A' | $CH_2CH=CH_2$ | $CH_3$ | 3-OC(O)$CH_3$ | H |
| A-343 | I.1A' | $CH_2C\equiv CH$ | $CH_3$ | 3-OC(O)$CH_3$ | H |
| A-344 | I.1A' | H | $CH_2CH_3$ | 3-OC(O)$CH_3$ | H |

(fortgesetzt)

| Nr. | Formel | $R^1$ | $R^3$ | $R^9$ | $R^{10}$ |
|---|---|---|---|---|---|
| A-345 | I.1A' | $CH_3$ | $CH_2CH_3$ | 3-OC(O)$CH_3$ | H |
| A-346 | I.1A' | $CH_2CH_3$ | $CH_2CH_3$ | 3-OC(O)$CH_3$ | H |
| A-347 | I.1A' | $CH_2CH_2CH_3$ | $CH_2CH_3$ | 3-OC(O)$CH_3$ | H |
| A-348 | I.1A' | $CH_2CH_2CH_2CH_3$ | $CH_2CH_3$ | 3-OC(O)$CH_3$ | H |
| A-349 | I.1A' | $CH_2CH=CH_2$ | $CH_2CH_3$ | 3-OC(O)$CH_3$ | H |
| A-350 | I.1A' | $CH_2C\equiv CH$ | $CH_2CH_3$ | 3-OC(O)$CH_3$ | H |
| A-351 | I.1A' | H | OH | 3-OC(O)$CH_3$ | H |
| A-352 | I.1A' | $CH_3$ | OH | 3-OC(O)$CH_3$ | H |
| A-353 | I.1A' | $CH_2CH_3$ | OH | 3-OC(O)$CH_3$ | H |
| A-354 | I.1A' | $CH_2CH_2CH_3$ | OH | 3-OC(O)$CH_3$ | H |
| A-355 | I.1A' | $CH_2CH_2CH_2CH_3$ | OH | 3-OC(O)$CH_3$ | H |
| A-356 | I.1A' | $CH_2CH=CH_2$ | OH | 3-OC(O)$CH_3$ | H |
| A-357 | I.1A' | $CH_2C\equiv CH$ | OH | 3-OC(O)$CH_3$ | H |
| A-358 | I.1A' | H | $CH_2OH$ | 3-OC(O)$CH_3$ | H |
| A-359 | I.1A' | $CH_3$ | $CH_2OH$ | 3-OC(O)$CH_3$ | H |
| A-360 | I.1A' | $CH_2CH_3$ | $CH_2OH$ | 3-OC(O)$CH_3$ | H |
| A-361 | I.1A' | $CH_2CH_2CH_3$ | $CH_2OH$ | 3-OC(O)$CH_3$ | H |
| A-362 | I.1A' | $CH_2CH_2CH_2CH_3$ | $CH_2OH$ | 3-OC(O)$CH_3$ | H |
| A-363 | I.1A' | $CH_2CH=CH_2$ | $CH_2OH$ | 3-OC(O)$CH_3$ | H |
| A-364 | I.1A' | $CH_2C\equiv CH$ | $CH_2OH$ | 3-OC(O)$CH_3$ | H |
| A-365 | I.1A' | H | $NH_2$ | 3-OC(O)$CH_3$ | H |
| A-366 | I.1A' | $CH_3$ | $NH_2$ | 3-OC(O)$CH_3$ | H |
| A-367 | I.1A' | $CH_2CH_3$ | $NH_2$ | 3-OC(O)$CH_3$ | H |
| A-368 | I.1A' | $CH_2CH_2CH_3$ | $NH_2$ | 3-OC(O)$CH_3$ | H |
| A-369 | I.1A' | $CH_2CH_2CH_2CH_3$ | $NH_2$ | 3-OC(O)$CH_3$ | H |

(fortgesetzt)

| Nr. | Formel | $R^1$ | $R^3$ | $R^9$ | $R^{10}$ |
|---|---|---|---|---|---|
| A-370 | I.1A' | $CH_2CH=CH_2$ | $NH_2$ | 3-OC(O)$CH_3$ | H |
| A-371 | I.1A' | $CH_2C≡CH$ | $NH_2$ | 3-OC(O)$CH_3$ | H |
| A-372 | I.1A' | H | $COOCH_3$ | 3-OC(O)$CH_3$ | H |
| A-373 | I.1A' | $CH_3$ | $COOCH_3$ | 3-OC(O)$CH_3$ | H |
| A-374 | I.1A' | $CH_2CH_3$ | $COOCH_3$ | 3-OC(O)$CH_3$ | H |
| A-375 | I.1A' | $CH_2CH_2CH_3$ | $COOCH_3$ | 3-OC(O)$CH_3$ | H |
| A-376 | I.1A' | $CH_2CH_2CH_2CH_3$ | $COOCH_3$ | 3-OC(O)$CH_3$ | H |
| A-377 | I.1A' | $CH_2CH=CH_2$ | $COOCH_3$ | 3-OC(O)$CH_3$ | H |
| A-378 | I.1A' | $CH_2C=CH$ | $COOCH_3$ | 3-OC(O)$CH_3$ | H |
| A-379 | I.1B' | H | $CH_3$ | H | H |
| A-380 | I.1B' | $CH_3$ | $CH_3$ | H | H |
| A-381 | I.1B' | $CH_2CH_3$ | $CH_3$ | H | H |
| A-382 | I.1B' | $CH_2CH_2CH_3$ | $CH_3$ | H | H |
| A-383 | I.1B' | $CH_2CH_2CH_2CH_3$ | $CH_3$ | H | H |
| A-384 | I.1B' | $CH_2CH=CH_2$ | $CH_3$ | H | H |
| A-385 | I.1B' | $CH_2C≡CH$ | $CH_3$ | H | H |
| A-386 | I.1B' | H | $CH_2CH_3$ | H | H |
| A-387 | I.1B' | $CH_3$ | $CH_2CH_3$ | H | H |
| A-388 | I.1B' | $CH_2CH_3$ | $CH_2CH_3$ | H | H |
| A-389 | I.1B' | $CH_2CH_2CH_3$ | $CH_2CH_3$ | H | H |
| A-390 | I.1B' | $CH_2CH_2CH_2CH_3$ | $CH_2CH_3$ | H | H |
| A-391 | I.1B' | $CH_2CH=CH_2$ | $CH_2CH_3$ | H | H |
| A-392 | I.1B' | $CH_2C≡CH$ | $CH_2CH_3$ | H | H |
| A-393 | I.1B' | H | OH | H | H |
| A-394 | I.1B' | $CH_3$ | OH | H | H |

(fortgesetzt)

| Nr. | Formel | $R^1$ | $R^3$ | $R^9$ | $R^{10}$ |
|---|---|---|---|---|---|
| A-395 | I.1B’ | $CH_2CH_3$ | OH | H | H |
| A-396 | I.1B’ | $CH_2CH_2CH_3$ | OH | H | H |
| A-397 | I.1B’ | $CH_2CH_2CH_2CH_3$ | OH | H | H |
| A-398 | I.1B’ | $CH_2CH{=}CH_2$ | OH | H | H |
| A-399 | I.1B’ | $CH_2C{\equiv}CH$ | OH | H | H |
| A-400 | I.1B’ | H | $CH_2OH$ | H | H |
| A-401 | I.1B’ | $CH_3$ | $CH_2OH$ | H | H |
| A-402 | I.1B’ | $CH_2CH_3$ | $CH_2OH$ | H | H |
| A-403 | I.1B’ | $CH_2CH_2CH_3$ | $CH_2OH$ | H | H |
| A-404 | I.1B’ | $CH_2CH_2CH_2CH_3$ | $CH_2OH$ | H | H |
| A-405 | I.18’ | $CH_2CH{=}CH_2$ | $CH_2OH$ | H | H |
| A-406 | I.1B’ | $CH_2C{\equiv}CH$ | $CH_2OH$ | H | H |
| A-407 | I.1B’ | H | $NH_2$ | H | H |
| A-408 | I.1B’ | $CH_3$ | $NH_2$ | H | H |
| A-409 | I.1B’ | $CH_2CH_3$ | $NH_2$ | H | H |
| A-410 | I.1B’ | $CH_2CH_2CH_3$ | $NH_2$ | H | H |
| A-411 | I.1B’ | $CH_2CH_2CH_2CH_3$ | $NH_2$ | H | H |
| A-412 | I.1B’ | $CH_2CH{=}CH_2$ | $NH_2$ | H | H |
| A-413 | I.1B’ | $CH_2C{\equiv}CH$ | $NH_2$ | H | H |
| A-414 | I.1B’ | H | $COOCH_3$ | H | H |
| A-415 | I.1B’ | $CH_3$ | $COOCH_3$ | H | H |
| A-416 | I.1B’ | $CH_2CH_3$ | $COOCH_3$ | H | H |
| A-417 | I.1B’ | $CH_2CH_2CH_3$ | $COOCH_3$ | H | H |
| A-418 | I.1B’ | $CH_2CH_2CH_2CH_3$ | $COOCH_3$ | H | H |
| A-419 | I.1B’ | $CH_2CH{=}CH_2$ | $COOCH_3$ | H | H |

(fortgesetzt)

| Nr. | Formel | $R^1$ | $R^3$ | $R^9$ | $R^{10}$ |
|---|---|---|---|---|---|
| A-420 | I.1B’ | $CH_2C{\equiv}CH$ | $COOCH_3$ | H | H |
| A-421 | I.1B’ | H | $CH_3$ | 2-F | H |
| A-422 | I.1B’ | $CH_3$ | $CH_3$ | 2-F | H |
| A-423 | I.1B’ | $CH_2CH_3$ | $CH_3$ | 2-F | H |
| A-424 | I.1B’ | $CH_2CH_2CH_3$ | $CH_3$ | 2-F | H |
| A-425 | I.1B’ | $CH_2CH_2CH_2CH_3$ | $CH_3$ | 2-F | H |
| A-426 | I.1B’ | $CH_2CH{=}CH_2$ | $CH_3$ | 2-F | H |
| A-427 | I.1B’ | $CH_2C{\equiv}CH$ | $CH_3$ | 2-F | H |
| A-428 | I.1B’ | H | $CH_2CH_3$ | 2-F | H |
| A-429 | I.1B’ | $CH_3$ | $CH_2CH_3$ | 2-F | H |
| A-430 | I.1B’ | $CH_2CH_3$ | $CH_2CH_3$ | 2-F | H |
| A-431 | I.1B’ | $CH_2CH_2CH_3$ | $CH_2CH_3$ | 2-F | H |
| A-432 | I.1B’ | $CH_2CH_2CH_2CH_3$ | $CH_2CH_3$ | 2-F | H |
| A-433 | I.1B’ | $CH_2CH{=}CH_2$ | $CH_2CH_3$ | 2-F | H |
| A-434 | I.1B’ | $CH_2C{=}CH$ | $CH_2CH_3$ | 2-F | H |
| A-435 | I.1B’ | H | OH | 2-F | H |
| A-436 | I.1B’ | $CH_3$ | OH | 2-F | H |
| A-437 | I.1B’ | $CH_2CH_3$ | OH | 2-F | H |
| A-438 | I.1B’ | $CH_2CH_2CH_3$ | OH | 2-F | H |
| A-439 | I.1B’ | $CH_2CH_2CH_2CH_3$ | OH | 2-F | H |
| A-440 | I.1B’ | $CH_2CH{=}CH_2$ | OH | 2-F | H |
| A-441 | I.1B’ | $CH_2C{\equiv}CH$ | OH | 2-F | H |
| A-442 | I.1B’ | H | $CH_2OH$ | 2-F | H |
| A-443 | I.1B’ | $CH_3$ | $CH_2OH$ | 2-F | H |
| A-444 | I.1B’ | $CH_2CH_3$ | $CH_2OH$ | 2-F | H |

(fortgesetzt)

| Nr. | Formel | $R^1$ | $R^3$ | $R^9$ | $R^{10}$ |
|---|---|---|---|---|---|
| A-445 | I.1B' | $CH_2CH_2CH_3$ | $CH_2OH$ | 2-F | H |
| A-446 | I.1B' | $CH_2CH_2CH_2CH_3$ | $CH_2OH$ | 2-F | H |
| A-447 | I.1B' | $CH_2CH=CH_2$ | $CH_2OH$ | 2-F | H |
| A-448 | I.1B' | $CH_2C\equiv CH$ | $CH_2OH$ | 2-F | H |
| A-449 | I.1B' | H | $NH_2$ | 2-F | H |
| A-450 | I.1B' | $CH_3$ | $NH_2$ | 2-F | H |
| A-451 | I.1B' | $CH_2CH_3$ | $NH_2$ | 2-F | H |
| A-452 | I.1B' | $CH_2CH_2CH_3$ | $NH_2$ | 2-F | H |
| A-453 | I.1B' | $CH_2CH_2CH_2CH_3$ | $NH_2$ | 2-F | H |
| A-454 | I.1B' | $CH_2CH=CH_2$ | $NH_2$ | 2-F | H |
| A-455 | I.1B' | $CH_2C=CH$ | $NH_2$ | 2-F | H |
| A-456 | I.1B' | H | $COOCH_3$ | 2-F | H |
| A-457 | I.1B' | $CH_3$ | $COOCH_3$ | 2-F | H |
| A-458 | I.1B' | $CH_2CH_3$ | $COOCH_3$ | 2-F | H |
| A-459 | I.1B' | $CH_2CH_2CH_3$ | $COOCH_3$ | 2-F | H |
| A-460 | I.1B' | $CH_2CH_2CH_2CH_3$ | $COOCH_3$ | 2-F | H |
| A-461 | I.1B' | $CH_2CH=CH_2$ | $COOCH_3$ | 2-F | H |
| A-462 | I.1B' | $CH_2C\equiv CH$ | $COOCH_3$ | 2-F | H |
| A-463 | I.1B' | H | $CH_3$ | 3-F | H |
| A-464 | I.1B' | $CH_3$ | $CH_3$ | 3-F | H |
| A-465 | I.1B' | $CH_2CH_3$ | $CH_3$ | 3-F | H |
| A-466 | I.1B' | $CH_2CH_2CH_3$ | $CH_3$ | 3-F | H |
| A-467 | I.1B' | $CH_2CH_2CH_2CH_3$ | $CH_3$ | 3-F | H |
| A-468 | I.1B' | $CH_2CH=CH_2$ | $CH_3$ | 3-F | H |
| A-469 | I.1B' | $CH_2C\equiv CH$ | $CH_3$ | 3-F | H |

(fortgesetzt)

| Nr. | Formel | $R^1$ | $R^3$ | $R^9$ | $R^{10}$ |
|---|---|---|---|---|---|
| A-470 | I.1B' | H | $CH_2CH_3$ | 3-F | H |
| A-471 | I.1B' | $CH_3$ | $CH_2CH_3$ | 3-F | H |
| A-472 | I.1B' | $CH_2CH_3$ | $CH_2CH_3$ | 3-F | H |
| A-473 | I.1B' | $CH_2CH_2CH_3$ | $CH_2CH_3$ | 3-F | H |
| A-474 | I.1B' | $CH_2CH_2CH_2CH_3$ | $CH_2CH_3$ | 3-F | H |
| A-475 | I.1B' | $CH_2CH=CH_2$ | $CH_2CH_3$ | 3-F | H |
| A-476 | I.1B' | $CH_2C≡CH$ | $CH_2CH_3$ | 3-F | H |
| A-477 | I.1B' | H | OH | 3-F | H |
| A-478 | I.1B' | $CH_3$ | OH | 3-F | H |
| A-479 | I.1B' | $CH_2CH_3$ | OH | 3-F | H |
| A-480 | I.1B' | $CH_2CH_2CH_3$ | OH | 3-F | H |
| A-481 | I.1B' | $CH_2CH_2CH_2CH_3$ | OH | 3-F | H |
| A-482 | I.1B' | $CH_2CH=CH_2$ | OH | 3-F | H |
| A-483 | I.1B' | $CH_2C≡CH$ | OH | 3-F | H |
| A-484 | I.1B' | H | $CH_2OH$ | 3-F | H |
| A-485 | I.1B' | $CH_3$ | $CH_2OH$ | 3-F | H |
| A-486 | I.1B' | $CH_2CH_3$ | $CH_2OH$ | 3-F | H |
| A-487 | I.1B' | $CH_2CH_2CH_3$ | $CH_2OH$ | 3-F | H |
| A-488 | I.1B' | $CH_2CH_2CH_2CH_3$ | $CH_2OH$ | 3-F | H |
| A-489 | I.1B' | $CH_2CH=CH_2$ | $CH_2OH$ | 3-F | H |
| A-490 | I.1B' | $CH_2C≡CH$ | $CH_2OH$ | 3-F | H |
| A-491 | I.1B' | H | $NH_2$ | 3-F | H |
| A-492 | I.1B' | $CH_3$ | $NH_2$ | 3-F | H |
| A-493 | I.1B' | $CH_2CH_3$ | $NH_2$ | 3-F | H |
| A-494 | I.1B' | $CH_2CH_2CH_3$ | $NH_2$ | 3-F | H |

(fortgesetzt)

| Nr. | Formel | $R^1$ | $R^3$ | $R^9$ | $R^{10}$ |
|---|---|---|---|---|---|
| A-495 | I.1B' | $CH_2CH_2CH_2CH_3$ | $NH_2$ | 3-F | H |
| A-496 | I.1B' | $CH_2CH=CH_2$ | $NH_2$ | 3-F | H |
| A-497 | I.1B' | $CH_2C{\equiv}CH$ | $NH_2$ | 3-F | H |
| A-498 | I.1B' | H | $COOCH_3$ | 3-F | H |
| A-499 | I.1B' | $CH_3$ | $COOCH_3$ | 3-F | H |
| A-500 | I.1B' | $CH_2CH_3$ | $COOCH_3$ | 3-F | H |
| A-501 | I.1B' | $CH_2CH_2CH_3$ | $COOCH_3$ | 3-F | H |
| A-502 | I.1B' | $CH_2CH_2CH_2CH_3$ | $COOCH_3$ | 3-F | H |
| A-503 | I.1B' | $CH_2CH=CH_2$ | $COOCH_3$ | 3-F | H |
| A-504 | I.1B' | $CH_2C{\equiv}CH$ | $COOCH_3$ | 3-F | H |
| A-505 | I.1B' | H | $CH_3$ | 4-F | H |
| A-506 | I.1B' | $CH_3$ | $CH_3$ | 4-F | H |
| A-507 | I.1B' | $CH_2CH_3$ | $CH_3$ | 4-F | H |
| A-508 | I.1B' | $CH_2CH_2CH_3$ | $CH_3$ | 4-F | H |
| A-509 | I.1B' | $CH_2CH_2CH_2CH_3$ | $CH_3$ | 4-F | H |
| A-510 | I.1B' | $CH_2CH=CH_2$ | $CH_3$ | 4-F | H |
| A-511 | I.1B' | $CH_2C{\equiv}CH$ | $CH_3$ | 4-F | H |
| A-512 | I.1B' | H | $CH_2CH_3$ | 4-F | H |
| A-513 | I.1B' | $CH_3$ | $CH_2CH_3$ | 4-F | H |
| A-514 | I.1B' | $CH_2CH_3$ | $CH_2CH_3$ | 4-F | H |
| A-515 | I.1B' | $CH_2CH_2CH_3$ | $CH_2CH_3$ | 4-F | H |
| A-516 | I.1B' | $CH_2CH_2CH_2CH_3$ | $CH_2CH_3$ | 4-F | H |
| A-517 | I.1B' | $CH_2CH=CH_2$ | $CH_2CH_3$ | 4-F | H |
| A-518 | I.1B' | $CH_2C{\equiv}CH$ | $CH_2CH_3$ | 4-F | H |
| A-519 | I.1B' | H | OH | 4-F | H |

(fortgesetzt)

| Nr. | Formel | $R^1$ | $R^3$ | $R^9$ | $R^{10}$ |
|---|---|---|---|---|---|
| A-520 | I.1B’ | $CH_3$ | OH | 4-F | H |
| A-521 | I.1B’ | $CH_2CH_3$ | OH | 4-F | H |
| A-522 | I.1B’ | $CH_2CH_2CH_3$ | OH | 4-F | H |
| A-523 | I.1B’ | $CH_2CH_2CH_2CH_3$ | OH | 4-F | H |
| A-524 | I.1B’ | $CH_2CH{=}CH_2$ | OH | 4-F | H |
| A-525 | I.1B’ | $CH_2C\equiv CH$ | OH | 4-F | H |
| A-526 | I,1B’ | H | $CH_2OH$ | 4-F | H |
| A-527 | I.1B’ | $CH_3$ | $CH_2OH$ | 4-F | H |
| A-528 | I.1B’ | $CH_2CH_3$ | $CH_2OH$ | 4-F | H |
| A-529 | I.1B’ | $CH_2CH_2CH_3$ | $CH_2OH$ | 4-F | H |
| A-530 | I.1B’ | $CH_2CH_2CH_2CH_3$ | $CH_2OH$ | 4-F | H |
| A-531 | I.1B’ | $CH_2CH{=}CH_2$ | $CH_2OH$ | 4-F | H |
| A-532 | I.1B’ | $CH_2C\equiv CH$ | $CH_2OH$ | 4-F | H |
| A-533 | I.1B’ | H | $NH_2$ | 4-F | H |
| A-534 | I.1B’ | $CH_3$ | $NH_2$ | 4-F | H |
| A-535 | I.1B’ | $CH_2CH_3$ | $NH_2$ | 4-F | H |
| A-536 | I.1B’ | $CH_2CH_2CH_3$ | $NH_2$ | 4-F | H |
| A-537 | I.1B’ | $CH_2CH_2CH_2CH_3$ | $NH_2$ | 4-F | H |
| A-538 | I.1B’ | $CH_2CH{=}CH_2$ | $NH_2$ | 4-F | H |
| A-539 | I.1B’ | $CH_2C\equiv CH$ | $NH_2$ | 4-F | H |
| A-540 | I.1B’ | H | $COOCH_3$ | 4-F | H |
| A-541 | I.1B’ | $CH_3$ | $COOCH_3$ | 4-F | H |
| A-542 | I.1B’ | $CH_2CH_3$ | $COOCH_3$ | 4-F | H |
| A-543 | I.1B’ | $CH_2CH_2CH_3$ | $COOCH_3$ | 4-F | H |
| A-544 | I.1B’ | $CH_2CH_2CH_2CH_3$ | $COOCH_3$ | 4-F | H |

(fortgesetzt)

| Nr. | Formel | $R^1$ | $R^3$ | $R^9$ | $R^{10}$ |
|---|---|---|---|---|---|
| A-545 | I.1B' | $CH_2CH=CH_2$ | $COOCH_3$ | 4-F | H |
| A-546 | I.1B' | $CH_2C\equiv CH$ | $COOCH_3$ | 4-F | H |
| A-547 | I.1B' | H | $CH_3$ | 3-F | 2-F |
| A-548 | I.1B' | $CH_3$ | $CH_3$ | 3-F | 2-F |
| A-549 | I.1B' | $CH_2CH_3$ | $CH_3$ | 3-F | 2-F |
| A-550 | I.1B' | $CH_2CH_2CH_3$ | $CH_3$ | 3-F | 2-F |
| A-551 | I.1B' | $CH_2CH_2CH_2CH_3$ | $CH_3$ | 3-F | 2-F |
| A-552 | I.1B' | $CH_2CH=CH_2$ | $CH_3$ | 3-F | 2-F |
| A-553 | I.1B' | $CH_2C\equiv CH$ | $CH_3$ | 3-F | 2-F |
| A-554 | I.1B' | H | $CH_2CH_3$ | 3-F | 2-F |
| A-555 | I.1B' | $CH_3$ | $CH_2CH_3$ | 3-F | 2-F |
| A-556 | I.1B' | $CH_2CH_3$ | $CH_2CH_3$ | 3-F | 2-F |
| A-557 | I.1B' | $CH_2CH_2CH_3$ | $CH_2CH_3$ | 3-F | 2-F |
| A-558 | I.1B' | $CH_2CH_2CH_2CH_3$ | $CH_2CH_3$ | 3-F | 2-F |
| A-559 | I.1B' | $CH_2CH=CH_2$ | $CH_2CH_3$ | 3-F | 2-F |
| A-560 | I.1B' | $CH_2C\equiv CH$ | $CH_2CH_3$ | 3-F | 2-F |
| A-561 | I.1B' | H | OH | 3-F | 2-F |
| A-562 | I.1B' | $CH_3$ | OH | 3-F | 2-F |
| A-563 | I.1B' | $CH_2CH_3$ | OH | 3-F | 2-F |
| A-564 | I.1B' | $CH_2CH_2CH_3$ | OH | 3-F | 2-F |
| A-565 | I.1B' | $CH_2CH_2CH_2CH_3$ | OH | 3-F | 2-F |
| A-566 | I.1B' | $CH_2CH=CH_2$ | OH | 3-F | 2-F |
| A-567 | I.1B' | $CH_2C\equiv CH$ | OH | 3-F | 2-F |
| A-568 | I.1B' | H | $CH_2OH$ | 3-F | 2-F |
| A-569 | I.1B' | $CH_3$ | $CH_2OH$ | 3-F | 2-F |

(fortgesetzt)

| Nr. | Formel | $R^1$ | $R^3$ | $R^9$ | $R^{10}$ |
|---|---|---|---|---|---|
| A-570 | I.1B' | $CH_2CH_3$ | $CH_2OH$ | 3-F | 2-F |
| A-571 | I.1B' | $CH_2CH_2CH_3$ | $CH_2OH$ | 3-F | 2-F |
| A-572 | I.1B' | $CH_2CH_2CH_2CH_3$ | $CH_2OH$ | 3-F | 2-F |
| A-573 | I.1B' | $CH_2CH=CH_2$ | $CH_2OH$ | 3-F | 2-F |
| A-574 | I.1B' | $CH_2C\equiv CH$ | $CH_2OH$ | 3-F | 2-F |
| A-575 | I.1B' | H | $NH_2$ | 3-F | 2-F |
| A-576 | I.1B' | $CH_3$ | $NH_2$ | 3-F | 2-F |
| A-577 | I.1B' | $CH_2CH_3$ | $NH_2$ | 3-F | 2-F |
| A-578 | I.1B' | $CH_2CH_2CH_3$ | $NH_2$ | 3-F | 2-F |
| A-579 | I.1B' | $CH_2CH_2CH_2CH_3$ | $NH_2$ | 3-F | 2-F |
| A-580 | I.1B' | $CH_2CH=CH_2$ | $NH_2$ | 3-F | 2-F |
| A-581 | I.1B' | $CH_2C\equiv CH$ | $NH_2$ | 3-F | 2-F |
| A-582 | I.1B' | H | $COOCH_3$ | 3-F | 2-F |
| A-583 | I.1B' | $CH_3$ | $COOCH_3$ | 3-F | 2-F |
| A-584 | I.1B' | $CH_2CH_3$ | $COOCH_3$ | 3-F | 2-F |
| A-585 | I.1B' | $CH_2CH_2CH_3$ | $COOCH_3$ | 3-F | 2-F |
| A-586 | I.1B' | $CH_2CH_2CH_2CH_3$ | $COOCH_3$ | 3-F | 2-F |
| A-587 | I.1B' | $CH_2CH=CH_2$ | $COOCH_3$ | 3-F | 2-F |
| A-588 | I.1B' | $CH_2C\equiv CH$ | $COOCH_3$ | 3-F | 2-F |
| A-589 | I.1B' | H | $CH_3$ | 3-F | 4-F |
| A-590 | I.1B' | $CH_3$ | $CH_3$ | 3-F | 4-F |
| A-591 | I.1B' | $CH_2CH_3$ | $CH_3$ | 3-F | 4-F |
| A-592 | I.1B' | $CH_2CH_2CH_3$ | $CH_3$ | 3-F | 4-F |
| A-593 | I.1B' | $CH_2CH_2CH_2CH_3$ | $CH_3$ | 3-F | 4-F |
| A-594 | I.1B' | $CH_2CH=CH_2$ | $CH_3$ | 3-F | 4-F |

(fortgesetzt)

| Nr. | Formel | $R^1$ | $R^3$ | $R^9$ | $R^{10}$ |
|---|---|---|---|---|---|
| A-595 | I.1B' | $CH_2C{\equiv}CH$ | $CH_3$ | 3-F | 4-F |
| A-596 | I.1B' | H | $CH_2CH_3$ | 3-F | 4-F |
| A-597 | I.1B' | $CH_3$ | $CH_2CH_3$ | 3-F | 4-F |
| A-598 | I.1B' | $CH_2CH_3$ | $CH_2CH_3$ | 3-F | 4-F |
| A-599 | I.1B' | $CH_2CH_2CH_3$ | $CH_2CH_3$ | 3-F | 4-F |
| A-600 | I.1B' | $CH_2CH_2CH_2CH_3$ | $CH_2CH_3$ | 3-F | 4-F |
| A-601 | I.1B' | $CH_2CH{=}CH_2$ | $CH_2CH_3$ | 3-F | 4-F |
| A-602 | I.1B' | $CH_2C{=}CH$ | $CH_2CH_3$ | 3-F | 4-F |
| A-603 | I.1B' | H | OH | 3-F | 4-F |
| A-604 | I.1B' | $CH_3$ | OH | 3-F | 4-F |
| A-605 | I.1B' | $CH_2CH_3$ | OH | 3-F | 4-F |
| A-606 | I.1B' | $CH_2CH_2CH_3$ | OH | 3-F | 4-F |
| A-607 | I.1B' | $CH_2CH_2CH_2CH_3$ | OH | 3-F | 4-F |
| A-608 | I.1B' | $CH_2CH{=}CH_2$ | OH | 3-F | 4-F |
| A-609 | I.1B' | $CH_2C{\equiv}CH$ | OH | 3-F | 4-F |
| A-610 | I.1B' | H | $CH_2OH$ | 3-F | 4-F |
| A-611 | I.1B' | $CH_3$ | $CH_2OH$ | 3-F | 4-F |
| A-612 | I.1B' | $CH_2CH_3$ | $CH_2OH$ | 3-F | 4-F |
| A-613 | I.1B' | $CH_2CH_2CH_3$ | $CH_2OH$ | 3-F | 4-F |
| A-614 | I.1B' | $CH_2CH_2CH_2CH_3$ | $CH_2OH$ | 3-F | 4-F |
| A-615 | I.1B' | $CH_2CH{=}CH_2$ | $CH_2OH$ | 3-F | 4-F |
| A-616 | I.1B' | $CH_2C{\equiv}CH$ | $CH_2OH$ | 3-F | 4-F |
| A-617 | I.1B' | H | $NH_2$ | 3-F | 4-F |
| A-618 | I.1B' | $CH_3$ | $NH_2$ | 3-F | 4-F |
| A-619 | I.1B' | $CH_2CH_3$ | $NH_2$ | 3-F | 4-F |

(fortgesetzt)

| Nr. | Formel | $R^1$ | $R^3$ | $R^9$ | $R^{10}$ |
|---|---|---|---|---|---|
| A-620 | I.1B' | $CH_2CH_2CH_3$ | $NH_2$ | 3-F | 4-F |
| A-621 | I.1B' | $CH_2CH_2CH_2CH_3$ | $NH_2$ | 3-F | 4-F |
| A-622 | I.1B' | $CH_2CH{=}CH_2$ | $NH_2$ | 3-F | 4-F |
| A-623 | I.1B' | $CH_2C{\equiv}CH$ | $NH_2$ | 3-F | 4-F |
| A-624 | I.1B' | H | $COOCH_3$ | 3-F | 4-F |
| A-625 | I.1B' | $CH_3$ | $COOCH_3$ | 3-F | 4-F |
| A-626 | I.1B' | $CH_2CH_3$ | $COOCH_3$ | 3-F | 4-F |
| A-627 | I.1B' | $CH_2CH_2CH_3$ | $COOCH_3$ | 3-F | 4-F |
| A-628 | I.1B' | $CH_2CH_2CH_2CH_3$ | $COOCH_3$ | 3-F | 4-F |
| A-629 | I.1B' | $CH_2CH{=}CH_2$ | $COOCH_3$ | 3-F | 4-F |
| A-630 | I.1B' | $CH_2C{\equiv}CH$ | $COOCH_3$ | 3-F | 4-F |
| A-631 | I.1B' | H | $CH_3$ | 3-$CH_3$ | H |
| A-632 | I.1B' | $CH_3$ | $CH_3$ | 3-$CH_3$ | H |
| A-633 | I.1B' | $CH_2CH_3$ | $CH_3$ | 3-$CH_3$ | H |
| A-634 | I.1B' | $CH_2CH_2CH_3$ | $CH_3$ | 3-$CH_3$ | H |
| A-635 | I.1B' | $CH_2CH_2CH_2CH_3$ | $CH_3$ | 3-$CH_3$ | H |
| A-636 | I.1B' | $CH_2CH{=}CH_2$ | $CH_3$ | 3-$CH_3$ | H |
| A-637 | I.1B' | $CH_2C{\equiv}CH$ | $CH_3$ | 3-$CH_3$ | H |
| A-638 | I.1B' | H | $CH_2CH_3$ | 3-$CH_3$ | H |
| A-639 | I.1B' | $CH_3$ | $CH_2CH_3$ | 3-$CH_3$ | H |
| A-640 | I.1B' | $CH_2CH_3$ | $CH_2CH_3$ | 3-$CH_3$ | H |
| A-641 | I.1B' | $CH_2CH_2CH_3$ | $CH_2CH_3$ | 3-$CH_3$ | H |
| A-642 | I.1B' | $CH_2CH_2CH_2CH_3$ | $CH_2CH_3$ | 3-$CH_3$ | H |
| A-643 | I.1B' | $CH_2CH{=}CH_2$ | $CH_2CH_3$ | 3-$CH_3$ | H |
| A-644 | I.1B' | $CH_2C{\equiv}CH$ | $CH_2CH_3$ | 3-$CH_3$ | H |

(fortgesetzt)

| Nr. | Formel | $R^1$ | $R^3$ | $R^9$ | $R^{10}$ |
|---|---|---|---|---|---|
| A-645 | I.1B' | H | OH | 3-$CH_3$ | H |
| A-646 | I.1B' | $CH_3$ | OH | 3-$CH_3$ | H |
| A-647 | I.1B' | $CH_2CH_3$ | OH | 3-$CH_3$ | H |
| A-648 | I.1B' | $CH_2CH_2CH_3$ | OH | 3-$CH_3$ | H |
| A-649 | I.1B' | $CH_2CH_2CH_2CH_3$ | OH | 3-$CH_3$ | H |
| A-650 | I.1B' | $CH_2CH=CH_2$ | OH | 3-$CH_3$ | H |
| A-651 | I.1B' | $CH_2C\equiv CH$ | OH | 3-$CH_3$ | H |
| A-652 | I.1B' | H | $CH_2OH$ | 3-$CH_3$ | H |
| A-653 | I.1B' | $CH_3$ | $CH_2OH$ | 3-$CH_3$ | H |
| A-654 | I.1B' | $CH_2CH_3$ | $CH_2OH$ | 3-$CH_3$ | H |
| A-655 | I.1B' | $CH_2CH_2CH_3$ | $CH_2OH$ | 3-$CH_3$ | H |
| A-656 | I.1B' | $CH_2CH_2CH_2CH_3$ | $CH_2OH$ | 3-$CH_3$ | H |
| A-657 | I.1B' | $CH_2CH=CH_2$ | $CH_2OH$ | 3-$CH_3$ | H |
| A-658 | I.1B' | $CH_2C\equiv CH$ | $CH_2OH$ | 3-$CH_3$ | H |
| A-659 | I.1B' | H | $NH_2$ | 3-$CH_3$ | H |
| A-660 | I.1B, | $CH_3$ | $NH_2$ | 3-$CH_3$ | H |
| A-661 | I.1B' | $CH_2CH_3$ | $NH_2$ | 3-$CH_3$ | H |
| A-662 | I.1B' | $CH_2CH_2CH_3$ | $NH_2$ | 3-$CH_3$ | H |
| A-663 | I.1B' | $CH_2CH_2CH_2CH_3$ | $NH_2$ | 3-$CH_3$ | H |
| A-664 | I.1B' | $CH_2CH=CH_2$ | $NH_2$ | 3-$CH_3$ | H |
| A-665 | I.1B' | $CH_2C\equiv CH$ | $NH_2$ | 3-$CH_3$ | H |
| A-666 | I.1B' | H | $COOCH_3$ | 3-$CH_3$ | H |
| A-667 | I.1B' | $CH_3$ | $COOCH_3$ | 3-$CH_3$ | H |
| A-668 | I.1B' | $CH_2CH_3$ | $COOCH_3$ | 3-$CH_3$ | H |
| A-669 | 1.1B' | $CH_2CH_2CH_3$ | $COOCH_3$ | 3-$CH_3$ | H |

(fortgesetzt)

| Nr. | Formel | $R^1$ | $R^3$ | $R^9$ | $R^{10}$ |
|---|---|---|---|---|---|
| A-670 | I.1B' | $CH_2CH_2CH_2CH_3$ | $COOCH_3$ | 3-$CH_3$ | H |
| A-671 | I.1B' | $CH_2CH=CH_2$ | $COOCH_3$ | 3-$CH_3$ | H |
| A-672 | I.1B' | $CH_2C≡CH$ | $COOCH_3$ | 3-$CH_3$ | H |
| A-673 | I.1B' | H | $CH_3$ | 3-OH | H |
| A-674 | I.1B' | $CH_3$ | $CH_3$ | 3-OH | H |
| A-675 | I.1B' | $CH_2CH_3$ | $CH_3$ | 3-OH | H |
| A-676 | I.1B' | $CH_2CH_2CH_3$ | $CH_3$ | 3-OH | H |
| A-677 | I.1B' | $CH_2CH_2CH_2CH_3$ | $CH_3$ | 3-OH | H |
| A-678 | I.1B' | $CH_2CH=CH_2$ | $CH_3$ | 3-OH | H |
| A-679 | I.1B' | $CH_2C≡CH$ | $CH_3$ | 3-OH | H |
| A-680 | I.1B' | H | $CH_2CH_3$ | 3-OH | H |
| A-681 | I.1B' | $CH_3$ | $CH_2CH_3$ | 3-OH | H |
| A-682 | I.1B' | $CH_2CH_3$ | $CH_2CH_3$ | 3-OH | H |
| A-683 | I.1B' | $CH_2CH_2CH_3$ | $CH_2CH_3$ | 3-OH | H |
| A-684 | I.1B' | $CH_2CH_2CH_2CH_3$ | $CH_2CH_3$ | 3-OH | H |
| A-685 | I.1B' | $CH_2CH=CH_2$ | $CH_2CH_3$ | 3-OH | H |
| A-686 | I.1B' | $CH_2C≡CH$ | $CH_2CH_3$ | 3-OH | H |
| A-687 | I.1B' | H | OH | 3-OH | H |
| A-688 | I.1B' | $CH_3$ | OH | 3-OH | H |
| A-689 | I.1B' | $CH_2CH_3$ | OH | 3-OH | H |
| A-690 | I.1B' | $CH_2CH_2CH_3$ | OH | 3-OH | H |
| A-691 | I.1B' | $CH_2CH_2CH_2CH_3$ | OH | 3-OH | H |
| A-692 | I.1B' | $CH_2CH=CH_2$ | OH | 3-OH | H |
| A-693 | I.1B' | $CH_2C≡CH$ | OH | 3-OH | H |
| A-694 | I.1B' | H | $CH_2OH$ | 3-OH | H |

(fortgesetzt)

| Nr. | Formel | $R^1$ | $R^3$ | $R^9$ | $R^{10}$ |
|---|---|---|---|---|---|
| A-695 | I.1B' | $CH_3$ | $CH_2OH$ | 3-OH | H |
| A-696 | I.1B' | $CH_2CH_3$ | $CH_2OH$ | 3-OH | H |
| A-697 | I.1B' | $CH_2CH_2CH_3$ | $CH_2OH$ | 3-OH | H |
| A-698 | I.1B' | $CH_2CH_2CH_2CH_3$ | $CH_2OH$ | 3-OH | H |
| A-699 | I.1B' | $CH_2CH=CH_2$ | $CH_2OH$ | 3-OH | H |
| A-700 | I.1B' | $CH_2C\equiv CH$ | $CH_2OH$ | 3-OH | H |
| A-701 | I.1B' | H | $NH_2$ | 3-OH | H |
| A-702 | I.1B' | $CH_3$ | $NH_2$ | 3-OH | H |
| A-703 | I.1B' | $CH_2CH_3$ | $NH_2$ | 3-OH | H |
| A-704 | I.1B' | $CH_2CH_2CH_3$ | $NH_2$ | 3-OH | H |
| A-705 | I.1B' | $CH_2CH_2CH_2CH_3$ | $NH_2$ | 3-OH | H |
| A-706 | I.1B' | $CH_2CH=CH_2$ | $NH_2$ | 3-OH | H |
| A-707 | I.1B' | $CH_2C\equiv CH$ | $NH_2$ | 3-OH | H |
| A-708 | I.1B' | H | $COOCH_3$ | 3-OH | H |
| A-709 | I.1B' | $CH_3$ | $COOCH_3$ | 3-OH | H |
| A-710 | I.1B' | $CH_2CH_3$ | $COOCH_3$ | 3-OH | H |
| A-711 | I.1B' | $CH_2CH_2CH_3$ | $COOCH_3$ | 3-OH | H |
| A-712 | I.1B' | $CH_2CH_2CH_2CH_3$ | $COOCH_3$ | 3-OH | H |
| A-713 | I.1B' | $CH_2CH=CH_2$ | $COOCH_3$ | 3-OH | H |
| A-714 | I.1B' | $CH_2C\equiv CH$ | $COOCH_3$ | 3-OH | H |
| A-715 | I.1B' | H | $CH_3$ | $3-OC(O)CH_3$ | H |
| A-716 | I.1B' | $CH_3$ | $CH_3$ | $3-OC(O)CH_3$ | H |
| A-717 | I.1B' | $CH_2CH_3$ | $CH_3$ | $3-OC(O)CH_3$ | H |
| A-718 | I.1B' | $CH_2CH_2CH_3$ | $CH_3$ | $3-OC(O)CH_3$ | H |
| A-719 | I.1B' | $CH_2CH_2CH_2CH_3$ | $CH_3$ | $3-OC(O)CH_3$ | H |

(fortgesetzt)

| Nr. | Formel | $R^1$ | $R^3$ | $R^9$ | $R^{10}$ |
|---|---|---|---|---|---|
| A-720 | I.1B' | $CH_2CH=CH_2$ | $CH_3$ | 3-OC(O)$CH_3$ | H |
| A-721 | I.1B' | $CH_2C≡CH$ | $CH_3$ | 3-OC(O)$CH_3$ | H |
| A-722 | I.1B' | H | $CH_2CH_3$ | 3-OC(O)$CH_3$ | H |
| A-723 | I.1B' | $CH_3$ | $CH_2CH_3$ | 3-OC(O)$CH_3$ | H |
| A-724 | I.1B' | $CH_2CH_3$ | $CH_2CH_3$ | 3-OC(O)$CH_3$ | H |
| A-725 | I.1B' | $CH_2CH_2CH_3$ | $CH_2CH_3$ | 3-OC(O)$CH_3$ | H |
| A-726 | I.1B' | $CH_2CH_2CH_2CH_3$ | $CH_2CH_3$ | 3-OC(O)$CH_3$ | H |
| A-727 | I.1B' | $CH_2CH=CH_2$ | $CH_2CH_3$ | 3-OC(O)$CH_3$ | H |
| A-728 | I.1B' | $CH_2C≡CH$ | $CH_2CH_3$ | 3-OC(O)$CH_3$ | H |
| A-729 | I.1B' | H | OH | 3-OC(O)$CH_3$ | H |
| A-730 | I.1B' | $CH_3$ | OH | 3-OC(O)$CH_3$ | H |
| A-731 | I.1B' | $CH_2CH_3$ | OH | 3-OC(O)$CH_3$ | H |
| A-732 | I.1B' | $CH_2CH_2CH_3$ | OH | 3-OC(O)$CH_3$ | H |
| A-733 | I.1B' | $CH_2CH_2CH_2CH_3$ | OH | 3-OC(O)$CH_3$ | H |
| A-734 | I.1B' | $CH_2CH=CH_2$ | OH | 3-OC(O)$CH_3$ | H |
| A-735 | I.1B' | $CH_2C≡CH$ | OH | 3-OC(O)$CH_3$ | H |
| A-736 | I.1B' | H | $CH_2OH$ | 3-OC(O)$CH_3$ | H |
| A-737 | 1.1B' | $CH_3$ | $CH_2OH$ | 3-OC(O)$CH_3$ | H |
| A-738 | I.1B' | $CH_2CH_3$ | $CH_2OH$ | 3-OC(O)$CH_3$ | H |
| A-739 | I.1B' | $CH_2CH_2CH_3$ | $CH_2OH$ | 3-OC(O)$CH_3$ | H |
| A-740 | I.1B' | $CH_2CH_2CH_2CH_3$ | $CH_2OH$ | 3-OC(O)$CH_3$ | H |
| A-741 | I.1B' | $CH_2CH=CH_2$ | $CH_2OH$ | 3-OC(O)$CH_3$ | H |
| A-742 | I.1B' | $CH_2C≡CH$ | $CH_2OH$ | 3-OC(O)$CH_3$ | H |
| A-743 | I.1B' | H | $NH_2$ | 3-OC(O)$CH_3$ | H |
| A-744 | I.1B' | $CH_3$ | $NH_2$ | 3-OC(O)$CH_3$ | H |

(fortgesetzt)

| Nr. | Formel | $R^1$ | $R^3$ | $R^9$ | $R^{10}$ |
|---|---|---|---|---|---|
| A-745 | I.1B' | $CH_2CH_3$ | $NH_2$ | 3-OC(O)$CH_3$ | H |
| A-746 | I.1B' | $CH_2CH_2CH_3$ | $NH_2$ | 3-OC(O)$CH_3$ | H |
| A-747 | I.1B' | $CH_2CH_2CH_2CH_3$ | $NH_2$ | 3-OC(O)$CH_3$ | H |
| A-748 | I.1B' | $CH_2CH=CH_2$ | $NH_2$ | 3-OC(O)$CH_3$ | H |
| A-749 | I.1B' | $CH_2C\equiv CH$ | $NH_2$ | 3-OC(O)$CH_3$ | H |
| A-750 | I.1B' | H | $COOCH_3$ | 3-OC(O)$CH_3$ | H |
| A-751 | I.1B' | $CH_3$ | $COOCH_3$ | 3-OC(O)$CH_3$ | H |
| A-752 | I.1B' | $CH_2CH_3$ | $COOCH_3$ | 3-OC(O)$CH_3$ | H |
| A-753 | I.1B' | $CH_2CH_2CH_3$ | $COOCH_3$ | 3-OC(O)$CH_3$ | H |
| A-754 | I.1B' | $CH_2CH_2CH_2CH_3$ | $COOCH_3$ | 3-OC(O)$CH_3$ | H |
| A-755 | I.1B' | $CH_2CH=CH_2$ | $COOCH_3$ | 3-OC(O)$CH_3$ | H |
| A-756 | I.1B' | $CH_2C\equiv CH$ | $COOCH_3$ | 3-OC(O)$CH_3$ | H |

Unter den voranstehend beispielhaft genannten Verbindungen und ihren Salzen sind solche Verbindungen und Salze bevorzugt, worin die exo-Doppelbindung am Piperazinring die (Z)-Konfiguration aufweist. Ebenso bevorzugt Gemische des (E)-Isomers mit dem (Z)-Isomer, worin das Z-Isomer im Überschuss vorliegt, insbesondere Isomerengemische mit einem E/Z-Verhältnis, von nicht mehr als 1:2 insbesondere nicht mehr als 1:5.

Unter den voranstehend beispielhaft genannten Verbindungen der Formel I.1B' und ihren Salzen sind solche Verbindungen und Salze bevorzugt, worin $R^3$ und das Wasserstoffatom in Position $R^4$ eine syn-Konfiguration aufweisen. Ebenso bevorzugt sind Gemische der syn und trans Isomeren, worin das syn-Isomer im Überschuss vorliegt, insbesondere Isomerengemische mit einem syn/trans-Verhältnis von nicht mehr als 1:2, insbesondere nicht mehr als 1:5.

Unter den hier beispielhaft genannten Verbindungen und ihren Salzen sind solche Verbindungen und Salze bevorzugt, worin das Kohlenstoffatom, welches die Gruppe $R^3$ trägt, S-Konfiguration aufweist, sowie Enantiomerenmischungen, die einen Enantiomerenüberschuss bezüglich des S-Enantiomers aufweisen, insbesondere solche mit einen ee-Wert (enantiomeric excess) von wenigstens 70 %, besonders bevorzugt wenigstens 80 % und bevorzugt wenigstens 90 %. Ebenso sind die Racemate dieser Verbindungen und ihrer Salze bevorzugt.

Die Verbindungen I.1 und deren landwirtschaftlich brauchbaren Salze eignen sich - sowohl als Isomerengemische als auch in Form der reinen Isomeren - als Herbizide. Sie eignen sich als solche oder als entsprechend formuliertes Mittel. Die herbiziden Mittel, die die Verbindung I.1, insbesondere die bevorzugten Ausgestaltungen davon, enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen 1.1, insbesondere die bevorzugten Ausgestaltungen davon, bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis und prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

Der Begriff Kulturpflanzen schließt auch solche ein, die durch Züchtung, Mutagenese oder gentechnische Methoden verändert wurden. Gentechnisch veränderte Pflanzen sind Pflanzen, deren genetisches Material in einer Weise verändert worden ist, wie sie unter natürlichen Bedingungen durch Kreuzen, Mutationen oder natürliche Rekombination (d.h. Neuzusammenstellung der Erbinformation) nicht vorkommt. Dabei werden in der Regel ein oder mehrere Gene in das Erbgut der Pflanze integriert, um die Eigenschaften der Pflanze zu verbessern.

Der Begriff Kulturpflanzen umfasst somit auch Pflanzen, die durch züchterische und gentechnische Maßnahmen eine Toleranz gegen bestimmter Herbizidklassen, wie Hydroxyphenylpyruvat-Dioxygenase (HPPD)-Inhibitoren, Acetolactat-Synthase (ALS)-Inhibitoren, wie z. B. Sulfonylharnstoffe (EP-A 257 993, US 5,013,659) oder Imidazolinone (siehe z. B. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073), Enolpyruvylshikimat-3-Phosphat-Synthase (EPSPS)-Inhibitoren wie z. B. Glyphosat (siehe z. B. WO 92/00377), Glutaminsynthetase (GS)-Inhibitoren wie z. B. Glufosinat (siehe z. B. EP-A 242 236, EP-A 242 246) oder Oxynil-Herbizide (siehe z. B. US 5,559,024) erworben haben.

Mit Hilfe klassischer Züchtungsmethoden (Mutagenese) wurden zahlreiche Kulturpflanzen, z. B. Clearfield®-Raps, erzeugt, die eine Toleranz gegen Imidazolinone, z. B. Imazamox, haben. Mit Hilfe gentechnischer Methoden wurden Kulturpflanzen, wie Soja, Baumwolle, Mais, Rüben und Raps, erzeugt, die resistent gegen Glyphosat oder Glufosinat sind, erzeugt, welche unter den Handelsnamen RoudupReady® (Glyphosat) und Liberty Link® (Glufosinat) erhältlich sind.

Der Begriff Kulturpflanzen umfasst somit auch Pflanzen, die mit Hilfe gentechnischer Maßnahmen ein oder mehrere Toxine, z. B. solche aus dem Bakterienstamm *Bacillus* ssp., produzieren. Toxine, die durch solche gentechnisch veränderten Pflanzen hergestellt werden, umfassen z. B. insektizide Proteine von *Bacillus* spp., insbesondere von *B. thuringiensis,* wie die Endotoxine Cry1Ab, Cry1Ac, Cry1F, Cry1Fa2, Cry2Ab, Cry3A, Cry3Bb1, Cry9c, Cry34Ab1 oder

Cry35Ab1; oder vegetative insektizide Proteine (VIPs), z. B. VIP1, VIP2, VIP3, oder VIP3A; insektizide Proteine von Nematodenkolonisierenden Bakterien, z. B. *Photorhabdus* spp. oder *Xenorhabdus* spp.; Toxine aus tierischen Organismen, z. B. Wepsen,-, Spinnen- oder Skorpionstoxine; pilzliche Toxine, z. B. aus Streptomyceten; pflanzliche Lektine, z. B. aus Erbse oder Gerste; Agglutinine; Proteinase-Inhibitoren, z. B. Trypsin-Inhibitoren, Serinprotease-Inhibitoren, Patatin, Cystatin oder Papain-Inhibitoren; Ribosomen-inaktivierende Proteine (RIPs), z. B. Ricin, Mais-RIP, Abrin, Luffin, Saporin oder Bryodin; Steroid-metabolisierende Enzyme, z. B. 3-Hydroxysteroid-Oxidase, Ecdysteroid-IDP-Glycosyl-Transferase, Cholesterinoxidase, Ecdyson-Inhibitoren oder HMG-CoA-Reduktase; Ionenkanalblocker, z. B. Inhibitoren von Natrium- oder Calziumkanälen; Juvenilhormon-Esterase; Rezeptoren für das diuretischen Hormon (Helicokininrezeptoren); Stilbensynthase, Bibenzylsynthase, Chitinasen und Glucanasen. Diese Toxine können in den Pflanzen auch als Prätoxine, Hybridproteine, verkürzte oder anderweitig modfizierte Proteine produziert werden. Hybridproteine zeichnen sich durch eine neue Kombination von verschiedenen Proteindomänen aus (siehe z. B. WO 2002/015701). Weitere Besipiele für derartige Toxine oder gentechnisch veränderte Pflanzen, die diese Toxine produzieren sind in EP-A 374 753, WO 93/007278, WO 95/34656, EP-A 427 529, EP-A 451 878, WO 03/018810 und WO 03/052073 offenbart. Die Methoden zur Herstellung dieser gentechnisch veränderten Pflanzen sind dem Fachmann bekannt und z. B. in den oben erwähnten Publikationen dargelegt. Zahlreiche der zuvor genannten Toxine verleihen den Pflanzen, die diese produzieren, eine Toleranz gegen Schädlinge aus allen taxonomischen Arthropodenklassen, insbesondere gegen Käfer (Coeleropta), Zweiflügler (Diptera) und Schmetterlinge (Lepidoptera) und gegen Nematoden (Nematoda).

Gentechnisch veränderte Pflanzen, die ein oder mehrere Gene, die für insektizide Toxine kodieren, produzieren sind z. B. in den oben erwähnten Publikationen beschrieben und zum Teil kommerziell erhältlich, wie z. B. YieldGard® (Maissorten, die das Toxin Cry1Ab produzieren), YieldGard® Plus (Maissorten, die die Toxine Cry1Ab und Cry3Bb1 produzieren), Starlink® (Maissorten, die das Toxin Cry9c produzieren), Herculex® RW (Maissorten, die die Toxine Cry34Ab1, Cry35Ab1 und das Enzym Phosphinothricin-N-Acetyltransferase [PAT] produzieren); NuCOTN® 33B (Baumwollsorten, die das Toxin Cry1Ac produzieren), Bollgard® I (Baumwollsorten, die das Toxin Cry1Ac produzieren), Bollgard® II (Baumwollsorten, die die Toxine Cry1Ac und Cry2Ab2 produzieren); VIPCOT® (Baumwollsorten, die ein VIP-Toxin produzieren); NewLeaf® (Kartoffelsorten, die das Toxin Cry3A produzieren); Bt-Xtra®, NatureGard®, KnockOut®, BiteGard®, Protecta®, Bt11 (z. B. Agrisure® CB) und Bt176 von Syngenta Seeds SAS, Frankreich, (Maissorten, die das Toxin Cry1Ab und das PAT-Enyzm produzieren), MIR604 von Syngenta Seeds SAS, Frankreich (Maissorten, die ein modifizierte Version des Toxins Cry3A produzieren, siehe hierzu WO 03/018810), MON 863 von Monsanto Europe S.A., Belgien (Maissorten, die das Toxin Cry3Bb1 produzieren), IPC 531 von Monsanto Europe S.A., Belgien (Baumwollsorten, die eine modifizierte Version des Toxins Cry1Ac produzieren) und 1507 von Pioneer Overseas Corporation, Belgien (Maissorten, die das Toxin Cry1F und das PAT-Enyzm produzieren).

Der Begriff Kulturpflanzen umfasst somit auch Pflanzen, die mit Hilfe gentechnischer Maßnahmen ein oder mehrere Proteine produzieren, die eine erhöhte Resistenz oder Widerstandfähigkeit gegen bakterielle, virale oder pilzliche Pathogene bewirken, wie z. B. sogenannte Pathogenesis-related-Proteine (PR-Proteine, siehe EP-A 0 392 225), Resistenzproteine (z. B. Kartoffelsorten, die zwei Resistenzgene gegen *Phytophthora infestans* aus der mexikanischen Wildkartoffel *Solanum bulbocastanum* produzieren) oder T4-Lysozym (z. B. Kartoffelsorten, die durch die Produktion diese Proteins resistent gegen Bakterien wie *Erwinia amylvora* ist).

Der Begriff Kulturpflanzen umfasst somit auch Pflanzen, deren Produktivität mit Hilfe gentechnischer Methoden verbessert wurde, indem z. B. die Ertragsfähigkeit (z. B. Biomasse, Kornertrag, Stärke-, Öl- oder Proteingehalt), die Toleranz gegenüber Trockenheit, Salz oder anderen begrenzenden Umweltfaktoren oder die Widerstandsfähigkeit gegenüber Schädlingen und pilzlichen, bakteriellen und viralen Pathogenen gesteigert wird.

Der Begriff Kulturpflanzen umfasst auch Pflanzen, deren Inhaltsstoffe insbesondere zur Verbesserung der menschlichen oder tierischen Ernährung mit Hilfe gentechnischer Methoden verändert wurden, indem z. B. Ölpflanzen gesundheitsfördernde langkettige Omega-3-Fettsäuren oder einfach ungesättigte Omega-9-Fettsäuren (z. B. Nexera®-Raps) produzieren.

Der Begriff Kulturpflanzen umfasst auch Pflanzen, die zur verbesserten Produktion von Rohstoffen mit Hilfe gentechnischer Methoden verändert wurden, indem z. B. der Amylopektin-Gehalt von Kartoffeln (Amflora®-Kartoffel) erhöht wurde.

Des Weiteren wurde gefunden, dass die Verbindungen der Formel I.1 auch zur Defoliation und/oder Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Verbindungen der Formel I.1 gefunden.

Als Desikkantien eignen sich die Verbindungen der Formel I.1 insbesondere zur Austrocknung der oberirdischen Teile von Kulturpflanzen wie Kartoffel, Raps, Sonnenblume und Sojabohne aber auch Getreide. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-,

Stein- und Schalenobst ermöglicht wird. Derselbe Mechanismus, d.h., die Förderung der Ausbildung von Trenngewebe zwischen Frucht- oder Blatt- und Sprossteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Qualität der Faser nach der Ernte.

Die Verbindungen I.1 bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I.1 oder eines landwirtschaftlich brauchbaren Salzes von I.1 und für die Formulierung von Pflanzenschutzmitteln übliche Hilfsstoffe.

Beispiele für die Formulierung von Pflanzenschutzmitteln übliche Hilfsmittel sind inerte Hilfsstoffe, feste Trägerstoffe, oberflächenaktive Stoffe (wie Dispergiermittel Schutzkolloide, Emulgatoren, Netzmittel und Haftmittel), organische und anorganische Verdicker, Bakterizide, Frostschutzmittel, Entschäumer ggf. Farbstoffe und für Saatgutformulierungen Kleber.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide wie Xanthan Gum (Kelzan® der Fa. Kelco), Rhodopol® 23 (Rhone Poulenc) oder Veegum® (Firma R.T. Vanderbilt) sowie organische und anorganische Schichtmineralienwie Attaclay® (Firma Engelhardt).

Beispiele für Antischaummittel sind Silikonemulsionen (wie z.Bsp. Silikon® SRE, Firma Wacker oder Rhodorsil® der Firma Rhodia ), langkettige Alkohole, Fettsäuren, Salze von Fettsäuren, fluororganische Verbindungen und deren Gemische.

Bakterizide können zur Stabilisierung der wäßrigen Herbizid-Formulierung zugesetzt werden. Beispiele für Bakterizide sind Bakterizide basierend auf Diclorophen und Benzylalkoholhemiformal (Proxel® der Fa. ICI oder Acticide® RS der Fa. Thor Chemie und Kathon® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C.I. Pigment Red 112 und C.I. Solvent Red 1 bekannten Farbstoffe, sowie pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Beispiele für Kleber sind Polyvinylpyrrolidon, Polyvinylacetat, Polyvinylalkohol und Tylose.

Als inerte Zusatzstoffe kommen beispielsweise in Betracht

Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Als oberflächenaktive Stoffe (Adjuvantien, Netz-, Haft-, Dispergier- sowie Emulgiermittel) kommen die Alkali-, Erdalkali-, Ammoniumsalze von aromatischen Sulfonsäuren, z.B. Ligninsulfonsäuren (z.B. Borrespers-Typen, Borregaard), Phenolsulfonsäuren, Naphthalinsulfonsäuren (Morwet-Typen, Akzo Nobel) und Dibutylnaphthalinsulfonsäure (Nekal-Typen, BASF SE), sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen sowie Proteine, denaturierte Proteine, Polysaccharide

(z.B. Methylcellulose), hydrophob modifizierte Stärken, Polyvinylalkohol (Mowiol typen Clariant), Polycarboxylate (BASF SE, Sokalan-Typen), Polyalkoxylate, Polyvinylamin (BASF SE, Lupamin-Typen), Polyethylenimin (BASF SE, Lupasol-Typen), Polyvinylpyrrolidon und deren Copolymere in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I.1 oder Ia als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Konzentrationen der Verbindungen der Formel I.1 in den anwendungsfertigen Zubereitungen können in weiten Bereichen variiert werden. Die Formulierungen enthalten im Allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I.1 können beispielsweise wie folgt formuliert werden:

1. Produkte zur Verdünnung in Wasser

A Wasserlösliche Konzentrate
10 Gew.-Teile Wirkstoff werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

B Dispergierbare Konzentrate
20 Gew.-Teile Wirkstoff werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

C Emulgierbare Konzentrate
15 Gew.-Teile Wirkstoff werden in 75 Gew.-Teilen eines organisches Lösungsmittels (z.B. Alkylaromaten)-unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

D Emulsionen
25 Gew.-Teile Wirkstoff werden in 35 Gew.-Teilen eines organisches Lösungsmittels (z.B. Alkylaromaten) unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

E Suspensionen
20 Gew.-Teile Wirkstoff werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

F Wasserdispergierbare und wasserlösliche Granulate
50 Gew.-Teile Wirkstoff werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

G Wasserdispergierbare und wasserlösliche Pulver
75 Gew.-Teile Wirkstoff werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

H Gelformulierungen
In einer Kugelmühle werden 20 Gew.-Teile Wirkstoff, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

2. Produkte für die Direktapplikation

I Stäube
5 Gew.-Teile Wirkstoff werden fein gemahlen und mit 95 Gew.-Teilen feinteiligem Kaolin innig vermischt. Man erhält dadurch ein Stäubemittel mit 5 Gew.-% Wirkstoffgehalt.
J Granulate (GR, FG, GG, MG)
0,5 Gew-Teile Wirkstoff werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.
K ULV- Lösungen (UL)
10 Gew.-Teile Wirkstoff werden in 90 Gew.-Teilen eines organischen Lösungsmittels z.B. Xylol gelöst. Dadurch erhält man ein Produkt für die Direktapplikation mit 10 Gew.-% Wirkstoffgehalt.

Die Applikation der Verbindungen I.1 oder der sie enthaltenden herbiziden Mittel kann im Vorauflauf-, im Nachauflaufverfahren oder zusammen mit dem Saatgut einer Kulturpflanze erfolgen. Es besteht auch die Möglichkeit, die herbiziden Mittel bzw. Wirkstoffe dadurch zu applizieren, dass mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In einer weiteren Ausführungsform kann die Applikation der Verbindungen der Formel I.1 bzw. der herbiziden Mittel durch Behandlung von Saatgut erfolgen.

Die Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting) basierend auf den erfindungsgemäßen Verbindungen der Formel I.1 bzw. daraus hergestellten Mitteln. Hierbei können die herbiziden Mittel verdünnt oder unverdünnt aufgetragen werden.

Der Begriff Saatgut umfasst Saatgut aller Arten, wie z.B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.). Zur Saatgutbehandlung werden die Verbindungen I.1 üblicherweise in Mengen von 0,001 bis 10 kg pro 100 kg Saatgut eingesetzt.

Es kann auch von Vorteil sein, die Verbindungen der Formel 1.1 in Kombination mit Safenern zu verwenden. Safener sind chemische Verbindungen, die Schaden an Nutzpflanzen verhindern oder reduzieren, ohne die herbizide Wirkung der Verbindungen der Formel I.1 auf unerwünschte Pflanzen wesentlich zu beeinflussen. Sie können sowohl vor der Aussaat (beispielsweise bei Saatgutbehandlungen, bei Stecklingen oder Setzlingen) als auch im Vor- oder Nachauflauf der Nutzpflanze verwendet werden. Die Safener und die Verbindungen der Formel I.1 können gleichzeitig oder nacheinander verwendet werden. Geeignete Safener sind beispielsweise (Chinolin-8-oxy)essigsäuren, 1-Phenyl-5-haloalkyl-1*H*-1,2,4-triazol-3-carbonsäuren, 1-Phenyl-4,5-dihydro-5-alkyl-1*H*-pyrazol-3,5-dicarbonsäuren, 4,5-Dihydro-5,5-diaryl-3-isoxazolcarbonsäuren, Dichloroacetamide, alpha-Oximinophenylacetonitrile, Acetophenonoxime, 4,6-Dihalo-2-phenylpyrimidine, N-[[4-(Aminocarbonyl)phenyl]sulfonyl]-2-benzoesäureamide, 1,8-Naphthalsäureanhydrid, 2-Halo-4-(haloalkyl)-5-thiazolcarbonsäuren, Phosphorthiolate und N-Alkyl-O-phenylcarbamate sowie ihre landwirtschaftlich brauchbaren Salze, und vorausgesetzt sie haben eine Säurefunktion, ihre landwirtschaftlich brauchbaren Derivate, wie Amide, Ester und Thioester.

Zur Verbreiterung des Wirkungsspektrums und zur Erzielung synergistischer Effekte können die Verbindungen der Formel I.1 mit zahlreichen Vertretern anderer herbizider oder wachstumsregulierender Wirkstoffgruppen oder mit Safenern gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-$CF_3$-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile sowie Phenylpyrazoline und Isoxazoline und deren Derivate in Betracht.

Außerdem kann es von Nutzen sein, die Verbindungen I.1 allein oder in Kombination mit anderen Herbiziden oder auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch weitere Additve wie nicht phytotoxische Öle und Ölkonzentrate zugesetzt werden.

Beispiele für Herbizide, die in Kombination mit den Piperazindionverbindungen der Formel I.1 gemäß der vorliegenden Erfindung verwendet werden können, sind:

b1) aus der Gruppe der Lipid-Biosynthese-Inhibitoren:

Alloxydim, Alloxydim-natrium, Butroxydim, Clethodim, Clodinafop, Clodinafop-propargyl, Cycloxydim, Cyhalofop, Cyhalofop-butyl, Diclofop, Diclofop-methyl, Fenoxaprop, Fenoxaprop-ethyl, Fenoxaprop-P, Fenoxaprop-P-ethyl, Fluazifop, Fluazifop-butyl, Fluazifop-P, Fluazifop-P-butyl, Haloxyfop, Haloxyfop-methyl, Haloxyfop-P, Haloxyfop-P-methyl, Metamifop, Pinoxaden, Profoxydim, Propaquizafop, Quizalofop, Quizalofopethyl, Quizalofop-tefuryl, Quizalofop-P, Quizalofop-P-ethyl, Quizalofop-P-tefuryl, Sethoxydim, Tepraloxydim, Tralkoxydim, Benfuresat, Butylat, Cycloat, Dalapon, Dimepiperat, EPTC, Esprocarb, Ethofumesat, Flupropanat, Molinat, Orbencarb, Pebulat, Prosulfocarb, TCA, Thiobencarb, Tiocarbazil, Triallat und Vernolat;

b2) aus der Gruppe der ALS-Inhibitoren:

Amidosulfuron, Azimsulfuron, Bensulfuron, Bensulfuron-methyl, Bispyribac, Bispyribacnatrium, Chlorimuron, Chlorimuron-ethyl, Chlorsulfuron, Cinosulfuron, Cloransulam, Cloransulam-methyl, Cyclosulfamuron, Diclosulam, Ethametsulfuron, Ethametsulfuronmethyl, Ethoxysulfuron, Flazasulfuron, Florasulam, Flucarbazon, Flucarbazon-natrium, Flucetosulfuron, Flumetsulam, Flupyrsulfuron, Flupyrsulfuron-methyl-natrium, Foramsulfuron, Halosulfuron, Halosulfuron-methyl, Imazamethabenz, Imazamethabenzmethyl, Imazamox, Imazapic, Imazapyr, Imazaquin, Imazethapyr, Imazosulfuron, Iodosulfuron, Iodosulfuron-methyl-natrium, Mesosulfuron, Metosulam, Metsulfuron, Metsulfuron-methyl, Nicosulfuron, Orthosulfamuron, Oxasulfuron, Penoxsulam, Primisulfuron, Primisulfuron-methyl, Propoxycarbazon, Propoxycarbazon-natrium, Prosulfuron, Pyrazosulfuron, Pyrazosulfuron-ethyl, Pyribenzoxim, Pyrimisulfan, Pyriftalid, Pyriminobac, Pyriminobac-methyl, Pyrithiobac, Pyrithiobac- natrium, Pyroxsulam, Rimsulfuron, Sulfometuron, Sulfometuron-methyl, Sulfosulfuron, Thiencarbazon, Thiencarbazon-methyl, Thifensulfuron, Thifensulfuron-methyl, Triasulfuron, Tribenuron, Tribenuron-methyl, Trifloxysulfuron, Triflusulfuron, Triflusulfuron-methyl und Tritosulfuron;

b3) aus der Gruppe der Photosynthese-Inhibitoren:

Ametryn, Amicarbazon, Atrazin, Bentazon, Bentazon-natrium, Bromacil, Bromofenoxim, Bromoxynil und seine Salze und Ester, Chlorobromuron, Chloridazon, Chlorotoluron, Chloroxuron, Cyanazin, Desmedipham, Desmetryn, Dimefuron, Dimethametryn, Diquat, Diquat-dibromid, Diuron, Fluometuron, Hexazinon, Ioxynil und seine Salze und Ester, Isoproturon, Isouron, Karbutilat, Lenacil, Linuron, Metamitron, Methabenzthiazuron, Metobenzuron, Metoxuron, Metribuzin, Monolinuron, Neburon, Paraquat, Paraquat-dichlorid, Paraquat-dimetilsulfat, Pentanochlor, Phenmedipham, Phenmediphamethyl, Prometon, Prometryn, Propanil, Propazin, Pyridafol, Pyridat, Siduron, Simazin, Simetryn, Tebuthiuron, Terbacil, Terbumeton, Terbuthylazin, Terbutryn, Thidiazuron und Trietazin;

b4) aus der Gruppe der Protoporphyrinogen-IX-Oxidase-Inhibitoren:

Acifluorfen, Acifluorfen-natrium, Azafenidin, Bencarbazon, Benzfendizon, Bifenox, Butafenacil, Carfentrazon, Carfentrazon-ethyl, Chlomethoxyfen, Cinidon-ethyl, Fluazolat, Flufenpyr, Flufenpyr-ethyl, Flumiclorac, Flumiclorac-pentyl, Flumioxazin, Fluoroglycofen, Fluoroglycofen-ethyl, Fluthiacet, Fluthiacet-methyl, Fomesafen, Halosafen, Lactofen, Oxadiargyl, Oxadiazon, Oxyfluorfen, Pentoxazon, Profluazol, Pyraclonil, Pyraflufen, Pyraflufen-ethyl, Saflufenacil, Sulfentrazon, Thidiazimin, 2-Chlor-5-[3,6-dihydro-3-methyl-2,6-dioxo-4-(trifluormethyl)-1 (2H)-pyrimidinyl]-4-fluor-N-[(isopropyl)methylsulfamoyl]benzamid (CAS 372137-35-4), [3-[2-Chlor-4-fluor-5-(1-methyl-6-trifluormethyl-2,4-dioxo-1,2,3,4,-tetrahydropyrimidin-3-yl)phenoxy]-2-pyridyloxy]essigsäure Ethyl Ester (CAS 353292-31-6), N-Ethyl-3-(2,6-dichlor-4-trifluormethylphenoxy)-5-methyl-1 *H*-pyrazol-1-carboxamid (CAS 452098-92-9), N-Tetrahydrofurfuryl-3-(2,6-dichlor-4-trifluormethylphenoxy)-5-methyl-1 *H*-pyrazol-1-carboxamid (CAS 915396-43-9), N-Ethyl-3-(2-chlor-6-fluor-4-trifluormethylphenoxy)-5-methyl-1*H*-pyrazol-1-carboxamid (CAS 452099-05-7) und N-Tetrahydrofurfuryl-3-(2-chlor-6-fluor-4-trifluormethylphenoxy)-5-methyl-1*H*-pyrazol-1-carboxamid (CAS 452100-03-7);

b5) aus der Gruppe der Bleacher-Herbizide:

Aclonifen, Amitrol, Beflubutamid, Benzobicyclon, Benzofenap, Clomazon, Diflufenican, Fluridon, Flurochloridon, Flurtamon, Isoxaflutol, Mesotrion, Norflurazon, Picolinafen, Pyrasulfutol, Pyrazolynat, Pyrazoxyfen, Sulcotrion, Tefuryltrion, Tembotrion, Topramezon, 4-Hydroxy-3-[[2-[(2-methoxyethoxy)methyl]-6-(trifluormethyl)-3-pyridyl]carbonyl]bicyclo[3.2.1]oct-3-en-2-one (CAS 352010-68-5) und 4-(3-Trifluormethylphenoxy)-2-(4-trifluormethylphenyl)pyrimidin (CAS 180608-33-7);

b6) aus der Gruppe der EPSP-Synthase-Inhibitoren:

Glyphosat, Glyphosat-isopropylammonium und Glyphosat-trimesium (Sulfosat);

b7) aus der Gruppe der Glutamin-Synthase-Inhibitoren:

Bilanaphos (Bialaphos), Bilanaphos-natrium, Glufosinat und Glufosinat-ammonium;

b8) aus der Gruppe der DHP-Synthase-Inhibitoren: Asulam;

b9) aus der Gruppe der Mitose-Inhibitoren:

Amiprophos, Amiprophos-methyl, Benfluralin, Butamiphos, Butralin, Carbetamid, Chlorpropham, Chlorthal, Chlorthal-dimethyl, Dinitramin, Dithiopyr, Ethalfluralin, Fluchloralin, Oryzalin, Pendimethalin, Prodiamin, Propham, Propyzamid, Tebutam, Thiazopyr und Trifluralin;

b10) aus der Gruppe der VLCFA-Inhibitoren:

Acetochlor, Alachlor, Anilofos, Butachlor, Cafenstrol, Dimethachlor, Dimethanamid, Dimethenamid-P, Diphenamid, Fentrazamid, Flufenacet, Mefenacet, Metazachlor, Metolachlor, Metolachlor-S, Naproanilid, Napropamid, Pethoxamid, Piperophos, Pretilachlor, Propachlor, Propisochlor, Pyroxasulfon (KIH-485) und Thenylchlor;

Verbindungen der Formel 2:

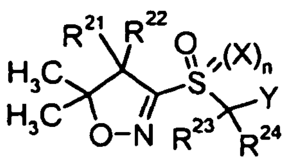

2

worin die Variablen folgende Bedeutungen haben:

Y Phenyl oder 5- oder 6-gliedriges Heteroaryl wie eingangs definiert, welche durch eine bis drei Gruppen $R^{aa}$ substituiert sein können;
$R^{21}$, $R^{22}$, $R^{23}$, $R^{24}$ H, Halogen, oder $C_1$-$C_4$-Alkyl;
X O oder NH;
n 0 oder 1.
Verbindungen der Formel 2 weisen insbesondere die folgenden Bedeutungen auf:
Y

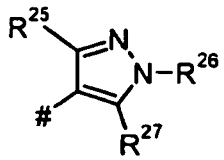 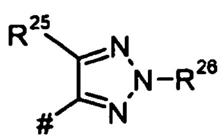 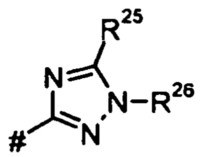 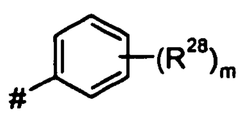

,

wobei # die Bindung zu dem Molekülgerüst bedeutet;
$R^{21}$, $R^{22}$, $R^{23}$, $R^{24}$ H, Cl, F oder $CH_3$;
$R^{25}$ Halogen, $C_1$-$C_4$-Alkyl oder $C_1$-$C_4$-Haloalkyl,
$R^{26}$ $C_1$-$C_4$-Alkyl;
$R^{27}$ Halogen, $C_1$-$C_4$-Alkoxy oder $C_1$-$C_4$-Haloalkoxy;
$R^{28}$ H, Halogen, $C_1$-$C_4$-Akyl, $C_1$-$C_4$-Haloalkyl oder $C_1$-$C_4$-Haloalkoxy; m 0, 1, 2 oder 3;
X Sauerstoff;
n 0 oder 1.
Bevorzugte Verbindungen der Formel 2 weisen folgende Bedeutungen auf:
Y

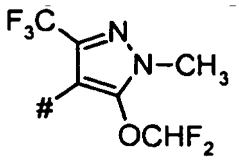 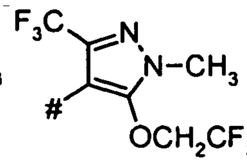 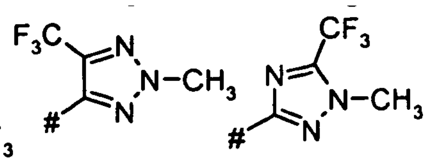 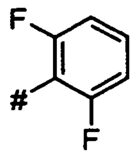

$R^{21}$ H;
$R^{22}$, $R^{23}$ F;
$R^{24}$ H oder F;
X Sauerstoff;
n 0 oder 1.

Besonders bevorzugte Verbindungen der Formel 2 sind:

3-[5-(2,2-Difluor-ethoxy)-1-methyl-3-trifluormethyl-1 H-pyrazol-4-ylmethansulfonyl]-4-fluor-5,5-dimethyl-4,5-dihydro-isoxazol; 3-{[5-(2,2-Difluor-ethoxy)-1-methyl-3-trifluormethyl-1H-pyrazol-4-yl]-fluor-methansulfonyl}-5,5-dimethyl-4,5-dihydro-isoxazol; 4-(4-Fluor-5,5-dimethyl-4,5-dihydro-isoxazol-3-sulfonylmethyl)-2-methyl-5-trifluormethyl-2H-[1,2,3]triazol; 4-[(5,5-Dimethyl-4,5-dihydro-isoxazol-3-sulfonyl)-fluor-methyl]-2-methyl-5-trifluormethyl-2H-[1,2,3]triazol; 4-(5,5-Dimethyl-4,5-dihydro-isoxazol-3-sulfonylmethyl)-2-methyl-5-trifluormethyl-2H-[1,2,3]triazol; 3-{[5-(2,2-Difluor-ethoxy)-1-methyl-3-trifluormethyl-1 H-pyrazol-4-yl]-difluor-methansulfonyl}-5,5-dimethyl-4,5-dihydro-isoxazol;
4-[(5,5-Dimethyl-4,5-dihydro-isoxazol-3-sulfonyl)-difluor-methyl]-2-methyl-5-trifluormethyl-2H-[1,2,3]triazol; 3-{[5-(2,2-Difluor-ethoxy)-1-methy)-3-trifluormethyl-1 H-pyrazol4-yl]-difluor-methansulfonyl}-4-fluor-5,5-dimethyl-4,5-dihydro-isoxazol; 4-[Difluor-(4-fluor-5,5-dimethyl-4,5-dihydro-isoxazol-3-sulfonyl)-methyl]-2-methyl-5-trifluormethyl-2H-[1,2,3]triazol;

b11) aus der Gruppe der Cellulose-Biosynthese-Inhibitoren:

Chlorthiamid, Dichlobenil, Flupoxam und Isoxaben;

b12) aus der Gruppe der Entkoppler-Herbizide:

Dinoseb, Dinoterb und DNOC und seine Salze;

b13) aus der Gruppe der Auxin-Herbizide:

2,4-D und seine Salze und Ester, 2,4-DB und seine Salze und Ester, Aminopyralid und seine Salze wie Aminopyralid-tris(2-hydroxypropyl)ammonium und seine Ester, Benazolin, Benazolin-ethyl, Chloramben und seine Salze und Ester, Clomeprop, Clopyralid und seine Salze und Ester, Dicamba und seine Salze und Ester, Dichlorprop und seine Salze und Ester, Dichlorprop-P und seine Salze und Ester, Fluroxypyr, Fluroxypyr-butometyl, Fluroxypyr-meptyl, MCPA und seine Salze und Ester, MCPAthioethyl, MCPB und seine Salze und Ester, Mecoprop und seine Salze und Ester, Mecoprop-P und seine Salze und Ester, Picloram und seine Salze und Ester, Quinclorac, Quinmerac, TBA (2,3,6) und seine Salze und Ester, Triclopyr und seine Salze und Ester, und 5,6-Dichloro-2-cyclopropyl-4-pyrimidinecarbonsäure (CAS 858956-08-8) und seine Salze und Ester;

b14) aus der Gruppe der Auxin-Transport-Inhibitoren: Diflufenzopyr, Diflufenzopyrnatrium, Naptalam und Naptalam-natrium;

b15) aus der Gruppe der sonstigen Herbizide: Bromobutid, Chlorflurenol, Chlorflurenol-methyl, Cinmethylin, Cumyluron, Dalapon, Dazomet, Difenzoquat, Difenzoquatmetilsulfate, Dimethipin, DSMA, Dymron, Endothal und seine Salze, Etobenzanid, Flamprop, Flamprop-isopropyl, Flamprop-methyl Flamprop-M-isopropyl, Flamprop-M-methyl, Flurenol, Flurenol-butyl, Flurprimidol, Fosamin, Fosamine-ammonium, Indanofan, Maleinsäure-hydrazid, Mefluidid, Metam, Methylazid, Methylbromid, Methyldymron, Methyljodid. MSMA, Ölsäure, Oxaziclomefon, Pelargonsäure, Pyributicarb, Quinoclamin, Triaziflam, Tridiphan und 6-Chlor-3-(2-cyclopropyl-6-methylphenoxy)-4-pyridazinol (CAS 499223-49-3) und seine Salze und Ester.

Beispiele für bevorzugte Safener sind Benoxacor, Cloquintocet, Cyometrinil, Cyprosulfamid, Dichlormid, Dicyclonon, Dietholate, Fenchlorazol, Fenclorim, Flurazol, Fluxofenim, Furilazol, Isoxadifen, Mefenpyr, Mephenat, Naphthalsäureanhydrid, Oxabetrinil, 4-(Dichloracetyl)-1-oxa-4-azaspiro[4.5]decan (MON4660, CAS 71526-07-3) und 2,2,5-Trimethyl-3-(dichloracetyl)-1,3-oxazolidin (R-29148, CAS 52836-31-4). Die Wirkstoffe der Gruppen b1) bis b15) und die Safener sind bekannte Herbizide und Safener, siehe z. B. The Compendium of Pesticide Common Names (http: //www.alanwood.net/pesticides/); B. Hock, C. Fedtke, R. R. Schmidt, Herbizide, Georg Thieme Verlag, Stuttgart 1995. Weitere herbizide Wirkstoffe sind aus WO 96/26202, WO 97/41116, WO 97/41117, WO 97/41118, WO 01/83459 und WO 2008/074991 sowie aus W. Krämer et al. (ed.) "Modern Crop Protection Compounds", Vol. 1, Wiley VCH, 2007 und der darin zitierten Literatur bekannt.

Die Verbindungen I.1 und die erfindungsgemäßen Zusammensetzungen können auch eine pflanzenstärkende Wirkung aufweisen. Sie eigenen sich daher zu Mobilisierung pflanzeneigener Abwehrkräfte gegen Befall durch unerwünschte Mikroorganismen, wie Schadpilze, aber auch Viren und Bakterien. Unter pflanzenstärkenden (resistenzinduzierenden) Stoffen sind in diesem Zusammenhang solche Substanzen zu verstehen, die in der Lage sind, das Abwehrsystem von behandelten Pflanzen so zu stimulieren, dass diese bei nachfolgender Inokulation mit unerwünschten Mikroorganismen weitgehende Resistenz gegen diese Mikroorganismen entfalten.

Die Verbindungen I.1 können eingesetzt werden, um Pflanzen innerhalb eines gewissen Zeitraumes nach der Behandlung gegen den Befall durch unerwünschte Mikroorganismen zu schützen. Der Zeitraum, innerhalb dessen Schutz herbeigeführt wird, erstreckt sich im Allgemeinen auf 1 bis 28 Tage, vorzugsweise 1 bis 14 Tage nach der Behandlung der Pflanzen mit den Verbindungen I.1 bzw. nach Behandlung des Saatguts, auf bis zu 9 Monate nach Aussaat.

Die Verbindungen I.1 und die erfindungsgemäßen Zusammensetzungen eignen sich auch zur Steigerung des Emteertrages.

Sie sind außerdem mindertoxisch und weisen eine gute Pflanzenverträglichkeit auf.

Im Folgenden wird die Herstellung von Piperazinverbindungen der Formel I.1 anhand von Beispielen erläutert ohne dabei den Gegenstand der vorliegenden Erfindung auf die gezeigten Beispiele zu begrenzen.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I.1 benutzt. Die so erhaltenen Verbindungen sind in der anschließenden Tabelle mit physikalischen Angaben aufgeführt

Die Charakterisierung der im Folgenden gezeigten Produkte erfolgte durch Bestimmung des Schmelzpunktes, durch NMR-Spektroskopie oder anhand der durch HPLC-MS-Spektrometrie ermittelten Massen ([m/z]) oder Retentionszeit (RT; [min.]).

[HPLC-MS = High Performance Liquid Chromatographie kombiniert mit Massen Spektrometrie; HPLC-Säule:

a) RP-18 Säule (Chromolith Speed ROD von Merck KgaA, Deutschland), 50*4,6 mm; Eluent: Acetonitril + 0,1 % Trifluoressigsäure (TFA)/ Wasser + 0,1 % TFA, mit einem Gradienten von 5 : 95 bis 100 : 0 in 5 Minuten bei 40°C, Flussrate 1,8 ml/min; oder

b) RP-18 Säule (XTerra MS 5mm von Waters) Eluent: Acetonitril + 0.1 % Ameisensäure (A)/ Wasser + 0.1 % Ameisensäure (B) mit einem Gradienten von 5:95 (A/B) bis 100:0 (A/B) in 8 Minuten bei 20-25°C, Flussrate 2 ml/min. MS: Quadrupol Elektrospray-Ionisation, 80 V (Positiv-Modus).]

Sofern nicht gegenteilig gekennzeichnet, wurden die HPLC/MS Daten mit Methode a) ausgenommen.

I. Herstellungsbeispiele

Beispiel 1: Herstellung von Herstellung von 1-Acetyl-6-benzyl-3-[1-(2-chlorpyridin-3-yl)-methyliden]-6-methylpiperazin-2,5-dion

Eine Lösung von 24,1 g 1,4-Diacetyl-3-benzyl-piperazin-2,5-dion (vgl. PCT/EP2008/057329) in 250 ml Dimethylformamid (DMF) wurde mit 11,3 g 2-Chlornicotinaldehyd und 16,5 g $K_2CO_3$ versetzt. Das Reaktionsgemisch wurde für 18 Std. bei 20-25°C gerührt, anschließend mit 500 ml Wasser und 10 g Zitronensäure versetzt und mehrmals mit $CH_2Cl_2$ extrahiert. Die vereinten organischen Phasen wurden nach Waschen mit Wasser getrocknet, filtriert und unter vermindertem Druck vom Lösungsmittel befreit. Nach Umkristallisieren aus Essigsäureethylester (EE) wurden 25 g der Titelverbindung als gelbe Kristalle vom Fp 190°C erhalten.

Beispiel 2: Herstellung von 3-Benzyl-6-[1-(2-chlorpyridin-3-yl)-methyliden]-3-methylpiperazin-2,5-dion

Eine Lösung von 25 g der Verbindung aus Beispiel 1 in 75 ml DMF wurde mit 3,5 g Hydrazinhydrat versetzt. Das Reaktionsgemisch wurde 18 Std. bei 20-25°C gerührt, anschließend mit Wasser versetzt, der ausgefallene Feststoff abfiltriert und mit Wasser und kaltem Aceton gewaschen. 20 g der Titelverbindung wurden erhalten.
HPLC-MS [m/z]: 371.2 $[M]^+$.

Beispiel 3: Herstellung von 3-Benzyl-6-[1-(2-chlorpyridin-3-yl)-methyliden]-1,3,4-trimethyl-piperazin-2,5-dion

Eine Lösung von 23 g der Verbindung aus Beispiel 2 in 230 ml DMF wurde bei 0°C mit 5,38g (60 %ig) NaH versetzt. Das Reaktionsgemisch wurde 2 Std. bei 0°C gerührt und anschließend mit 38,2 g $CH_3I$versetzt. Das Reaktionsgemisch wurde 18 Std. bei 20-25°C gerührt, dann mit Wasser versetzt. Es wurde mehrfach mit Methyl-tert-butylether (MTBE) extrahiert. Die vereinten organischen Phasen wurden nach Waschen mit Wasser getrocknet, filtriert und unter vermindertem Druck vom Lösungsmittel befreit. Nach Umkristallisieren aus MTBE wurden 21 g der Titelverbindung mit einem Fp. von 146°C erhalten. HPLC-MS [m/z]: 370.2 $[M+H]^+$

Beispiel 3b: Analog wurde 3-Benzyl-6-[1-(2-iodopyridin-3-yl)-methyliden]-1,3,4-trimethyl-piperazin-2,5-dion hergestellt.

HPLC-MS [m/z]: 461.8 $[M+1]^+$

Beispiel 4: Herstellung von 3-(5-Benzyl-1,4,5-trimethyl-3,6-dioxo-piperazin-2-ylidenmethyl)-pyridin-2-carbonitril

Eine Lösung von 14,8 g der Verbindung aus Beispiel 3 in 100 ml N-Methylpyrrolidin (NMP) wurde mit 7,2 g CuCN und 7,6 g CuI versetzt. Das Reaktionsgemisch wurde 16 Std. bei 155 C gerührt und nach Abkühlung auf 20-25°C in EE eingetragen. Das Gemisch wurde mit MTBE verdünnt, dann die so erhaltene organische Phase nach Waschen mit Wasser, Trocknen und Filtrieren unter vermindertem Druck vom Lösungsmittel befreit. Aus dem Rückstand wurden nach säulenchromatographischer Reinigung 12,1g der Titelverbindung vom Fp. 163°C erhalten. HPLC-MS [m/z]: 361.5 [M+1]+

Beispiel 5: Herstellung von 3-Benzyl-6-[1-(2-hydroxypyridin-3-yl)-methyliden]-1,3,4-trimethyl-piperazin-2,5-dion

Eine Suspension von 2.0 g 3-Benzyl-6-(2-iodbenzyliden)-1,3,4-trimethyl-piperazin-2,5-dion (s. Bsp. 2) in 10 ml Wasser und 20 ml 10%iger KOH-Lösung wurde mit 0,08 g CuI versetzt. Das Reaktionsgemisch wurde 4,5 Std. in der Mikrowelle mit 15W Leistung erhitzt. Es wurde mit Wasser/Acetonitril verdünnt, die ausgefallen Cu-Salze abfiltriert, das Eluat mit 10% HCl-Lösung angesäuert und auf etwa 20% des Volumens eingeengt. Die wässrige Phase wurde mit EE und $CH_2Cl_2$ extrahiert, getrocknet und mit Diethylether ($Et_2O$) ausgerührt. Als Rückstand wurden 8,78 mg der Titelverbindung leicht verunreinigtes Produkt erhalten, das direkt roh weiter umgesetzt wurde.
HPLC-MS [m/z]: 352,4 $[M+1]^+$

Beispiel 6: Herstellung von {3-[5-Benzyl-1,4,5-trimethyl-3,6-dioxopiperazin-2-ylidenmethyl]-pyridin-2-yloxy}-acetonitril

300 mg der Hydroxyverbindung aus Beispiel 5 in 10 ml Acetonitril wurden bei 20-25°C mit 471 mg $Ag_2CO_3$ versetzt, dann 154 mg Bromacetonitril zugetropft. Die Reaktionsmischung wurde 60 Std. refluxiert, dann wurden weitere 102 mg Bromacetonitril zugegeben und 18 Std. unter Rückfluß erhitzt. Nach Abkühlen wurde dem Reaktionsgemisch Wasser zugegeben, die Salze abfiltriert, das Eluat auf 1/3 eingeengt, dann mit $CH_2Cl_2$ verdünnt und nach Phasentrennung die organische Phase mit 5%iger NaOH-Lösung gewaschen, getrocknet und vom Lösungsmittel befreit. Man erhielt

nach Reinigung mittels RP-LC 29 mg der Titelverbindung. HPLC-MS [m/z]: 391.4 $[M+1]^+$

Beispiel 7: Herstellung von 3-Benzyl-6-[1-(2-diethylaminopyridin-3-yl)methyliden]-1,3,4-trimethylpiperazin-2,5-dion

Zu einer Lösung von 100 mg 3-Benzyl-6-[1-(2-aminopyridin-3-yl)-methyliden]-1,3,4-trimethyl-piperazin-2,5-dion (hergestellt analog Bsp. 1 aus 2-Aminonicontinaldehyd) in 5 ml DMF wurden bei 0°C 35 mg 60%iges NaH gegeben. Das Reaktionsgemisch wurde 2 Std. bei 0°C gerührt und anschließend mit 200 mg Ethyliodid versetzt, dann 18 Std. bei 20-25°C gerührt, anschließend mit einer gesätt. Zitronensäurelösung versetzt und mehrmals mit EE extrahiert. Die vereingten organischen Phasen wurden nach Waschen mit Wasser getrocknet, filtriert und vom Lösungsmittel befreit. Nach Chromatographie an Kieselgel (Eluent EE) wurden 50 mg der Titelverbindung erhalten.
HPLC-MS [m/z]: 406.8 $[M]^+$

Beispiel 8: Herstellung von {3-[5-Benzyl-1,4,5-trimethyl-3,6-dioxopiperazin-2-ylidenmethyl]-pyridin-2-yl}oxy}-cyanoessigsäureethylester

Eine Lösung von 500 mg 3-Benzyl-6-[1-(2-iodopyridin-3-yl)-methyliden]-1,3,4-trimettiylpiperazin-2,5-dion (Herstellung s. Bsp 3b) in 25 ml Dioxan gelöst und mit 20,6 mg CuI, 26,7 mg Picolinsäure, 1059 mg $Cs_2CO_3$ und 250 mg Cyanessigsäureethylester versetzt. Nach 1 Std. Rückfluß wurden weitere 206 mg CuI zugegeben und das Gemisch 18 Std. refluxiert. Nach Abkühlen wurde dem Reaktionsgemisch $NH_aCl$-Lösung zugesetzt, mit EE extrahiert und die organische Phase getrocknet, welche nach Entfernen des Lösungsmittels mit EE/Cyclohexan chromatographiert wurde. Man erhielt 183 mg der Titelverbindung. HPLC-MS [m/z]: 447.5 $[M]^+$

Beispiel 9: Herstellung von 3-Benzyl-1,3,4-trimethyl-6-[1-(2-morpholin-4-yl-pyridin-3-yl)methyliden]-piperazin-2,5-dion

Eine Lösung von 230,5 mg 3-Benzyl-6-[1-(2-iodo-pyridin-3-yl)-meth-(*Z*)-yliden]-1,3,4-trimethyl-piperazin-2,5-dion (Herstellung s. Bsp 3b) in 1 ml Morpholin und 1 ml DMF wurde mit 244 mg $Cs_2CO_3$ versetzt und 40 min in der Mikrowelle mit 15W Leistung bei 140 °C gerührt (Druck ca. 2 bar) anschließend in eine 5 %ige Zitronensäurelösung eingerührt und mit $CH_2Cl_2$ extrahiert. Nach Entfernen des Lösungsmittels kristallisierte der Rückstand über Nacht bei 20-25°C. Die Kristalle wurden mit kaltem Ethylacetat gewaschen und getrocknet. Man erhielt 154 mg der Titelverbindung. HPLC-MS [m/z]: 421.1 $[M+1]^+$

Beispiel 10: Herstellung von 3-Benzyl-1,3,4-trimethyl-6-[1-[2-(5-methyl-thiophen-2-yl)-pyridin-3-yl]-meth-(Z)-yliden]-piperazin-2,5-dion

134,5 mg 4,4,5,5-Tetramethyl-2-(5-methyl-thiophen-2-yl)-[1,3,2]dioxaborolan, 140,1 mg $K_3PO_4$, 230 mg 3-Benzyl-6-[1-(2-iodo-pyridin-3yl)-meth-(2)-yliden]-1,3,4-trimethyl-piperazin-2,5-dion (Herstellung s. Bsp 3b), 57,2 mg 2-Dicyclohexylphosphino-2,4,6 -triisopropyl-1,1-biphenyl und 28,8 mg (0,05 mmol) Bis-(dibenzylidenaceton)-palladium in 10 ml n-Butanol und 50 μl Wasser wurden 20 Std. bei 100°C gerührt. Nach Zugabe von ca. 20 ml Wasser wurde das Reaktionsgemisch 2x mit je 30 ml EE extrahiert, die vereinigten organischen Phasen getrocknet und das Lösungsmittel im Vakuum entfernt. Aus dem Rückstand wurden nach Chromatographie an Kieselgel (Cyclohexan/EE) 110 mg der Titelverbindung erhalten. HPLC-MS [m/z]: 431.9 $[M+1]^+$

Beispiel 11: Herstellung von 3-[5-(3-Hydroxybenzyl)-1,4,5-trimethyl-3,6-dioxo-piperazin-2-ylidenmethyl]-pyridin-2-carbonitril

11a) 3-[1-(2-Chloropyridin-3-yl)-methyliden]1,4,6-trimethylpiperazin-2,5-dion 1,4-Diacetyl-3-methyl-piperazin-2,5-dion (Herstellung analog 1,4-Diacetyl-3-benzyl-piperazin-2,5-dion vgl. WO 2008/152073) wurde analog Bsp. 1 mit 2-Chlornicotinaldehyd zu 3-[1-(2-Chloropyridin-3-yl)-methyliden]1,4,6-trimethylpiperazin-2,5-dion kondensiert.
11b) 3-(3-Benzyloxybenzyl)-6-[1-(2-chlorpyridin-3-ylrmethyliden]-1,3,4-trimethylpiperazin-2,5-dion
Zu einer Lösung von 1 g der Chloropyridin-Verbindung aus Bsp. 11a in 40 ml Tetrahydrofuran (THF) wurden 4,3 ml einer 1 M Lithium Hexamethyldisilazan (LiHMDS) Lösung in THF bei -78°C langsam zugetropft. Das Reaktionsgemisch wurde 10 min bei -75°C gerührt, dann bei -75°C mit 650 mg 1-Benzyloxy-3-chlormethylbenzol in 2 ml TH F versetzt und 1 Std. bei dieser Temperatur, dann 18 Std. bei 20-25°C gerührt. Nach Zusatz von Zitronensäurelösung wurde es mehrmals mit EE extrahiert. Die vereinigten organischen Phasen wurden nach Waschen mit Wasser getrocknet, filtriert und vom Lösungsmittel befreit. Nach Chromatographie an Kieselgel wurden 890 mg 3-(3-Benzyloxybenzyl)-6-[1-(2-chlorpyridin-3-yl)-methyliden]-1,3,4-trimethylpiperazin-2,5-dion erhalten.

HPLC-MS [m/z]: 478.2 [M+H]+
11 c) 3-[5-(3-Benzyloxybenzyl)-1,4,5-trimethyl-3,6-dioxopiperazin-(2Z)-yliden-methyl]-pyridine-2-carbonitril
521,6 mg (1,31 mmol) 2-(Di-tert-butylphosphino)-1,1'-binaphtyl, 217,6 mg $Pd(TFA)_2$, 244,5 mg Zn-Staub, 889 mg des Piperazins aus Bsp. 11b) und 724,4 mg $Zn(CN)_2$ in 40 ml Dimethylacetamid wurden 6 Std. bei 125°C und 14 Std. bei 20-25°C gerührt, dann wurden ca. 40 ml 5%ige $NH_3$-Lösung zugegeben. Das Reaktionsgemisch wurde mit EE extrahiert, die vereinigten organischen Phasen mit Wasser gewaschen, getrocknet und vom Lösungsmittel befreit. Aus dem Rückstand wurde nach Chromatographie an Kieselgel (Cyclohexan/EE) 455 mg 3-[5-(3-Benzyloxybenzyl)-1,4,5-trimethyl-3,6-dioxopiperazin-(2Z)-ylidenmethyl]-pyridin-2-carbonitril erhalten.
HPLC-MS [m/z]: 467.4 $[M+1]^+$
11 d) 3-[5-(3-Hydroxybenzyl)-1,4,5-trimethyl-3,6-dioxopiperazin-2-ylidenmethyl]-pyridin-2-carbonitril
Eine Lösung von 380 mg 3-[5-(3-Benzyloxybenzyl)-1,4,5-trimethyl-3,6-dioxopiperazin-(2Z)-ylidenmethyl]-pyridin-2-carbonitril, und 2 ml einer 2M $BCl_3xS(CH_3)_2$ Lösung in $CH_2Cl_2$ in 80 ml $CH_2Cl_2$ wurde 2 Std. refluxiert und 18 Std. bei 20-25°C gerührt, nach Einrühren von Wasser und $CH_2Cl_2$ wurde sie mit $CH_2Cl_2$ extrahiert. Nach Waschen mit 5%iger $NaHCO_3$ Lsg. wurden die vereinigten organischen Phasen getrocknet, dann das Lösungsmittel entfernt. Nach Chromatographie an Kieselgel (Cyclohexan/EE) wurden aus dem Rückstand 130 mg 3-[5-(3-Hydroxybenzyl)-1,4,5-trimethyl-3,6-dioxopiperazin-2-ylidenmethyl]-pyridin-2-carbonitril erhalten.
HPLC-MS [m/z]: 376.8 $[M+1]^+$

Beispiel 12: Enantiomerentrennung von 3-[(R)-5-Benzy)-1,4,5-tnmethy)-3,6-dioxopiperazin-(2Z)-ylidenmethyl]-pyridin-2-carbonitril und 3-[(S)-5-Benzyl-1,4,5-trimethyl-3,6-dioxo-piperazin-(2Z)-ylidenmethyl]-pyridin-2-carbonitril [I-306 / I-307]

Die Trennung der 3-R- und 3-S-Enantiomere erfolgte durch Chromatographie an der stationären Phase: 250 x 4,6 mm CHIRALPAK® IA 5 μm (Fa. Chiral Technologies Europe); Mobile Phase: 70 : 30 : 0,1 % (v/v) n-Heptan : Ethanol : Diethylamin; Flußrate: 1,0 ml/min; Temperatur: 25°C; Detektion erfolgte bei 260 nm. Das R-Enantionmer eluierte bei einer Retentionszeit von 9,46 min; das S-Enantiomer nach 11.99 min. Die chemische Reinheit (nach Flächen-% bei 260 nm) beider Enantiomere betrug jeweils > 99,5%. Der Enantiomerenüberschuß (ee) betrug > 99,5 (R), bzw. > 98,0 (S).

Tabelle I: Verbindungen der Formel I, welche der Formel I.A' entsprechen:

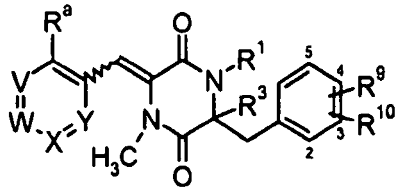

I.A'

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-1 | Br | N | CH | CH | CH | $CH_3$ | $CH_3$ | H | Z | 118 |
| I-2 | CN | N | CH | CH | CH | $CH_3$ | $CH_3$ | H | Z | 163 |
| I-3 ** | CN | CH | CH | CH | N | $CH_3$ | $CH_3$ | H | Z | 2,253 / 361,1 $[M+H]^+$ |
| I-4 | $C(O)NH_2$ | N | CH | CH | CH | $CH_3$ | $CH_3$ | H | Z | 2,329 / 379 $[M+H]^+$ |
| I-5 ** | Br | CH | N | CH | CH | $CH_3$ | $CH_3$ | H | Z | 2,628 / 416 [M+H]+ |
| I-6 ** | Br | CH | $N^+$-$CH_3$ | CH | CH | $CH_3$ | $CH_3$ | H | Z | 2,253 / 385,9 $[M+H]^+$ |
| I-7 | I | N | CH | CH | CH | $CH_3$ | $CH_3$ | H | Z | 134 |
| I-8 | Cl | N | CH | CH | CH | $CH_3$ | $CH_3$ | H | Z | 146 |
| I-9 | $OCH_3$ | N | CH | CH | CH | $CH_3$ | $CH_3$ | H | Z | 135 |
| I-10 | $OCH_2CF_3$ | N | CH | CH | CH | $CH_3$ | $CH_3$ | H | Z | 98 |
| I-11 ** | Br | N | CH | C-Br | CH | $CH_3$ | $CH_3$ | H | Z | 208 |
| I-12 ** | Cl | N | CH | C-F | CH | $CH_3$ | $CH_3$ | H | Z | 118-120 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-13 ** | $OCH_3$ | N | CH | C-F | CH | $CH_3$ | $CH_3$ | H | Z | 135-136 |
| I-14 | Br | N | CH | CH | CH | $CH_3$ | $CH_3$ | H | E | 131 |
| I-15 | CN | N | CH | CH | CH | $CH_3$ | $CH_3$ | H | E | 158 |
| I-16 ** | CN | N | CH | C-F | CH | $CH_3$ | $CH_3$ | H | Z | 130-132 |
| I-17 | 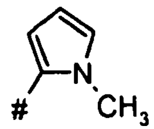 | N | CH | CH | CH | $CH_3$ | $CH_3$ | H | Z | 2,397 / 416,0 $[M+H]^+$ |
| I-18 | 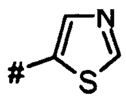 | N | CH | CH | CH | $CH_3$ | $CH_3$ | H | E | 2,788 / 420,1 $[M+H]^+$ |
| I-19 | 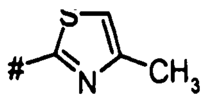 | N | CH | CH | CH | $CH_3$ | $CH_3$ | H | E | 3.055 / 433,1 $[M+H]^+$ |
| I-20 | 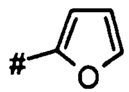 | N | CH | CH | CH | $CH_3$ | $CH_3$ | H | Z | 2,286 / 403,1 $[M+H]^+$ |
| I-21 | 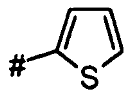 | N | CH | CH | CH | $CH_3$ | $CH_3$ | H | Z | 2,927 / 419,1 $[M+H]^+$ |
| I-22 | $NO_2$ | N | CH | CH | CH | $CH_3$ | $CH_3$ | H | Z | 160-162 |
| I-23 | $CF_3$ | N | CH | CH | CH | $CH_3$ | $CH_3$ | H | E | 205-207 |
| I-24 | 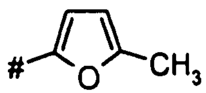 | N | CH | CH | CH | $CH_3$ | $CH_3$ | H | Z | 2,436 / 416,0 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-25 | 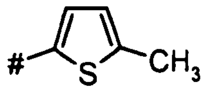 | N | CH | CH | CH | $CH_3$ | $CH_3$ | H | Z | 2,981 / 431,9 $[M+H]^+$ |
| I-26 | 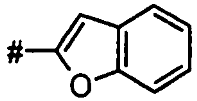 | N | CH | CH | CH | $CH_3$ | $CH_3$ | H | Z | 3,009 / 452,0 $[M+H]^+$ |
| I-27 | 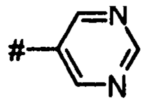 | N | CH | CH | CH | $CH_3$ | $CH_3$ | H | Z | 2,472 / 414,1 $[M+H]^+$ |
| I-28 | #-C≡C-Si$(CH_3)_3$ | N | CH | CH | CH | $CH_3$ | $CH_3$ | H | Z | 174 |
| I-29 | #-C≡CH | N | CH | CH | CH | $CH_3$ | $CH_3$ | H | Z | 161-163 |
| I-30 | 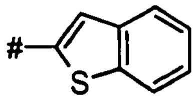 | N | CH | CH | CH | $CH_3$ | $CH_3$ | H | Z | 3,504 / 468,1 $[M+H]^+$ |
| I-31 | 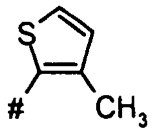 | N | CH | CH | CH | $CH_3$ | $CH_3$ | H | Z | 2,893 / 431,8 $[M]^+$ |
| I-32 | 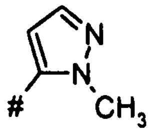 | N | CH | CH | CH | $CH_3$ | $CH_3$ | H | Z | 2,587 / 416,2 $[M+H]^+$ |
| I-33 | 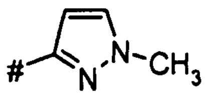 | N | CH | CH | CH | $CH_3$ | $CH_3$ | H | Z | 2,583 / 416,1 $[M+H]^+$ |
| I-34 | 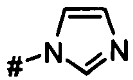 | N | CH | CH | CH | $CH_3$ | $CH_3$ | H | Z | 2,112 / 402,1 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-35 | 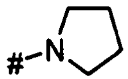 | N | CH | CH | CH | $CH_3$ | $CH_3$ | H | Z | 2,159 / 404,8 $[M]^+$ |
| I-36 | $OCH_2CH_3$ | N | CH | CH | CH | $CH_3$ | $CH_3$ | H | Z | 3,119 / 379,8 $[M]^+$ |
| I-37 | 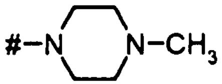 | N | CH | CH | CH | $+CH_3$ | $CH_3$ | H | Z | 2,151 / 434,2 $[M+H]^+$ |
| I-38 | 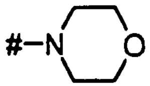 | N | CH | CH | CH | $CH_3$ | $CH_3$ | H | Z | 2,155/421,1 $[M+H]^+$ |
| I-39 | #-$NHCH_2CH_2NH_2$ | N | CH | CH | CH | $CH_3$ | $CH_3$ | H | Z | 1,837 / 393,8 $[M]^+$ |
| I-40 | 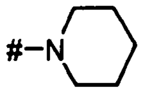 | N | CH | CH | CH | $CH_3$ | $CH_3$ | H | Z | 2,364 / 419,25 $[M+H]^+$ |
| I-41 | 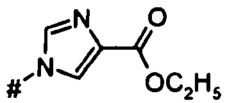 | N | CH | CH | CH | $CH_3$ | $CH_3$ | H | Z | 2,771 / 474,1 $[M+H]^+$ |
| I-42 | 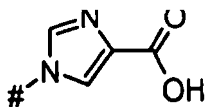 | N | CH | CH | CH | $CH_3$ | $CH_3$ | H | Z | 2,319 / 446,1 $[M+H]^+$ |
| I-43 | 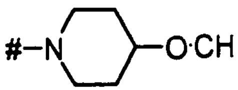 | N | CH | CH | CH | $CH_3$ | $CH_3$ | H | Z | 2,249 / 449,25 $[M+H]^+$ |
| I-44 | OH | N | CH | CH | CH | $CH_3$ | $CH_3$ | H | Z | 2,061 / 354,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-45 | $CH(CN)_2$ | N | CH | CH | CH | $CH_3$ | $CH_3$ | H | Z | 2,347 / 400,5 $[M+H]^+$ |
| I-46 ** | $CH_3$ | N | $C-CF_3$ | CH | CH | $CH_3$ | $CH_3$ | H | Z | 184-186 |
| I-47 ** | Cl | N | $C-CF_3$ | CH | CH | $CH_3$ | $CH_3$ | H | Z | 173-180 |
| 1-48 ** | #-CH=CH-CH=CH-C1) | | N | $C-CF_3$ | CH | $CH_3$ | $CH_3$ | H | Z | 197-203 |
| I-49 ** | #-CH=CH-CH=CH-C1) | | N | $C-CF_3$ | CH | $CH_3$ | $CH_3$ | H | E | 118-123 |
| I-50 | $SCH_3$ | N | CH | CH | CH | $CH_3$ | $CH_3$ | H | Z | 155-157 |
| I-51 ** | CN | N | CH | CH | N | $CH_3$ | $CH_3$ | H | Z | 176-177 |
| I-52 ** | CN | N | CH | $C-CH_3$ | CH | $CH_3$ | $CH_3$ | H | Z | 196-197 |
| I-53 ** | Cl | N | CH | CH | N | $CH_3$ | $CH_3$ | H | Z | 133-135 |
| I-54 ** | Cl | N | CH | $C-CH_3$ | CH | $CH_3$ | $CH_3$ | H | Z | 133-135 |
| I-55 | CN | N | CH | CH | CH | $CH_2F$ | $CH_3$ | H | Z | 3,323 / 378,8 $[M]^+$ |
| I-56 | 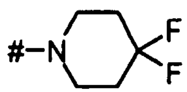 | N | CH | CH | CH | $CH_3$ | $CH_3$ | H | Z | 3,579 / 455,1 $[M+H]^+$ |
| I-57 ** | Br | CH | CH | CH | N | $CH_3$ | $CH_3$ | H | Z | b) 5,783 / 415,97 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-58 | $OCH_2CH=CH_2$ | N | CH | CH | CH | $CH_3$ | $CH_3$ | H | Z | 2,259/361,8 $[M+H]^+$ |
| I-59 | $OCH_2CN$ | N | CH | CH | CH | $CH_3$ | $CH_3$ | H | Z | 3,070 / 391,2 $[M+H]^+$ |
| I-60 | $CH(CN)COOCH_3$ | N | CH | CH | CH | $CH_3$ | $CH_3$ | H | Z | 2,628 / 432,8 $[M+H]^+$ |
| I-61 | $CH(COOCH_3)_2$ | N | CH | CH | CH | $CH_3$ | $CH_3$ | H | Z | 2,648 / 465,7 $[M+H]^+$ |
| I-62 | $CH(COCH_3)-(COOCH_3)$ | N | CH | CH | CH | $CH_3$ | $CH_3$ | H | Z | 2,135 / 449,7 $[M+H]^+$ |
| I-63 | $CH(COCH_3)-(COOCH_3)$ | N | CH | CH | CH | $CH_3$ | $CH_3$ | H | Z | 2,251 / 449,8 $[M+H]^+$ |
| I-64 | $CH(CN)COOCH_3$ | N | CH | CH | CH | $CH_3$ | $CH_3$ | H | Z | 2,722 / 446,8 $[M+H]^+$ |
| I-65 | $CH(CN)SO_2CH_3$ | N | CH | CH | CH | $CH_3$ | $CH_3$ | H | Z | 2,602 / 452,7 $[M+H]^+$ |
| I-66 | $CH(CN)CO-OCH(C_2H_5)_2$ | N | CH | CH | CH | $CH_3$ | $CH_3$ | H |  | 3,233 / 472,8 $[M+H]^+$ |
| I-67 | $CH(CN)CO-OCH(C_2H_5)_2$ | N | CH | CH | CH | $CH_3$ | $CH_3$ | H | Z | 3,302 / 472,8 $[M+H]^+$ |
| I-68 | $CH(CN)P(OC_2H_5$ )2 | N | CH | CH | CH | $CH_3$ | $CH_3$ | H | Z | 2,759 / 510,7 $[M+H]^+$ |
| I-69 | $CH(CN)CO-O(CH_2)_2OC_2H_5$ | N | CH | CH | CH | $CH_3$ | $CH_3$ | H | Z | 3,010 / 491,2 $[M+H]^+$ |
| I-70 | CH(CN)CON(CH 3)2 | N | CH | CH | CH | $CH_3$ | $CH_3$ | H | Z | 2,445 / 445,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-71 | $CH(CN)COC_2H_5$ | N | CH | CH | CH | $CH_3$ | $CH_3$ | H | Z | 2,951 /431,2 $[M+H]^+$ |
| I-72 | $CH(CN)COC_6H_5$ | N | CH | CH | CH | $CH_3$ | $CH_3$ | H | Z | 3,182 / 479,2 $[M+H]^+$ |
| I-73 | 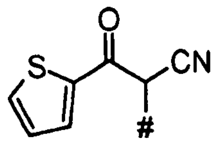 | N | CH | CH | CH | $CH_3$ | $CH_3$ | H | Z | 3,259 / 485,2 $[M+H]^+$ |
| I-74 ** | Cl | N | CH | C-F | CH | $CH_3$ | $CH_3$ | 3-F | Z | 128-130 |
| I-75 | $CONH_2$ | N | CH | CH | CH | $CH_3$ | $CH_3$ | 3-F | Z | 138-140 |
| I-76 | Cl | N | CH | CH | CH | $CH_3$ | $CH_3$ | 3-F | Z | 113-115 |
| I-77 ** | CN | N | CH | C-F | CH | $CH_3$ | $CH_3$ | 3-F | E | 152-155 |
| I-78 ** | CN | N | CH | C-F | CH | $CH_3$ | $CH_3$ | 3-F | Z | 157-158 |
| I-79 | $N(CH_2C{\equiv}CH)_2$ | N | CH | CH | CH | $CH_3$ | $CH_3$ | H | Z | 191 |
| I-80 | $N(CH_2CH_2CH_3)_2$ | N | CH | CH | CH | $CH_3$ | $CH_3$ | H | Z | 80 |
| I-81 | $N(CH_2CH_3)_2$ | N | CH | CH | CH | $CH_3$ | $CH_3$ | H | Z | 2,300 / 406,8 $[M]^+$ |
| 1-82 | $C(S)NH_2$ | N | CH | CH | CH | $CH_3$ | $CH_3$ | H | Z | 155 |
| I-83 | $NH_2$ | N | CH | CH | CH | $CH_3$ | $CH_3$ | H | Z | 2,010 / 350,8 $[M]^+$ |
| I-84 | $NHCH_3$ | N | CH | CH | CH | $CH_3$ | $CH_3$ | H | Z | 2,033 / 364,8 $[M]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-85 | $N(CH_3)_2$ | N | CH | CH | CH | $CH_3$ | $CH_3$ | H | Z | 2,035 / 378,8 $[M]^+$ |
| I-86 | $N(COCH_3)_2$ | N | CH | CH | CH | $CH_3$ | $CH_3$ | H | Z | 149 |
| I-87 | $N(CH_3)COCH_3$ | N | CH | CH | CH | $CH_3$ | $CH_3$ | H | Z | 166 |
| I-88 | $N(CH_2CH=CH_2)_2$ | N | CH | CH | CH | $CH_3$ | $CH_3$ | H | Z | 2,894 / 431,4 $[M+H]^+$ |
| I-89 | $NHCH_2CH=CH_2$ | N | CH | CH | CH | $CH_3$ | $CH_3$ | H | Z | 2,576 / 391,4 $[M+H]^+$ |
| I-90 ** | CN | N | CH | C-CN | CH | $CH_3$ | $CH_3$ | H | Z | 205 |
| I-91 ** | $SCH_3$ | N | CH | C-$SCH_3$ | CH | $CH_3$ | $CH_3$ | H | Z | 2,243 / 427,7 $[M+H]^+$ |
| I-92 | $CH_3$ | N | CH | CH | CH | $CH_3$ | $CH_3$ | H | Z | 125-126 |
| I-93 ** | Cl | N | C-Cl | CH | CH | $CH_3$ | $CH_3$ | H | Z | 186-188 |
| I-94 ** | CN | N | C-CN | CH | CH | $CH_3$ | $CH_3$ | H | Z | 3,252 / 386,1 $M+H]^+$ |
| 1-95 ** | $CH_3$ | N | C-Cl | CH | CH | $CH_3$ | $CH_3$ | H | Z | 3,538 / 384,1 $[M+H]^+$ |
| I-96 ** | Cl | N | C-$CH_3$ | CH | CH | $CH_3$ | $CH_3$ | H | Z | 3,170 / 384,1 $[M+H]^+$ |
| I-97 ** | $OCH_3$ | N | C-$OCH_3$ | CH | CH | $CH_3$ | $CH_3$ | H | Z | 161-163 |
| I-98 ** | $CH_3$ | N | C-CN | CH | CH | $CH_3$ | $CH_3$ | H | Z | 3,080 / 375,1 $[M+H]^+$ |
| I.99 ** | CN | N | C-CN | CH | CH | $CH_3$ | $CH_3$ | H | Z | 3,102 / 375,1 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-100 ** | $CH_3$ | N | C-$OCH_3$ | CH | CH | $CH_3$ | $CH_3$ | H | Z | 2,632 / 380,1 $[M+H]^+$ |
| I-101 ** | $CH_3$ | N | 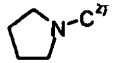 | CH | CH | $CH_3$ | $CH_3$ | H | Z | 2,458 / 419,1 $[M+H]^+$ |
| I-102 ** | $OCH_3$ | N | C-$CH_3$ | CH | CH | $CH_3$ | $CH_3$ | H | Z | 3,334 / 380,4 $[M+H]^+$ |
| I-103 ** | $SCH_3$ | N | C-$CH_3$ | CH | CH | $CH_3$ | $CH_3$ | H | Z | 3,395 / 396,4 $[M+H]^+$ |
| I-104 ** | $OCH_3$ | N | CH | C-Br | CH | $CH_3$ | $CH_3$ | H | Z | 133-135 |
| I-105 | 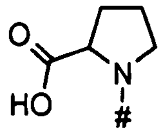 | N | CH | CH | CH | $CH_3$ | $CH_3$ | H | Z | 2,127 / 449,4 $[M+H]^+$ |
| I-106 | 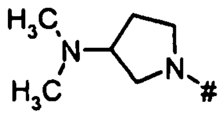 | N | CH | CH | CH | $CH_3$ | $CH_3$ | H | Z | 1,923 / 448,4 $[M+H]^+$ |
| I-107 | 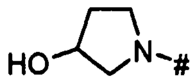 | N | CH | CH | CH | $CH_3$ | $CH_3$ | H | Z | 2,060/421,4 $[M+H]^+$ |
| I-108 | 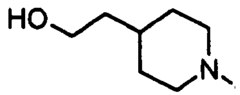 | N | CH | CH | CH | $CH_3$ | $CH_3$ | H | Z | 2,299 / 463,4 $[M+H]^+$ |
| I-109 | $SO_2CH_3$ | N | CH | CH | CH | $CH_3$ | $CH_3$ | H | Z | 2,546 / 414,2 $[M+H]^+$ |
| I-110 | $SO_2CH_3$ | N | CH | CH | CH | $CH_3$ | $CH_3$ | H | Z | 2,342 / 334,2 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-111 ** | Cl | N | CH | CH | C-$CH_3$ | $CH_3$ | $CH_3$ | H | Z | 3,051 / 383,8 $[M]^+$ |
| I-112 ** | Cl | N | CH | CH | C-Cl | $CH_3$ | $CH_3$ | H | Z | 3,374 / 404,1 $[M]^+$ |
| I-113 ** | Cl | N | CH | C-CH=$CH_2$ | CH | $CH_3$ | $CH_3$ | H | Z | 3,404 / 396,3 $[M+H]^+$ |
| I-114 ** | Cl | N | CH | CH | C-CH= $CH_2$ | $CH_3$ | $CH_3$ | H | Z | 3,340 / 396,3 $[M+H]^+$ |
| I-115 | Cl | N | CH | CH | CH | $CH_3$ | $CH_3$ | 3-$OCH_3$, 4-F | Z | 3,037 / 418,2 $[M]^+$ |
| I-116 | Cl | N | CH | CH | CH | $CH_3$ | $CH_3$ | 3-$OCH_3$, 4-F | E | 3,034 / 418,2 $[M]^+$ |
| I-117 | Cl | N | CH | CH | CH | $CH_3$ | $CH_3$ | 3,4-$F_2$ | Z | 3,117/406,2 $[M+H]^+$ |
| I-118 | CN | N | CH | CH | CH | $CH_3$ | $CH_3$ | 3-$OCH_3$, 4-F | Z | 2,978 / 409,2 [M+H]+ |
| I-119 | Cl | N | CH | CH | CH | $CH_3$ | $CH_3$ | 2-F | Z | 2,916 / 387,7 $[M]^+$ |
| 1-120 | CN | N | CH | CH | CH | $CH_3$ | $CH_3$ | 3,4-$F_2$ | Z | 2,922 / 396,8 $[M]^+$ |
| I-121 | Cl | N | CH | CH | CH | $CH_3$ | $CH_3$ | 3-$OCH_2$-$C_6H_5$ | Z | 3,501 / 476,8 $[M+H]^+$ |
| I-122 | CN | N | CH | CH | CH | $CH_3$ | $CH_3$ | 3-$OCH_2$-$C_6H_5$ | Z | 3,610 / 467,5 $[M+H]^+$ |
| I-123 | CN | N | CH | CH | CH | $CH_3$ | $CH_3$ | 3-OH | Z | 2,387 / 477,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-124 | Cl | N | CH | CH | CH | $CH_3$ | $CH_3$ | $4\text{-}OCH_2\text{-}C_6H_5$ | Z | 3,598 / 476,8 $[M+H]^+$ |
| I-125 | CN | N | CH | CH | CH | $CH_3$ | $CH_3$ | $4\text{-}OCH_2\text{-}C_6H_5$ | Z | 3,443 / 467,5 $[M+H]^+$ |
| I-126 | CN | N | CH | CH | CH | $CH_3$ | $CH_3$ | 4-OH | Z | 2,428 / 377,4 $[M+H]^+$ |
| I-127 | CN | N | CH | CH | CH | $(CH_2)_2CH_3$ | $CH_3$ | H | Z | 3,088 / 389,8 $[M+H]^+$ |
| I-128 | CN | N | CH | CH | CH | $CH_2CH_3$ | $CH_3$ | H | Z | 2,787 / 374,8 $[M]^+$ |
| I-129 | CN | N | CH | CH | CH | $CH_2\text{-}CH{=}CH_2$ | $CH_3$ | H | Z | 2,937 / 386,8 $[M]^+$ |
| I-130 | CN | N | CH | CH | CH | $CH_2C{\equiv}CH$ | $CH_3$ | H | Z | 3,044 / 385,3 $[M+H]^+$ |
| I-131 | CN | N | CH | CH | CH | $CH_2C{\equiv}C\text{-}CH_3$ | $CH_3$ | H | Z | 3,152 / 399,2 $[M+H]^+$ |
| I-132 | CN | N | CH | CH | CH | $COCH_3$ | $CH_3$ | H | Z | 3,246 / 389,2 $[M+H]^+$ |
| I-133 | CN | N | CH | CH | CH | H | $CH_3$ | H | Z | 2,438 / 347,2 $[M+H]^+$ |
| I-134 | CN | N | CH | CH | CH | $CH_2\text{-}CH{=}CHCH_3$ | $CH_3$ | H | Z | 3,215 / 400,8 [M+H]+ |
| I-135 | CN | N | CH | CH | CH | $CH_2\text{-}C(CH_3){=}CH_2$ | $CH_3$ | H | Z | 3,108 / 400,8 $[M+H]^+$ |
| I-136 | $NH\text{-}C(O)CH_2CH_2CH_3$ | N | CH | CH | CH | $CH_3$ | $CH_3$ | H | Z | 2,423 / 421,4 $[M+1]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-137 | NH-C(O)$CH_2CH_3$ | N | CH | CH | CH | $CH_3$ | $CH_3$ | H | Z | 2,235 / 406,8 $[M]^+$ |
| I-138 ** | $OCH_3$ | N | CH | CH | C-$OCH_3$ | $CH_3$ | $CH_3$ | H | Z | 126 |
| I-139 ** | CN | N | CH | CH | C-CH= $CH_2$ | $CH_3$ | $CH_3$ | H | Z | 3,121/386,8 $[M]^+$ |
| I-140 ** | CN | N | CH | C-CH=$CH_2$ | CH | $CH_3$ | $CH_3$ | H | Z | 3.219 / 387,2 $[M+H]^+$ |
| I-141 | Cl- +N1)$CH_2$C(O)NH-# | | CH | CH | CH | $CH_3$ | $CH_3$ | H | Z | 1,934 / 426,7 $[M]^+$ |
| I-142 | NH-C(O)$CF_3$ | N | CH | CH | CH | $CH_3$ | $CH_3$ | H | Z | 2,794 / 446,7 $[M]^+$ |
| I-143 ** | CN | N | CH | CH | C-CN | $CH_3$ | $CH_3$ | H | Z | 3,098 / 385,8 $[M]^+$ |
| I-144 | 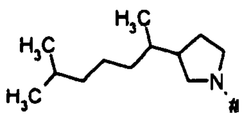 | N | CH | CH | CH | $CH_3$ | $CH_3$ | H | Z | 3,691 / 517,9 $[M+H]^+$ |
| I-145 | 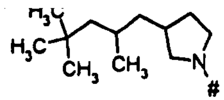 | N | CH | CH | CH | $CH_3$ | $CH_3$ | H | Z | 3,605 / 517,9 [M+H]+ |
| I-146 | 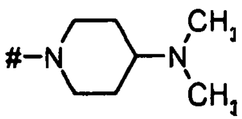 | N | CH | CH | CH | $CH_3$ | $CH_3$ | H | Z | 1,974 / 462,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-147 | 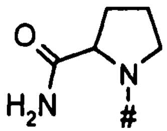 | N | CH | CH | CH | $CH_3$ | $CH_3$ | H | Z | 2,152 / 448,8 $[M+H]+$ |
| I-148 | 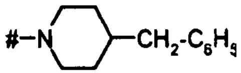 | N | CH | CH | CH | $CH_3$ | $CH_3$ | H | Z | 3,141 / 509,8 $[M+H]^+$ |
| I-149 | 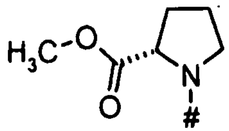 | N | CH | CH | CH | $CH_3$ | $CH_3$ | H | Z | 2,135 / 463,8 $[M+H]+$ |
| I-150 | 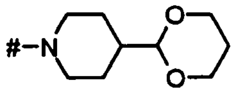 | N | CH | CH | CH | $CH_3$ | $CH_3$ | H | Z | 2,342 / 491,8 $[M+H]^+$ |
| I-151 | 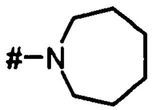 | N | CH | CH | CH | $CH_3$ | $CH_3$ | H | Z | 2,509 / 433,8 $[M+H]^+$ |
| I-152 | 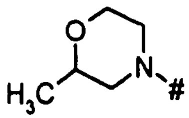 | N | CH | CH | CH | $CH_3$ | $CH_3$ | H | Z | 2,316 / 435,8 $[M+H]^+$ |
| I-153 | 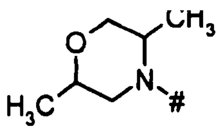 | N | CH | CH | CH | $CH_3$ | $CH_3$ | H | Z | 2,416 / 449,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-154 | 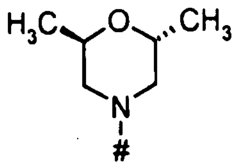 | N | CH | CH | CH | $CH_3$ | $CH_3$ | H | Z | 2,409 / 449,8 $[M+H]^+$ |
| I-155 | 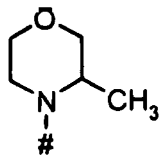 | N | CH | CH | CH | $CH_3$ | $CH_3$ | H | Z | 2,265 / 435,8 $[M+H]^+$ |
| I-156 | 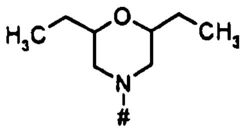 | N | CH | CH | CH | $CH_3$ | $CH_3$ | H | Z | 2,888 / 477,8 $[M+H]^+$ |
| I-157 | 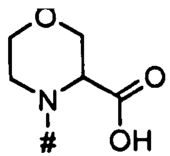 | N | CH | CH | CH | $CH_3$ | $CH_3$ | H | Z | 2,989 / 462,8 $[M-2]^+$ |
| I-158 | 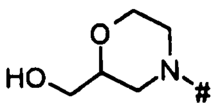 | N | CH | CH | CH | $CH_3$ | $CH_3$ | H | Z | 2,372 / 451,8 $[M+H]^+$ |

(fortgesetzt)

| Nr. | Rª | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-159 | 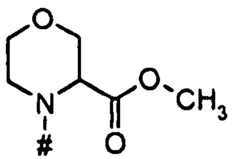 | N | CH | CH | CH | $CH_3$ | $CH_3$ | H | Z | 2,155 / 478,8 $[M+H]^+$ |
| 1-160 | 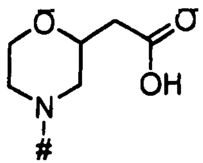 | N | CH | CH | CH | $CH_3$ | $CH_3$ | H | Z | 2,155 / 478,8 $[M+H]^+$ |
| I-161 | 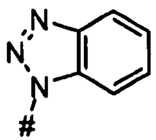 | N | CH | CH | CH | $CH_3$ | $CH_3$ | H | Z | 3,287 / 452,8 $[M]^+$ |
| I-162 | 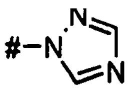 | N | CH | CH | CH | $CH_3$ | $CH_3$ | H | Z | 2,533 / 402,5 $[M]^+$ |
| I-163 | 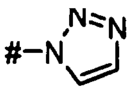 | N | CH | CH | CH | $CH_3$ | $CH_3$ | H | Z | 2,621 / 403,8 $[M+H]^+$ |
| I-164 | 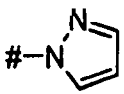 | N | CH | CH | CH | $CH_3$ | $CH_3$ | H | Z | 2,858 / 402,8 $[M+H]^+$ |
| I-165 | COOH | N | CH | CH | CH | $CH_3$ | $CH_3$ | H | Z | 2,011 / 379,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-166 | 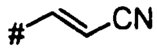 | N | CH | CH | CH | $CH_3$ | $CH_3$ | H | Z/E 1:1 | 2,814 / 386,9 $[M+H]^+$ 2,995 / 386,9 $[M+H]^+$ |
| I-167 | #-$CH_2$-CN | N | CH | CH | CH | $CH_3$ | $CH_3$ | H | Z | 2,352 / 374,8 $[M+H]^+$ |
| I-168 | 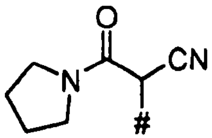 | N | CH | CH | CH | $CH_3$ | $CH_3$ | H | Z | 2,741 /471,8 $[M+H]^+$ |
| I-169 | 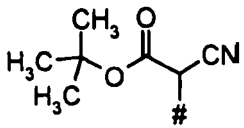 | N | CH | CH | CH | $CH_3$ | $CH_3$ | H | Z | 3,250 / 474,8 $[M+H]^+$ |
| I-170 ** | $CH_3$ | N | C-F | CH | CH | $CH_3$ | $CH_3$ | H | Z | 2,964 / 367,8 $[M+H]^+$ |
| I-171 ** | $N(C_2H_5)_2$ | N | C-$N(C_2H_5)_2$ | CH | CH | $CH_3$ | $CH_3$ | H | Z | 3,111 / 377,9 $[M+H]^+$ |
| I-172 ** | Cl | N | C-$N(C_2H_5)_2$ | CH | CH | $CH_3$ | $CH_3$ | H | Z | 3,938 / 440,9 $[M+H]^+$ |
| I-173 ** | O-$CH(CH_3)_2$ | N | C-O-$CH(CH_3)_2$ | CH | CH | $CH_3$ | $CH_3$ | H | Z | 4,225 / 451,8 $[M+H]^+$ |
| I-174 ** | Cl | N | $N(CH_3)_2$ | CH | CH | $CH_3$ | $CH_3$ | H | Z | 2,410 / 362,9 [M+H]+ |
| I-175 ** | $SCH_3$ | N | C-$SCH_3$ | CH | CH | $CH_3$ | $CH_3$ | H | Z | 3,866 / 427,7 $[M+H]^+$ |
| I-176 ** | $OCH_2CH=CH_2$ | N | C-$OCH_2$-CH=C $H_2$ | CH | CH | $CH_3$ | $CH_3$ | H | Z | 3,995 / 447,8 $[M+H]^+$ |

(fortgesetzt)

| Nr. | Rª | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-177 ** | Cl | N | $C^{2)}$-CH=CH-C($OCH_3$)=CH-$C^{3)}$ | | CH | $CH_3$ | $CH_3$ | H | Z | 3,564 / 450,3 $[M+H]^+$ |
| I-178 ** | Cl | N | $C^{2)}$-CH=CH-CH=CH-$C^{3)}$ | | CH | $CH_3$ | $CH_3$ | H | Z | 3,811 / 420,3 $[M+H]^+$ |
| I-179 ** | CN | N | C2)-CH=CH-C($OCH_3$)=CH-$C^{3)}$ | | CH | $CH_3$ | $CH_3$ | H | Z/E 3:1 | 3,526 / 441,4 $[M+H]^+$ 3,526/441,4 $[M+H]^+$ |
| I-180 ** | CN | N | $C^{2)}$-CH=CH-CH=CH-$C^{3)}$ | | CH | $CH_3$ | $CH_3$ | H | Z | 3,716/410,8 $[M+H]^+$ |
| I-181 ** | $OCH_3$ | N | $C^{2)}$-CH=CH-C($OCH_3$)=CH-$C^{3)}$ | | CH | $CH_3$ | $CH_3$ | H | Z | 3,660 / 446,4 $[M+H]^+$ |
| I-182 ** | $OCH_3$ | N | $C^{2)}$-CH=CH-CH=CH-$C^{3)}$ | | CH | $CH_3$ | $CH_3$ | H | Z | 3,588 / 415,8 $[M+H]^+$ |
| I-183 ** | $OCH_3$ | N | $C^{2)}$-CH=CH-C($CH_3$)=CH-$C^{3)}$ | | CH | $CH_3$ | $CH_3$ | H | Z | 3,230 / 429,8 $[M+H]^+$ |
| I-184 ** | 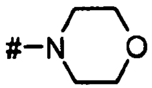 | N | $C^{2)}$-CH=CH-C($OCH_3$)=CH-$C^{3)}$ | | CH | $CH_3$ | $CH_3$ | H | Z | 2,693 / 501,4 $[M+H]^+$ |
| I-185 ** | 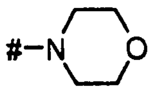 | N | $C^{2)}$-CH=CH-CH=CH-$C^{3)}$ | | CH | $CH_3$ | $CH_3$ | H | Z | 2,570 / 471,4 $[M+H]^+$ |
| I-186 | 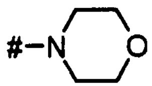 | N | CH | CH | CH | $CH_2$-CH=$CH_2$ | $CH_3$ | H | Z | 148 |
| I-187 | 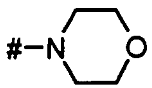 | N | CH | CH | CH | $CH_2CH_2CH_3$ | $CH_3$ | H | Z | 65 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-188 | 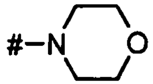 | N | CH | CH | CH | H | $CH_3$ | H | Z | 191 |
| I-189 | $OCH_2CH_3$ | N | CH | CH | CH | H | $CH_3$ | H | Z | 158 |
| I-190 | $OCH_2CH_3$ | N | CH | CH | CH | $CH_2$-$CH=CH_2$ | $CH_3$ | H | Z | 2,574 / 407,4 [M+H$^+$] |
| I-191 | 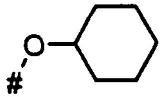 | N | CH | CH | CH | $CH_3$ | $CH_3$ | H | Z | 3,982 / 434,9 $[M+H]^+$ |
| I-192 | 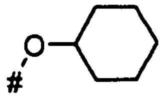 | N | CH | CH | CH | $CH_3$ | $CH_3$ | H | E | 3,751 / 433,9 $[M]^+$ |
| I-193 | 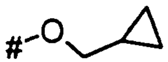 | N | CH | CH | CH | $CH_3$ | $CH_3$ | H | Z | 3,477 / 405,8 $[M]^+$ |
| I-194 | 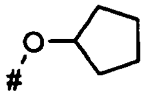 | N | CH | CH | CH | $CH_3$ | $CH_3$ | H | Z | 3,664 / 419,8 $[M]^+$ |
| I-195 ** | $CH_3$ | N | CH | CH | C-$CH_3$ | $CH_3$ | $CH_3$ | H | Z | 97-99 |
| I-196 | #-CH=C(CN)$_2$ | N | CH | CH | CH | $CH_3$ | $CH_3$ | H | Z | 3,434 / 411,2 $[M+H]^+$ |
| I-197 | Cl | N | CH | CH | CH | $CH_3$ | $CH_3$ | 4-F | Z | 152 |
| I-198 | CN | N | CH | CH | CH | $CH_3$ | $CH_3$ | 2-F | Z | 207 |
| I-199 | Cl | N | CH | CH | CH | $CH_3$ | $CH_3$ | 2,4-$F_2$ | Z | 148 |
| I-200 | Cl | N | CH | CH | CH | $CH_3$ | $CH_3$ | 2,4-$F_2$ | E | 68 |
| I-201 | Cl | N | CH | CH | CH | $CH_3$ | $CH_3$ | 2-0-$CHF_2$ | Z | 156 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-202 | Cl | N | CH | CH | CH | $CH_3$ | $CH_3$ | 3,4-$F_2$ | E | 164 |
| I-203 | CN | N | CH | CH | CH | $CH_3$ | $CH_3$ | 4-F | Z | 203 |
| I-204 | CN | N | CH | CH | CH | $CH_3$ | $CH_3$ | 2,4-$F_2$ | Z | 190 |
| I-205 | CN | N | CH | CH | CH | $CH_3$ | $CH_3$ | 2-O-$CHF_2$ | Z | 178 |
| I-206 | $OCH_2CH_3$ | N | CH | CH | CH | $CH_2CH_2CH_3$ | $CH_3$ | H | Z | 129 |
| I-207 | $OCHF_2$ | N | CH | CH | CH | $CH_3$ | $CH_3$ | H | Z | 3,141 / 401,8 $[M+H]^+$ |
| I-208 ** | $OCH_3$ | N | CH | C-CN | CH | $CH_3$ | $CH_3$ | H | Z | 3,105 / 391,2 $[M+H]^+$ |
| I-209 ** | CN | N | CH | CH | C-$CH_3$ | $CH_3$ | $CH_3$ | H | Z | 167 - 168 |
| I-210 | 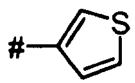 | N | CH | CH | CH | $CH_3$ | $CH_3$ | H | Z | 2,347 / 417,7 $[M+H]^+$ |
| I-211 | 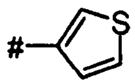 | N | CH | CH | CH | $CH_3$ | $CH_3$ | H | E | 2,377 / 417,7 $[M+H]^+$ |
| I-212 | Br | N | CH | CH | CH | $CH_2CH_2CH_3$ | $CH_3$ | H | Z | 152 |
| I-213 ** | $N(CH_3)_2$ | N | C2)-$N(CH_3)_2$ | CH | CH | $CH_3$ | $CH_3$ | H | Z | 2,601 / 421,8 $[M+H]^+$ |
| I-214 ** | $CH_3$ | N | 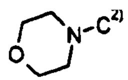 | CH | CH | $CH_3$ | $CH_3$ | H | Z | 140 |
| I-215 ** | $CH_3$ | N | 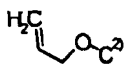 | CH | CH | $CH_3$ | $CH_3$ | H | Z | 3,220 / 405,9 $[M+H]^+$ |
| I-216 ** | $CH_3$ | N | 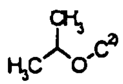 | CH | CH | $CH_3$ | $CH_3$ | H | Z | 2,936 / 408,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-217 ** | CN | N | $C^{2)}$- $N(CH_3)_2$ | CH | CH | $CH_3$ | $CH_3$ | H | Z | 150 |
| I-218 ** | CN | N | $C^{2)}$-$SCH_3$ | CH | CH | $CH_3$ | $CH_3$ | H | Z | 120 |
| I-219 ** | Cl | N | $C^{2)}$-$N(CH_3)$-$N=C(CH_3)$-$C^{3)}$ | | CH | $CH_3$ | $CH_3$ | H | Z | 170 |
| I-220 ** | OH | N | $C^{2)}$-$N(CH_3)_2$ | CH | CH | $CH_3$ | $CH_3$ | H | Z | 2,393 / 394,8 $[M+H]^+$ |
| I-221 ** | Cl | N | $C^{2)}$-$C(CH_3)=CH$-$CH=C(CH_3)$-$C^{3)}$ | | CH | $CH_3$ | $CH_3$ | H | Z | 165 - 168 |
| I-222 ** | Cl | N | $C^{2)}$-$C(CH_2CH_3)=CH$-$CH=CH$-$C^{3)}$ | | CH | $CH_3$ | $CH_3$ | H | Z | 4,170 / 448,2 $[M+H]^+$ |
| I-223 ** | CN | N | $C^{2)}$-$C(CH_3)=CH$-$CH=C(CH_3)$-$C^{3)}$ | | CH | $CH_3$ | $CH_3$ | H | Z | 3,876 / 438,8 [M+H]+ |
| I-224 ** | CN | N | $C^{2)}$-$C(CH_2CH_3)=CH$-$CH=CH$-$C^{3)}$ | | CH | $CH_3$ | $CH_3$ | H | Z | 3,978 / 448,2 $[M+H]^+$ |
| I-225 ** | $OCH_3$ | N | $C^{2)}$-$CH=C(OCH_3)$-$CH=CH$-$C^{3)}$ | | CH | $CH_3$ | $CH_3$ | H | Z | 190 - 192 |
| I-226 ** | $OCH_3$ | N | $C^{2)}$-$C(CH_3)=CH$-$CH=C(CH_3)$-$C^{3)}$ | | CH | $CH_3$ | $CH_3$ | H | Z | 132 -134 |
| I-227 ** | Cl | N | $C^{2)}$-CH=CH-S-$C^{3)}$ | | CH | $CH_3$ | $CH_3$ | H | Z | 193-194 |
| I-228 ** | CN | N | $C^{2)}$-CH=CH-S-$C^{3)}$ | | CH | $CH_3$ | $CH_3$ | H | Z | 3,219 / 417,3 $[M+H]^+$ |
| I-229 ** | $OCH_3$ | N | $C^{2)}$-CH=CH-CH=CH-$C^{3)}$ | | CH | $CH_3$ | $CH_3$ | H | Z | 3,549 / 415,8 $[M+H]^+$ |
| I-230 | Br | N | CH | CH | CH | H | $CH_3$ | H | E | 164 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-231 | Br | N | CH | CH | CH | H | $CH_3$ | H | Z | 238 |
| I-232 | OH | N | CH | CH | CH | H | $CH_3$ | H | Z | 255 |
| I-233 | CN | N | CH | CH | CH | $CH_2C{\equiv}CCH_3$ | $CH_3$ | H | Z | 3,152 / 399,2 $[M+H]^+$ |
| I-234 ** | Cl | N | CH | CH | C-Br | $CH_3$ | $CH_3$ | H | Z | 3,165 / 450,1 $[M+H]^+$ |
| I-235 | $C(CN)_2CH_2CH_2C$ N | N | CH | CH | CH | $CH_3$ | $CH_3$ | H | Z | 3,098 / 452,9 $[M+H]^+$ |
| I-236 | $CH_3$ | N | CH | CH | CH | $CH_3$ | $CH_3$ | H | Z | 125 - 126 |
| I-237 | Cl | N | CH | CH | CH | $CH_3$ | $CH_3$ | 3-O-$CH_2$-$C_6H_5$ | Z | 3,501 / 476,25 $[M+H]^+$ |
| I-238 ** | $OC_2H_5$ | N | C-$OC_2H_5$ | CH | CH | $CH_3$ | $CH_3$ | H | Z | 120 |
| I-239 | 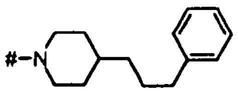 | N | CH | CH | CH | $CH_3$ | $CH_3$ | H | Z | 3,477 / 536,8 $[M]^+$ |
| I-240 | 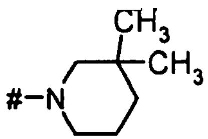 | N | CH | CH | CH | $CH_3$ | $CH_3$ | H | Z | 2,721 / 446,8 $[M+H]^+$ |
| I-241 | 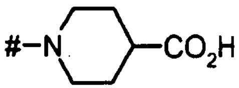 | N | CH | CH | CH | $CH_3$ | $CH_3$ | H | Z | 2,183 / 462,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-242 | 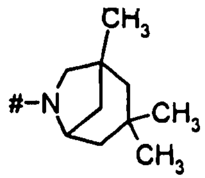 | N | CH | CH | CH | $CH_3$ | $CH_3$ | H | Z | 2,969 / 486,8 $[M+H]^+$ |
| I-243 | 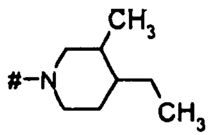 | N | CH | CH | CH | $CH_3$ | $CH_3$ | H | Z | 2,964 / 460,8 $[M+H]^+$ |
| I-244 | 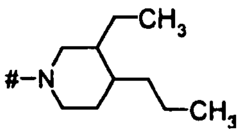 | N | CH | CH | CH | $CH_3$ | $CH_3$ | H | Z | 3,335 / 488,8 $[M+H]^+$ |
| I-245 | 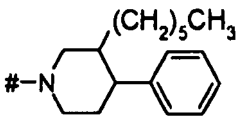 | N | CH | CH | CH | $CH_3$ | $CH_3$ | H | Z | 3,947 / 578,8 $[M+H]^+$ |
| I-246 | 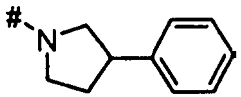 | N | CH | CH | CH | $CH_3$ | $CH_3$ | H | Z | 2,827 / 480,8 $[M+H]^+$ |
| I-247 | 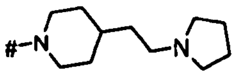 | N | CH | CH | CH | $CH_3$ | $CH_3$ | H | Z | 2,075 / 515,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-248 | 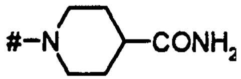 | N | CH | CH | CH | $CH_3$ | $CH_3$ | H | Z | 2,049 / 461,8 $[M+H]^+$ |
| I-249 | 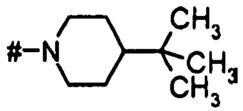 | N | CH | CH | CH | $CH_3$ | $CH_3$ | H | Z | 3,019 / 474,8 $[M+H]^+$ |
| I-250 | Cl | N | CH | CH | CH | $CH_3$ | $CH_3$ | 2,3-$F_2$ | Z | 173 |
| I-251 | Cl | N | CH | CH | CH | $CH_3$ | $CH_3$ | 3-F | E | 60 |
| I-252 | CN | N | CH | CH | CH | $CH_3$ | $CH_3$ | 3-F | Z | 189 |
| I-253 | CN | N | CH | CH | CH | $CH_3$ | $CH_3$ | 2,3-$F_2$ | Z | 209 |
| I-254 | 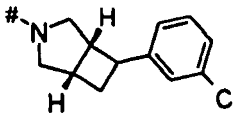 | N | CH | CH | CH | $CH_3$ | $CH_3$ | H | E | 3,295 / 541,3 $[M+H]^+$ |
| I-255 | 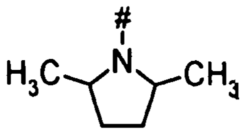 | N | CH | CH | CH | $CH_3$ | $CH_3$ | H | Z | 2,503/433,4 $[M+H]^+$ |

(fortgesetzt)

| Nr. | Rª | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-256 | 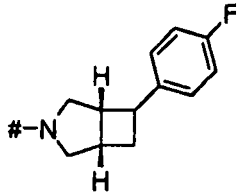 | N | CH | CH | CH | $CH_3$ | $CH_3$ | H | Z | 3,108/525,3 [M+H]+ |
| I-257 | 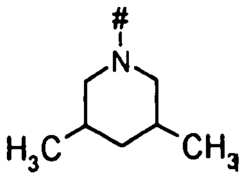 | N | CH | CH | CH | $CH_3$ | $CH_3$ | H | Z | 2,731/447,1 $[M+H]^+$ |
| I-258 | 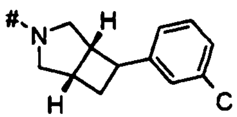 | N | CH | CH | CH | $CH_3$ | $CH_3$ | H | Z | 3,295 / 541,3 [M+H]+ |
| I-259 | 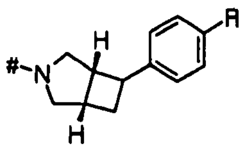 | N | CH | CH | CH | $CH_3$ | $CH_3$ | H | E | 3,108 / 525,3 $[M+H]^+$ |
| I-260 | 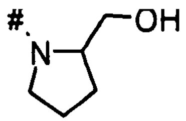 | N | CH | CH | CH | $CH_3$ | $CH_3$ | H | Z | 2,192 /435,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-261 | 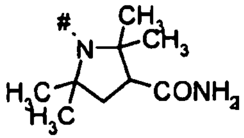 | N | CH | CH | CH | $CH_3$ | $CH_3$ | H | Z | 2,187 / 504,5 $[M+H]^+$ |
| I-262 | 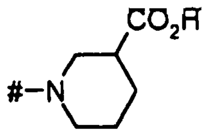 | N | CH | CH | CH | $CH_3$ | $CH_3$ | H | Z | 2,227 / 462,8 [M +H]$^+$ |
| I-263 | 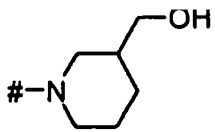 | N | CH | CH | CH | $CH_3$ | $CH_3$ | H | Z | 2,182 / 448,8 $[M+H]^+$ |
| I-264 | 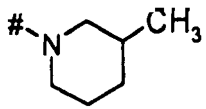 | N | CH | CH | CH | $CH_3$ | $CH_3$ | H | Z | 2,565 / 432,8 $[M+H]^+$ |
| I-265 | 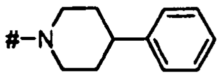 | N | CH | CH | CH | $CH_3$ | $CH_3$ | H | Z | 2,997 / 494,8 $[M+H]^+$ |
| I-266 | 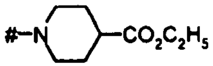 | N | CH | CH | CH | $CH_3$ | $CH_3$ | H | Z | 2,694 / 491,4 $[M+H]^+$ |
| I-267 | 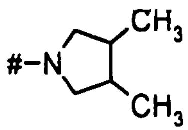 | N | CH | CH | CH | $CH_3$ | $CH_3$ | H | Z | 2,837 / 447,4 $[M+H]^+$ |
| I-268 | 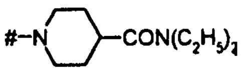 | N | CH | CH | CH | $CH_3$ | $CH_3$ | H | Z | 2,61 / 517,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-269 | 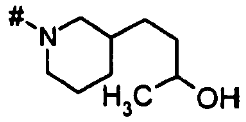 | N | CH | CH | CH | $CH_3$ | $CH_3$ | H | Z | 2,439 / 490,8 $[M+H]^+$ |
| I-270 | 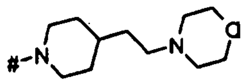 | N | CH | CH | CH | $CH_3$ | $CH_3$ | H | Z | 1,962 / 531,8 $[M+H]^+$ |
| I-271 ** | 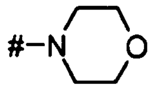 | N | 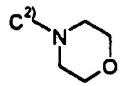 | CH | CH | $CH_3$ | $CH_3$ | H | Z | 200 |
| I-272 | Cl | N | CH | CH | CH | $CH_2CH=CH_2$ | $CH_2CH$ $=CH_2$ | H | Z | b) 6,020 / 422 $[M+H]^+$ |
| I-273 | CN | N | CH | CH | CH | $CH_2CH=CH_2$ | $CH_2CH$ $=CH_2$ | H | Z | b) 5,830 / 413 $[M+H]^+$ |
| I-274 | 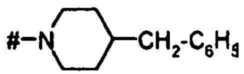 | N | CH | CH | CH | $CH_3$ | $CH_3$ | H | Z | 3,141 / 508,8 $[M+H]^+$ |
| I-275 | CN | N | CH | CH | CH | $(CH_2)_5CH_3$ | $CH_3$ | H | Z | 4,024 / 431,4 $[M+H]^+$ |
| I-276 | CN | N | CH | CH | CH | $(CH_2)_4CH_3$ | $CH_3$ | H | Z | 3,741 /417,5 $[M+H]^+$ |
| I-277 | $CH=CH_2$ | N | CH | CH | CH | $CH_3$ | $CH_3$ | H | Z | 125 |
| I-278 | $CO_2CH_2CH_3$ | N | CH | CH | CH | $CH_3$ | $CH_3$ | H | Z | 64 |
| I-279 | $CO_2CH_3$ | N | CH | CH | CH | $CH_3$ | $CH_3$ | H | Z | 151 |

(fortgesetzt)

| Nr. | Rª | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-280 | 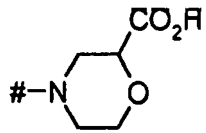 | N | CH | CH | CH | $CH_3$ | $CH_3$ | H | Z | 2,160 / 464,8 $[M+H]^+$ |
| I-281 | Cl | N | CH | CH | CH | $COCH_3$ | $CH_3$ | H | Z | 142 |
| I-282 | Cl | N | CH | CH | CH | H | $CH_3$ | H | Z | 189 |
| I-283 | Cl | N | CH | CH | CH | $CH_2CH_2CH_3$ | $CH_3$ | H | Z | 139 |
| I-284 | $OCH_2CH_3$ | N | CH | CH | CH | $CH_3$ | $CH_3$ | H | E | 152 |
| I-285 | $CF_3$ | N | CH | CH | CH | $CH_3$ | $CH_3$ | H | Z | 123 |
| I-286 | 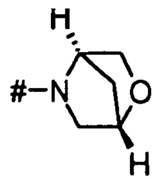 | N | CH | CH | CH | $CH_3$ | $CH_3$ | H | Z | 2,129 / 432,8 [M+H]+ |
| I-287 | 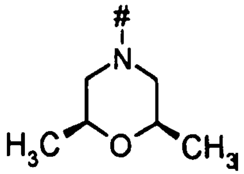 | N | CH | CH | CH | $CH_3$ | $CH_3$ | H | Z | 2,376 / 448,8 $[M+H]^+$ |
| I-288 | $N[CH_2CH=C(CH_3)]_2$ | N | CH | CH | CH | $CH_3$ | $CH_3$ | H | Z | 3,224 / 487,4 $[M+H]^+$ |
| I-289 | $N(CH_2Ph)_2$ | N | CH | CH | CH | $CH_3$ | $CH_3$ | H | Z | 3,474 / 531,4 $[M+H]^+$ |
| I-290 | $N[(CH_2)_3OH]_2$ | N | CH | CH | CH | $CH_3$ | $CH_3$ | H | Z | 2,215 / 467,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-291 | $N[(CH_2)_3OCH_2CH_3]_2$ | N | CH | CH | CH | $CH_3$ | $CH_3$ | H | Z | 3,165 / 495,5 $[M+H]^+$ |
| I-292 | 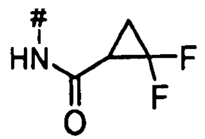 | N | CH | CH | CH | $CH_3$ | $CH_3$ | H | Z | 2,480 / 454,8 $[M+H]^+$ |
| I-293 | $NHCO(CH_2)_3Cl$ | N | CH | CH | CH | $CH_3$ | $CH_3$ | H | Z | 2,309 / 444,8 $[M+H]^+$ |
| I-294 | $NHCOCH_2CH-(OCH_3)CH_2CH_3$ | N | CH | CH | CH | $CH_3$ | $CH_3$ | H | Z | 2,517 / 464,8 $[M+H]^+$ |
| I-295 | $NHCOC(CH_3)=CHCH_2Cl$ | N | CH | CH | CH | $CH_3$ | $CH_3$ | H | Z | 2,699 / 466,8 $[M+H]^+$ |
| I-296 | 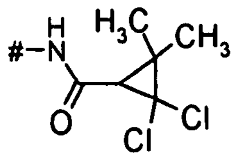 | N | CH | CH | CH | $CH_3$ | $CH_3$ | H | Z | 3,076 / 514,8 $[M+H]^+$ |
| I-297 | $NHCOCH-[CH(CH_3)_2]NH-CH(CH_3)_2$ | N | CH | CH | CH | $CH_3$ | $CH_3$ | H | Z | 2,342 /491,6 $[M+H]^+$ |
| I-298 | 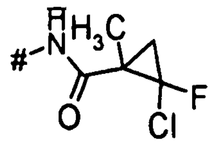 | N | CH | CH | CH | $CH_3$ | $CH_3$ | H | Z | 2,764 / 484,8 $[M+H]^+$ |
| I-299 | 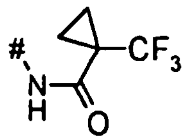 | N | CH | CH | CH | $CH_3$ | $CH_3$ | H | Z | 2,875 / 486,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-300 | NH-COCH=C($CF_3$)C $H_3$ | N | CH | CH | CH | $CH_3$ | $CH_3$ | H | Z | 2,943 / 486,8 [M+H]+ |
| I-301 | 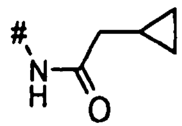 | N | CH | CH | CH | $CH_3$ | $CH_3$ | H | Z | 2,459 / 432,9 [M+H]+ |
| I-302 | $NHCOCH_2CH_2C\ F_3$ | N | CH | CH | CH | $CH_3$ | $CH_3$ | H | Z | 2,628 / 474,8 $[M+H]^+$ |
| I-303 | $NHCOCH_2C(CH_3\ )3$ | N | CH | CH | CH | $CH_3$ | $CH_3$ | H | Z | 2,752 / 448,9 $[M+H]^+$ |
| I-304 | 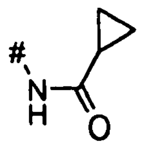 | N | CH | CH | CH | $CH_3$ | $CH_3$ | H | Z | 2,299 / 418,9 $[M+H]^+$ |
| I-305 | NH-$COCH_2CH_2CO_2\ CH_3$ | N | CH | CH | CH | $CH_3$ | $CH_3$ | H | Z | 2,309 / 464,8 $[M+H]^+$ |
| I-306 | CN | N | CH | CH | CH | $CH_3$ | $CH_3$ | H | Z | R enantiomer |
| I-307 | CN | N | CH | CH | CH | $CH_3$ | $CH_3$ | H | Z | S enantiomer |
| I-308 | $CF_3$ | N | CH | CH | CH | H | $CH_3$ | H | Z | 230 |
| I-309 | $CF_3$ | N | CH | CH | CH | $CH_2CH_2CH_3$ | $CH_3$ | H | Z | 170 |
| I-310 | $CF_3$ | N | CH | CH | CH | $CH_2CH=CH_2$ | $CH_3$ | H | Z | 130 |
| I-311 | $CF_3$ | N | CH | CH | CH | $CH_2C≡CH$ | $CH_3$ | H | Z | 173 |
| I-312 | $CF_3$ | N | CH | CH | CH | $CH_2CH_3$ | $CH_3$ | H | Z | 128 |
| I-313 | CN | N | CH | CH | CH | $CH_2CH_2CH_3$ | $CH_3$ | H | E | 168 |
| I-314 | $OCH(CH_3)_2$ | N | CH | CH | CH | $CH_3$ | $CH_3$ | H | Z | 121 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-315 ** | $N(CH_3)_2$ | N | $C^{3*}-CH_3$ | CH | CH | $CH_3$ | $CH_3$ | H | Z | 2,203 / 393,4 $[M+H]^+$ |
| I-316 ** | Cl | N | CH | $C-OCH_3$ | CH | $CH_3$ | $COCH_3$ | H | Z | 109 |
| I-317 | $3-NO_2-C_6H_5$ | N | CH | CH | CH | $CH_3$ | $CH_3$ | H | Z | 197- 198 |
| I-318 ** | Cl | N | CH | $C-OCH_3$ | CH | $CH_3$ | H | H | Z | 143-144 |
| I-319 ** | Cl | N | CH | CH | C-I | $CH_3$ | $CH_3$ | H | Z | 167 |
| I-320 | $OCH(CH_3)_2$ | N | CH | CH | CH | $CH_3$ | $CH_3$ | H | E | 3.129/393.8 $[M+H]^+$ |
| I-321 | $CON(CH_3)_2$ | N | CH | CH | CH | $CH_3$ | $CH_3$ | H | Z | 165 |
| I-322 | $CONHCH_3$ | N | CH | CH | CH | $CH_3$ | $CH_3$ | H | Z | 169 |
| I-323 | $CON(CH_3)(OCH_3)$ | N | CH | CH | CH | $CH_3$ | $CH_3$ | H | Z | 156 |
| I-324 | $COCH_3$ | N | CH | CH | CH | $CH_3$ | $CH_3$ | H | Z | 166 |
| I-325 ** | Cl | N | CH | $C^{3)}-C_2H_5$ | CH | $CH_3$ | $CH_3$ | H | Z | 130-131 |
| I-326 ** | Cl | N | CH | $C^{3)}-CH_2-OCH_3$ | CH | $CH_3$ | $CH_3$ | H | Z | 120-122 |
| I-327 ** | $OCH_2CH_3$ | N | CH | CH | $C-OC_2H_5$ | $CH_3$ | $CH_3$ | H | Z | 3,300 / 424,3 $[M+H]^+$ |
| I-328 ** | Cl | N | $C^{2)}-CH=C-(OCH_2O)-C=CH-C^{3)}$ | | CH | $CH_3$ | $CH_3$ | H | Z | 3,400 / 463,8 $[M+H]^+$ |
| I-329 ** | CN | N | $C^{2)}-CH=C-(OCH_2O)-C=CH-C^{3)}$ | | CH | $CH_3$ | $CH_3$ | H | Z | 3,213 / 454,7 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-330 ** | CN | N | 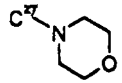 | CH | CH | $CH_3$ | $CH_3$ | H | Z | 3,023 / 445,8 [M+H]+ |
| I-331** | CN | N | CH | $C^{3)}-CH_2-OCH_3$ | CH | $CH_3$ | $CH_3$ | H | Z | 170,5 |
| I-332 ** | CN | N | CH | $C-C_2H_5$ | CH | $CH_3$ | $CH_3$ | H | Z | 154,5 |
| I-333 ** | $OCH_2CH_3$ | N | CH | $C-CH_2-OCH_3$ | CH | $CH_3$ | $CH_3$ | H | Z | 3,084 / 423,8 $[M+H]^+$ |
| I-334 | $NO_2$ | N | CH | CH | CH | H | $CH_3$ | H | Z | 173 |
| I-335 | $NO_2$ | N | CH | CH | CH | $CH_3$ | $COCH_3$ | H | Z | 173 |
| I-336 | $NO_2$ | N | CH | CH | CH | $CH_3$ | $CH_2CH=CH_2$ | H | Z | 166 |
| I-337 | 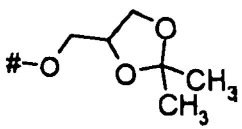 | N | CH | CH | CH | $CH_3$ | $CH_3$ | H | Z | 3,155 / 466,8 $[M+H]^+$ |
| I-338 | $N(CH_3)CH_2CH_3$ | N | CH | CH | CH | $CH_3$ | $CH_3$ | H | Z | 2,146 / 393,8 $[M+H]^+$ |
| I-339 ** | CN | N | $C^{2)}-N(CH_3)-C(CH_3)=N-C^{3)}$ | | CH | $CH_3$ | $CH_3$ | H | Z | 4,430 / 429,8 $[M+H]^+$ |
| I-340 | $OCH_2CH_2CH_2CH$ 3 | N | CH | CH | CH | $CH_3$ | $CH_3$ | H | Z | 3,597 / 407,8 $[M+H]^+$ |
| I-341 | 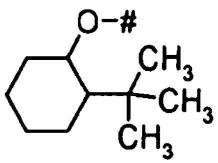 | N | CH | CH | CH | $CH_3$ | $CH_3$ | H | Z | 4,467 / 489,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-342 | $OCH_2CH_2N(CH_3)$ $-CH_2C_6H_5$ | N | CH | CH | CH | $CH_3$ | $CH_3$ | H | Z | 2,719 / 499,8 $[M+H]^+$ |
| I-343 | 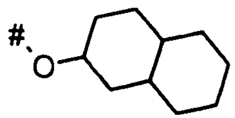 | N | CH | CH | CH | $CH_3$ | $CH_3$ | H | Z | 4,497 / 488,8 $[M+H]^+$ |
| I-344 | $O(CH_2)_2O-(CH_2)_3CH_3$ | N | CH | CH | CH | $CH_3$ | $CH_3$ | H | Z | 3,596 / 452,8 $[M+H]^+$ |
| I-345 | $O(CH_2)_2-(4-CH_3O)C_6H_5$ | N | CH | CH | CH | $CH_3$ | $CH_3$ | H | Z | 3,645 / 486,8 $[M+H]^+$ |
| I-346 | $O(CH_2)_2-C{\equiv}C(CH_2)_4CH_3$ | N | CH | CH | CH | $CH_3$ | $CH_3$ | H | Z | 4,245 / 474,8 $[M+H]^+$ |
| I-347 | $OCH_2C(CH_3)=C\ H_2$ | N | CH | CH | CH | $CH_3$ | $CH_3$ | H | Z | 3,445 / 406,8 $[M+H]^+$ |
| I-348 | $PO(OC_2H_5)_2$ | N | CH | CH | CH | $CH_3$ | $CH_3$ | H | Z | 2,771/472,4 $[M+H]^+$ |
| I-349 | 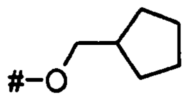 | N | CH | CH | CH | $CH_3$ | $CH_3$ | H | Z | 3,830 / 434,8 $[M+H]^+$ |
| I-350 | 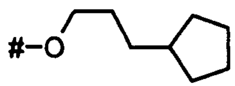 | N | CH | CH | CH | $CH_3$ | $CH_3$ | H | Z | 4,308 / 462,8 $[M+H]^+$ |
| I-351 | 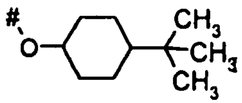 | N | CH | CH | CH | $CH_3$ | $CH_3$ | H | Z | 4,590 / 489,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-352 | 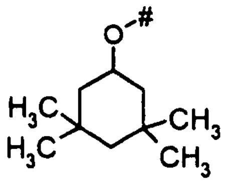 | N | CH | CH | CH | $CH_3$ | $CH_3$ | H | Z | 4,597 / 489,8 $[M+H]^+$ |
| I-353 | 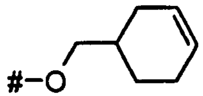 | N | CH | CH | CH | $CH_3$ | $CH_3$ | H | Z | 3,846 / 446,8 $[M+H]^+$ |
| I-354 | $O(CH_2)_2OCH_2C_6H_5$ | N | CH | CH | CH | $CH_3$ | $CH_3$ | H | Z | 3,583 / 486,8 $[M+H]^+$ |
| I-355 | $O(CH_2)_2CH(CH_3)$ $-CH_2CH_3$ | N | CH | CH | CH | $CH_3$ | $CH_3$ | H | Z | 3,989 / 436,8 $[M+H]^+$ |
| I-356 | $O(CH_2)_3CH(CH_3)$ $_2$ | N | CH | CH | CH | $CH_3$ | $CH_3$ | H | Z | 4,013/436,8 [M+H]+ |
| I-357 | $OCH_2CH(CH_3)C$ $H_2-C(CH_3)_3$ | N | CH | CH | CH | $CH_3$ | $CH_3$ | H | Z | 4,327 / 462,9 $[M+H]^+$ |
| I-358 | 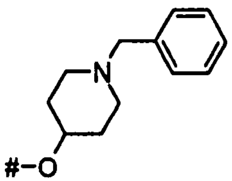 | N | CH | CH | CH | $CH_3$ | $CH_3$ | H | Z | 2,724 / 525,8 $[M+H]^+$ |
| I-359 | $O(CH_2)_4C\equiv CH$ | N | CH | CH | CH | $CH_3$ | $CH_3$ | H | Z | 3,475 / 432,8 $[M+H]^+$ |
| I-360 | $O(CH_2)_2C\equiv CH$ | N | CH | CH | CH | $CH_3$ | $CH_3$ | H | Z | 3.277 / 404.8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-361 | 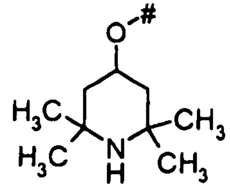 | N | CH | CH | CH | $CH_3$ | $CH_3$ | H | Z | 2,626 / 491,8 $[M+H]^+$ |
| I-362 | 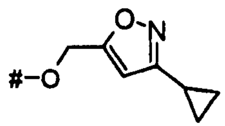 | N | CH | CH | CH | $CH_3$ | $CH_3$ | H | Z | 3,347 / 473,8 $[M+H]^+$ |
| I-363 | $N[(CH_2)_5CH_3)_2$ | N | CH | CH | CH | $CH_3$ | $CH_3$ | H | Z | 3,737 / 519,5 $[M+H]^+$ |
| I-364 | $N(CH_2C{\equiv}CC_2H_5)$ 2 | N | CH | CH | CH | $CH_3$ | $CH_3$ | H | Z | 3,625/483,4 $[M+H]^+$ |
| I-365 | $N(CH_2C{\equiv}CCH_3)_2$ | N | CH | CH | CH | $CH_3$ | $CH_3$ | H | Z | 3,165/455,4 $[M+H]^+$ |
| I-366 | $N[CH_2$-(4-CN-$C_6H_5)]_2$ | N | CH | CH | CH | H | $CH_3$ | H | Z | 3,622/581,8 $[M+H]^+$ |
| I-367 | $OCH_3$ | N | CH | CH | CH | $CH_3$ | $CH_3$ | H | Z | 222 |
| I-368 ** | CN | N | CH | C-$OCH_3$ | CH | H | $CH_3$ | H | Z | 154 |
| I-369 | $OCH_2CH_2CH_3$ | N | CH | CH | CH | $CH_3$ | $CH_3$ | H | Z | 98-99 |
| I-370 | $O(CH_2)_2$-(4-F-$C_6H_5$) | N | CH | CH | CH | $CH_3$ | $CH_3$ | H | Z | 3,693 / 474,7 $[M+H]^+$ |
| I-371 | $O(CH_2)_2C_6H_5$ | N | CH | CH | CH | $CH_3$ | $CH_3$ | H | Z | 3,665/456,8 $[M+H]^+$ |
| I-372 | $OCH_2$-(2-Cl-$C_6H_5$) | N | CH | CH | CH | $CH_3$ | $CH_3$ | H | Z | 3,786 / 477,7 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-373 | $O(CH_2)_2OCH_2CH$ 3 | N | CH | CH | CH | $CH_3$ | $CH_3$ | H | Z | 3,083 / 424,8 $[M+H]^+$ |
| I-374 | 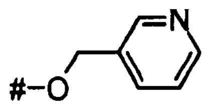 | N | CH | CH | CH | $CH_3$ | $CH_3$ | H | Z | 2,352 / 443,8 $[M+H]^+$ |
| I-375 | $N(CH_3)CH_2CH_2C$ $H_3$ | N | CH | CH | CH | $CH_3$ | $CH_3$ | H | Z | 2,313 / 407,8 $[M+H]^+$ |
| I-376 | $N(CH_3)CH_2CH=$ $CH_2$ | N | CH | CH | CH | $CH_3$ | $CH_3$ | H | Z | 2,240 / 405,8 $[M+H]^+$ |
| I-377 | $N(CH_3)(CH_2)_3CH$ 3 | N | CH | CH | CH | $CH_3$ | $CH_3$ | H | Z | 2,482 / 420,8 $[M+H]^+$ |
| I-378 | $N(CH_3)CH(CH_3)_2$ | N | CH | CH | CH | $CH_3$ | $CH_3$ | H | Z | 2,265 / 407,8 $[M+H]^+$ |
| I-379 | $N(CH_2CH_3)_2$ | N | CH | CH | CH | $CH_3$ | $CH_3$ | H | Z | 2,435 / 421,8 $[M+H]^+$ |
| I-380 | $O(CH_2)_5CH_3$ | N | CH | CH | CH | $CH_3$ | $CH_3$ | H | Z | 4,029 / 436,8 $[M+H]^+$ |
| I-381 | $OCH_2C(CH_3)_3$ | N | CH | CH | CH | $CH_3$ | $CH_3$ | H | Z | 3,705 / 422,8 $[M+H]^+$ |
| I-382 | $OCH_2CH_2CF_3$ | N | CH | CH | CH | $CH_3$ | $CH_3$ | H | Z | 3,389 / 448,7 $[M+H]^+$ |
| I-383 | $OCH_2CH=CH_2$ | N | CH | CH | CH | $CH_3$ | $CH_3$ | H | Z | 3,228 / 392,8 $[M+H]^+$ |
| I-384 | CN | N | CH | CH | CH | $(CH_2)_2-CH=CH_2$ | $CH_3$ | H | Z | 3,376 / 401,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-385 ** | $OCH_2CH_3$ | N | 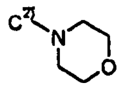 | CH | CH | $CH_3$ | $CH_3$ | H | Z | 3,371/464,8 [M+H]+ |
| I-386 ** | $OCH_3$ | N | CH | C-$CH_2$-$OCH_3$ | CH | $CH_3$ | $CH_3$ | H | Z | 2,958 / 410,35 [M+H]+ |
| I-387 ** | $OCH_3$ | N | CH | C-$C_2H_5$ | CH | $CH_3$ | $CH_3$ | H | Z | 3,338 / 394,35 [M+H]+ |
| I-388 ** | $OCH_2CH_3$ | N | CH | C-$C_2H_5$ | CH | $CH_3$ | $CH_3$ | H | Z | 3,454 / 407,8 [M+H]+ |
| I-389 ** | $OCH_2CH_3$ | N | CH | C-$C_2H_5$ | CH | H | $CH_3$ | H | Z | 2,637 / 395,8 [M+H]+ |
| I-390 ** | 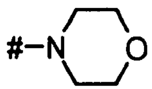 | N | CH | C-$CH_2$-$OCH_3$ | CH | $CH_3$ | $CH_3$ | H | Z | 131 - 132 |
| I-391 ** | 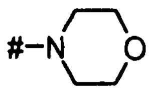 | N | C-Cl | CH | CH | $CH_3$ | $CH_3$ | H | Z | 3,261 / 454,8 [M+H]+ |
| I-392 ** | $CH_3$ | N | C2)-N($C_2H_5$)2 | CH | CH | $CH_3$ | $CH_3$ | H | Z | 2,42 / 420,8 [M+H]+ |
| I-393 ** | $CH_3$ | N | C2)-N($CH_3$)2 | CH | CH | $CH_3$ | $CH_3$ | H | Z | 118 |
| I-394 ** | $OCH_3$ | N | C-Cl | CH | CH | $CH_3$ | $CH_3$ | H | Z | 177 |
| I-395 ** | CN | N | C-$OCH_3$ | CH | CH | $CH_3$ | $CH_3$ | H | Z | 3,283/391,25 [M+H]+ |
| I-396 ** | CN | N | C2)-N($CH_3$)-N=CH-C | | CH | $CH_3$ | $CH_3$ | H | Z | 172 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-397 ** | $OCH_2CH_3$ | N | C2)-CH=CH-S-C3) | | CH | $CH_3$ | $CH_3$ | H | Z | 3,633 / 436,25 $[M+H]^+$ |
| I-398 ** | $OCH_2CH_3$ | N | C2)-N($CH_3$)-N=C($CH_3$)-C3) | | CH | $CH_3$ | $CH_3$ | H | Z | 182 |
| I-399 ** | $OCH_2CH_3$ | N | C-$CH_3$ | CH | CH | $CH_3$ | $CH_3$ | H | Z | 3,523 / 394,35 $[M+H]^+$ |
| I-400 ** | $CH_3$ | N | C2)-N$(CH_3)_2$ | CH | CH | $CH_3$ | $CH_3$ | H | Z | 2,145 / 392,8 $[M+H]^+$ x) |
| I-401 ** | Cl | N | C2)-N($CH_3$)-N=C($CH_3$)-C3) | | CH | $CH_3$ | $CH_3$ | H | Z | 180 |
| I-402 | CN | N | CH | CH | CH | $CH_3$ | $C_3H_5$ | H | Z | b) 5,150/387 $[M+H]^+$ |
| I-403 | OCH($CH_3$)CO-NH($CH_3$) | N | CH | CH | CH | $CH_3$ | $CH_3$ | H | Z | 2,652/437,8 $[M+H]^+$ |
| I-404 | OCH($CH_3$)CO-N$(CH_3)_2$ | N | CH | CH | CH | $CH_3$ | $CH_3$ | H | Z | 2,812 / 451,8 $[M+H]^+$ |
| I-405 | 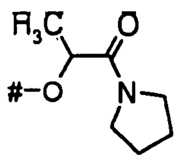 | N | CH | CH | CH | $CH_3$ | $CH_3$ | H | Z | 2,781 / 477,8 $[M+H]^+$ |
| I-406 | 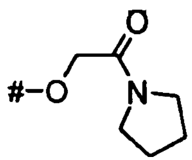 | N | CH | CH | CH | $CH_3$ | $CH_3$ | H | Z | 2,393 / 463,8 $[M+H]^+$ |
| I-407 | OCH($CH_3$)CO-N$(CH_2CH_3)_2$ | N | CH | CH | CH | $CH_3$ | $CH_3$ | H | Z | 2,587 / 465,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-408 | $OCH_2CH_2-C(CH_3)=CH_2$ | N | CH | CH | CH | $CH_3$ | $CH_3$ | H | Z | 3,567 / 420,8 $[M+H]^+$ |
| I-409 | $OCH(C_2H_5)C\equiv C\ CH_3$ | N | CH | CH | CH | $CH_3$ | $CH_3$ | H | Z | 3,595 / 432,8 $[M+H]^+$ |
| I-410 | $OCH_2CH_2F$ | N | CH | CH | CH | $CH_3$ | $CH_3$ | H | Z | 2,972/398,8 [M+H]+ |
| I-411 | $OCH_2CH(CH_3)-OCH_3$ | N | CH | CH | CH | $CH_3$ | $CH_3$ | H | Z | 3,050 / 424,8 $[M+H]^+$ |
| I-412 | $OCH_2C(CH_3)_2CN$ | N | CH | CH | CH | $CH_3$ | $CH_3$ | H | Z | 3,143 / 433,8 $[M+H]^+$ |
| I-413 | $OCH_2C\equiv CCH_3$ | N | CH | CH | CH | $CH_3$ | $CH_3$ | H | Z | 3,375 / 404,4 $[M+H]^+$ |
| I-414 | $OCH_2C(C_2H_5)=C\ H_2$ | N | CH | CH | CH | $CH_3$ | $CH_3$ | H | Z | 3,770 / 420,4 $[M+H]^+$ |
| I-415 | 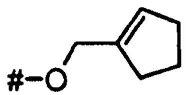 | N | CH | CH | CH | $CH_3$ | $CH_3$ | H | Z | 3,862 / 432,4 $[M+H]^+$ |
| I-416 | $OCH_2C(CH_3)=C\ H-CH_2CH_3$ | N | CH | CH | CH | $CH_3$ | $CH_3$ | H | Z | 3,969 / 434,4 $[M+H]^+$ |
| I-417 ** | $CH_3$ | N | C-OH | CH | CH | $CH_3$ | $CH_3$ | H | Z | 2,189/366,25 $[M+H]_+$ |
| I-418 ** | $OCH_2CH_3$ | N | CH | C-F | CH | $CH_3$ | $CH_3$ | H | Z | 3,368 / 415,8 $[M+H]^+$ |
| I-419 | 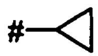 | N | CH | CH | CH | H | $CH_3$ | H | Z | 166 |
| I-420 ** | $OCH_2CH_3$ | N | CH | CH | $C-CH_3$ | $CH_3$ | $CH_3$ | H | Z | 3,182 / 393,8 [M+H]+ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-421 ** | $OCH_3$ | N | C3)-Cl | C3)-CH=CH-CH=CH-C | | $CH_3$ | $CH_3$ | H | Z | 4,109 / 449,8 $[M+H]^+$ |
| I-422 | $N[(CH_2)_3CH_3]_2$ | N | CH | CH | CH | $CH_3$ | $CH_3$ | H | Z | 3,955 / 463,9 $[M+H]^+$ |
| I-423 | $N[CH_2CH(CH_3)_2]$ 2 | N | CH | CH | CH | $CH_3$ | $CH_3$ | H | Z | 3,086 / 463,9 $[M+H]^+$ |
| I-424 | $NH(CH_2)_3CH_3$ | N | CH | CH | CH | $CH_3$ | $CH_3$ | H | Z | 2,539 / 407,9 $[M+H]^+$ |
| I-425 | $NHCH_2CH(CH_3)_2$ | N | CH | CH | CH | $CH_3$ | $CH_3$ | H | Z | 2,524/407,9 $[M+H]^+$ |
| I-426 | $NH(CH_2)_5CH_3$ | N | CH | CH | CH | $CH_3$ | $CH_3$ | H | Z | 2,853 / 435,6 $[M+H]^+$ |
| I-427 | $OCH_2CF_2$ | N | CH | CH | CH | $CH_3$ | $CH_3$ | H | Z | 3,280 / 416,3 $[M+H]^+$ |
| I-428 | $O(CH_2)_2OCH_3$ | N | CH | CH | CH | $CH_3$ | $CH_3$ | H | Z | 2,866 / 409,8 $[M+H]^+$ |
| I-429 | $PO(OH)_2$ | N | CH | CH | CH | $CH_3$ | $CH_3$ | H | Z | 1,760 / 416,3 $[M+H]^+$ |
| I-430 | $O(CH_2)_3OH$ | N | CH | CH | CH | $CH_3$ | $CH_3$ | H | Z | 2,608 / 410,8 $[M+H]^+$ |
| I-431 | $OCH_2CH(CH_3)O$ H | N | CH | CH | CH | $CH_3$ | $CH_3$ | H | Z | 2,689 / 410,8 $[M+H]^+$ |
| I-432 | $OCH(CH_3)C{\equiv}CH$ | N | CH | CH | CH | $CH_3$ | $CH_3$ | H | Z | 3,230 / 404,8 [M+H]+ |
| I-433 | 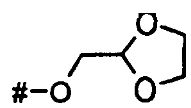 | N | CH | CH | CH | $CH_3$ | $CH_3$ | H | Z | 2,852 / 438,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-434 | $OCH_2C(Cl)C=C H_2$ | N | CH | CH | CH | $CH_3$ | $CH_3$ | H | Z | 3,438 / 426,7 $[M+H]^+$ |
| I-435 | 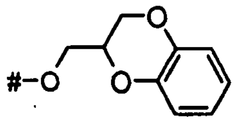 | N | CH | CH | CH | $CH_3$ | $CH_3$ | H | Z | 3,617 / 500,7 $[M+H]^+$ |
| I-436 | 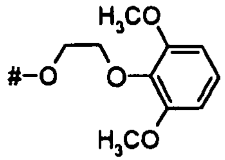 | N | CH | CH | CH | $CH_3$ | $CH_3$ | H | Z | 3,412 / 532,7 $[M+H]^+$ |
| I-437 | $OCH_2CH(CH_3)_2$ | N | CH | CH | CH | $CH_3$ | $CH_3$ | H | Z | 3,655 / 408,4 $[M+H]^+$ |
| I-438 | $O(CH_2)_2CH(CH_3)$ 2 | N | CH | CH | CH | $CH_3$ | $CH_3$ | H | Z | 3,893 / 422,4 $[M+H]^+$ |
| I-439 | 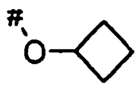 | N | CH | CH | CH | $CH_3$ | $CH_3$ | H | Z | 3,537 / 406,4 $[M+H]^+$ |
| I-440 | $OCH_3$ | N | CH | CH | CH | $CH_2CH_2CH_3$ | $CH_3$ | H | Z | 158 |
| I-441 ** | $OCH_3$ | N | $C^{2)}$-$OCH_3$ | CH | CH | $CH_3$ | $CH_3$ | H | Z | 103 - 104 |
| I-442 | 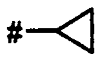 | N | CH | CH | CH | $CH_3$ | $CH_3$ | H | Z | 98-99 |
| I-443 | $CH_2CH_3$ | N | CH | CH | CH | $CH_3$ | $CH_3$ | H | Z | 1,965 / 363,8 $[M+H]^+$ |
| I-444 | $CH_2CH_2CH_3$ | N | CH | CH | CH | $CH_3$ | $CH_3$ | H | Z | 2,110 / 377,8 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-445 | CN | N | CH | CH | CH | $CH_2CH_2CH_2Cl$ | $CH_3$ | H | Z | 3,330 / 423,2 $[M+H]^+$ |
| I-446 | CN | N | CH | CH | CH | $(CH_2)_2$-$CH(CH_3)_2$ | $CH_3$ | H | Z | 3,702 / 417,4 $[M+H]^+$ |
| I-447 | CN | N | CH | CH | CH | $CH_2CH(CH_3)_2$ | $CH_3$ | H | Z | 3,478 / 403,4 $[M+H]^+$ |
| I-448 | $CO_2CH_2CH_3$ | N | CH | CH | CH | H | $CH_3$ | H | Z | 2,370 393,8 $[M+H]^+$ |
| I-449 | $OCH_2CH_3$ | N | CH | CH | CH | $CH_2CH_3$ | $CH_3$ | H | Z | 3,377 / 394,4 $[M+H]^+$ |
| I-450 | $OCH_2CH_3$ | N | CH | CH | CH | $(CH_2)_5CH_3$ | $CH_3$ | H | Z | 4,316 / 450,4 $[M+H]^+$ |
| I-451 | $OCH_2CH_3$ | N | CH | CH | CH | $(CH_2)_4CH_3$ | $CH_3$ | H | Z | 4,107 / 436,4 $[M+H]^+$ |
| I-452 | $OCH_2CH_3$ | N | CH | CH | CH | $CH(CH_3)_2$ | $CH_3$ | H | Z | 4,321 / 408,4 $[M+H]^+$ |
| I-453 | $OCH_2CH_3$ | N | CH | CH | CH | $CH_2CH(CH_3)_2$ | $CH_3$ | H | Z | 3,870 / 422,4 $[M+H]^+$ |
| I-454 | $OCH_2CH_3$ | N | CH | CH | CH | $CH_2CH$=$CH$-$CH_3$ | $CH_3$ | H | Z | 3,751 / 420,4 $[M+H]^+$ |
| I-455 | $OCH_2CH_3$ | N | CH | CH | CH | $CH_2C$=$CCH_3$ | $CH_3$ | H | Z | 3,609 / 418,4 $[M+H]^+$ |
| I-456 | Br | N | CH | CH | CH | $CH_2C\equiv CH$ | $CH_3$ | H | Z | 3,167 / 438,1 $[M+H]^+$ |
| I-457 | Br | N | CH | CH | CH | $CH_2$-$C(CH_3)$=$CH_2$ | $CH_3$ | H | Z | 3,435 / 454,4 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-458 | Br | N | CH | CH | CH | $(CH_2)_3CH_3$ | $CH_3$ | H | Z | 3,671 /456,3 $[M+H]^+$ |
| I-459 | Br | N | CH | CH | CH | $CH_2CH_3$ | $CH_3$ | H | Z | 3,132 / 428,3 $[M+H]^+$ |
| I-460 | Br | N | CH | CH | CH | $(CH_2)_5CH_3$ | $CH_3$ | H | Z | 4,152 / 484,3 $[M+H]^+$ |
| I-461 | Br | N | CH | CH | CH | $(CH_2)_4CH_3$ | $CH_3$ | H | Z | 3,918 / 470,2 $[M+H]^+$ |
| I-462 | Br | N | CH | CH | CH | $CH(CH_3)_2$ | $CH_3$ | H | Z | 4,190 / 442,3 $[M+H]^+$ |
| I-463 | Br | N | CH | CH | CH | $CH_2CH(CH_3)_2$ | $CH_3$ | H | Z | 3,648 / 456,4 $[M+H]^+$ |
| I-464 | Br | N | CH | CH | CH | $CH_2CH=CH-CH_3$ | $CH_3$ | H | Z | 3,525 / 454,2 $[M+H]^+$ |
| I-465 | Br | N | CH | CH | CH | $CH_2CH_2CH_3$ | $CH_3$ | H | Z | 3,406 / 442,4 $[M+H]^+$ |
| I-466 | Br | N | CH | CH | CH | $CH_2C{\equiv}CCH_3$ | $CH_3$ | H | Z | 3,373 / 452,2 $[M+H]^+$ |
| I-467 ** | $OCH_3$ | N | CH | CH | C-I | $CH_3$ | $CH_3$ | H | Z | 103 - 104 |
| I-468 ** | 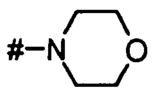 | N | CH | $C-C_2H_5$ | CH | $CH_3$ | $CH_3$ | H | Z | 2,451 / 448,9 $[M+H]^+$ x) |
| I-469 ** | $CF_3$ | N | CH | CH | $C-OCH_3$ | H | $CH_3$ | H | Z | 150 |
| I-470 ** | Cl | N | $C^{2)}-N(CH_3)-N=C(C_3H_5)-C^{3)}$ | | CH | $CH_3$ | $CH_3$ | H | Z | 3,358 / 463,7 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-471 ** | $CF_3$ | N | CH | CH | C-$OCH_3$ | $CH_3$ | $CH_3$ | H | Z | 170 |
| I-472 ** | $CF_3$ | N | C3)-CH=CH-S-C | | CH | $CH_3$ | $CH_3$ | H | Z | 243 |
| I-473 ** | $CF_3$ | N | CH | CH | C-$OCH_3$ | $CH_2CH=CH_2$ | $CH_3$ | H | Z | 3,337 / 459,7 $[M+H]^+$ |
| I-474 ** | $OCH_2CH_3$ | N | C3)-N($CH_3$)-N=C($C_3H_5$)-C3) | | CH | $CH_3$ | $CH_3$ | H | Z | 3,547 / 473,8 $[M+H]^+$ |
| I-475 ** | CN | N | C3)-N($CH_3$)-N=C($C_3H_5$)-C3) | | CH | $CH_3$ | $CH_3$ | H | Z | 3,354 / 454,8 $[M+H]^+$ |
| I-476 ** | $OCH_3$ | N | CH | C3)-CH=CH-CH=CH-C | | $CH_3$ | $CH_3$ | H | Z | 3,574 / 415,9 $[M+H]^+$ |
| I-477 | $OCH_2CH_3$ | N | CH | CH | CH | H | $CH_3$ | H | Z | 161 |
| I-478 | CN | N | CH | CH | CH | $CH_3$ | $NH_2$ | H | Z | 3,798 / 345,9 $[M-NH_3+H]^+$ |
| I-479 | $PO(OH)(OC_2H_5)$ | N | CH | CH | CH | $CH_3$ | $CH_3$ | H | Z | 162-163 |
| I-480 | $NHCH_3$ | N | CH | CH | CH | H | $CH_3$ | H | Z | 175 |
| I-481 | $PO(CH_3)(OC_2H_5)$ | N | CH | CH | CH | $CH_3$ | $CH_3$ | H | Z | 65-70 |
| I-482 | $SCH_2C_6H_5$ | N | CH | CH | CH | $CH_3$ | $CH_3$ | H | Z | 154-158 |
| I-483 ** | $OCH_2CH_3$ | N | CH | C-F | CH | $CH_2CH_2CH_3$ | $CH_3$ | 3-F | Z | 3,879 / 444,8 $[M+H]^+$ |
| I-484 ** | Cl | N | CH | C-$OC_2H_5$ | CH | $CH_2CH_2CH_3$ | $CH_3$ | 3-F | Z | 3,372 / 459,8 $[M+H]^+$ |
| I-485 | $SO_2CH_2C_6H_5$ | N | CH | CH | CH | $CH_3$ | $CH_3$ | H | Z | 3,403 / 490,1 $[M+H]^+$ |
| I-486 | $SO_2CH_2C_6H_5$ | N | CH | CH | CH | $CH_3$ | $CH_3$ | H | E | 88-89 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-487 | CN | N | CH | CH | CH | $COC(CH_3)_3$ | $CH_3$ | H | Z | 3,674 / 430,8 $[M+H]^+$ |
| I-488 | $CH_2CH_2CH_2CH_3$ | N | CH | CH | CH | H | $CH_3$ | H | Z | 2,010 / 378,3 $[M+H]^+$ |
| I-489 | $CH_2CH_2CH_2CH_3$ | N | CH | CH | CH | $CH_3$ | $CH_3$ | H | Z | 2,275 / 391,9 $[M+H]^+$ |
| I-490 | $CH_2CH_2CH_2CH_3$ | N | CH | CH | CH | $CH_2CH=CH_2$ | $CH_3$ | H | Z | 2,640 / 418,2 $[M+H]^+$ |
| I-491 | $CH_2CH_2CH_2CH_3$ | N | CH | CH | CH | H | $CH_3$ | H | E | 2,275 / 377,8 $[M+H]^+$ |
| I-492 | $CH_2CH_2CH_2$-C=C=$CH_2$ | N | CH | CH | CH | $CH_3$ | $CH_3$ | H | Z | 3,587 / 432,1 $[M+H]^+$ |
| I-493 | $C(CH_3)$=N-OH (Z) | N | CH | CH | CH | $CH_3$ | $CH_3$ | H | Z | 2,022/393,15 $[M+H]^+$ |
| I-494 | $C(CH_3)$=N-$OCH_3$ (Z) | N | CH | CH | CH | $CH_3$ | $CH_3$ | H | Z | 119 |
| I-495 | $C(CH_3)CHOH$ | N | CH | CH | CH | $CH_3$ | $CH_3$ | H | Z | 80 |
| I-496 | $C(CH_3)$=N-$OCH_3$ (E) | N | CH | CH | CH | $CH_3$ | $CH_3$ | H | Z | 2,419 / 406,8 $[M+H]^+$ |
| I-497 | 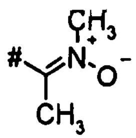 | N | CH | CH | CH | $CH_3$ | $CH_3$ | H | Z | 190 |
| I-498 | $COCH_3$ | N | CH | CH | CH | H | $CH_3$ | H | Z | 154 |
| I-499 | $C(CH_3)$=N-OH (E) | N | CH | CH | CH | $CH_3$ | $CH_3$ | H | Z | 200 |
| I-500 | $C(OC_2H_5)=CH_2$ | N | CH | CH | CH | $CH_3$ | $CH_3$ | H | Z | 108 |
| I-501 | $PO(C_2H_5)_2$ | N | CH | CH | CH | $CH_3$ | $CH_3$ | H | Z | 165-176 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-502 | $PO(CH_3)(OH)$ | N | CH | CH | CH | $CH_3$ | $CH_3$ | H | Z | 79-82 |
| I-503 | $OCH_2C_6H_5$ | N | CH | CH | CH | $CH_3$ | $CH_3$ | H | Z | 165-170 |
| I-504 | $OCH_2C_6H_5$ | N | CH | CH | CH | $CH_3$ | $CH_3$ | H | E | 135-137 |
| I-505 ** | $OCH_3$ | N | CH | $C-(CH_2)_4-C$ | | $CH_3$ | $CH_3$ | H | Z | 3,508/420,35 $[M+H]^+$ |
| I-506 ** | $CF_3$ | N | CH | CH | $C-OCH_3$ | $CH_2CH_3$ | $CH_3$ | H | Z | 3,205 / 447,7 $[M+H]^+$ |
| I-507 ** | $OCH_2CH_3$ | N | $C^{2)}-N(CH_3)-C(CH_3)=N-C$ | | CH | $CH_3$ | $CH_3$ | H | Z | 242 |
| I-508 ** | $OCH_3$ | N | $C^{2)}-N(CH_3)-C(CH_3)=N-C$ | | CH | $CH_3$ | $CH_3$ | H | Z | 230 |
| I-509 ** | 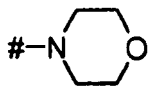 | N | $C^{2)}-N(CH_3)-C(CH_3)=N-C$ | | CH | $CH_3$ | $CH_3$ | H | Z | 212 |
| I-510 | $OCH_2OCH_3$ | N | CH | CH | CH | $CH_3$ | $CH_3$ | H | Z | 2,441 / 396,1 $[M+H]^+$ |
| I-511 | $OCH_3$ | N | CH | CH | CH | $CH_2CH=CH_2$ | $CH_3$ | H | Z | 181 |
| I-512 | $OCH_3$ | N | CH | CH | CH | $CH_2CH_3$ | $CH_3$ | H | Z | 151 |
| I-513 | $OCH_3$ | N | CH | CH | CH | $CH_2C{\equiv}CH$ | $CH_3$ | H | Z | 170 |
| I-514 | $SCH_2CH_2CH_3$ | N | CH | CH | CH | $CH_3$ | $CH_3$ | H | Z | 3,427 /409,8 $[M+H]^+$ |
| I-515 | $SCH(CH_3)_2$ | N | CH | CH | CH | $CH_3$ | $CH_3$ | H | Z | 3,384 / 409,8 $[M+H]^+$ |
| I-516 | $SCH_2CH_3$ | N | CH | CH | CH | $CH_3$ | $CH_3$ | H | Z | 3,311 / 396,15 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-517 | $SCH_2CH_3$ | N | CH | CH | CH | $CH_3$ | $CH_3$ | H | E | 2,839 / 396,15 $[M+H]^+$ |
| I-518 ** | Cl | N | CH | $C-OC_2H_5$ | CH | $CH_3$ | $CH_3$ | H | E | 133 |
| I-519 ** | $OCH_2CH_3$ | N | CH | CH | $C-OCH_3$ | $CH_3$ | $CH_3$ | H | Z | 3,051 / 410,15 $[M+H]^+$ |
| I-520 ** | $OCH_2CH_3$ | N | CH | CH | C-Cl | $CH_3$ | $CH_3$ | H | Z | 3,581/41,15 $[M+H]^+$ |
| I-521 ** | $OCH_3$ | N | CH | C-Cl | CH | $CH_3$ | $CH_3$ | H | Z | 2,900/400,15 $[M+H]^+$ |
| I-522 ** | $OCH_2CH_3$ | N | CH | C-F | CH | $CH_3$ | $CH_3$ | H | Z | 100 |
| I-523 ** | Cl | N | CH | CH | $C-OC_2H_5$ | $CH_3$ | $CH_3$ | H | Z | 125 |
| I-524 ** | $OCH_3$ | N | CH | $C-OCH_3$ | CH | $CH_3$ | $CH_3$ | H | Z | 140 |
| I-525 | $OCH_2C_6H_5$ | N | CH | CH | CH | H | $CH_3$ | H | Z | 169.-172 |
| I-526 | $COCH_3$ | N | CH | CH | CH | $CH_2CH_3$ | $CH_3$ | H | Z | 144-146 |
| I-527 | $COCH_3$ | N | CH | CH | CH | $CH_2CH_2CH_3$ | $CH_3$ | H | Z | 3,054 / 405,8 $[M+H]^+$ |
| I-528 | OH | N | CH | CH | CH | H | $CH_3$ | H | Z | 1,957 / 337,8 $[M+H]^+$ |
| I-529 | $COCH_2CH_3$ | N | CH | CH | CH | $CH_3$ | $CH_3$ | H | Z | 118 |
| I-530 | $COCH_3$ | N | CH | CH | CH | $CH_3$ | $CH_3$ | H | E | 166 |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-531 | 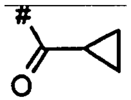 | N | CH | CH | CH | $CH_3$ | $CH_3$ | H | Z | 135 |
| I-532 | $CH_2CH_2CH_2CH_3$ | N | CH | CH | CH | $CH_2CH_2CH_3$ | $CH_3$ | H | Z | 2,498 / 419,9 $[M+H]^+$ |
| I-533 | $NHCH_3$ | N | CH | CH | CH | $CH_2CH=CH_2$ | $CH_3$ | H | Z | 159 - 160 |
| I-534 | $NHCH_3$ | N | CH | CH | CH | $CH_2C{\equiv}CH$ | $CH_3$ | H | Z | 231 |
| I-535 | $N(CH_3)CH_2CH= CH_2$ | N | CH | CH | CH | $CH_2CH=CH_2$ | $CH_3$ | H | Z | 2,565 / 430,8 $[M+H]^+$ |
| I-536 | $COCH(CH_3)_2$ | N | CH | CH | CH | $CH_3$ | $CH_3$ | H | Z | 3,310/406,15 $[M+H]^+$ |
| I-537 | $COCH_2Br$ | N | CH | CH | CH | $CH_3$ | $CH_3$ | H | Z | 101 |
| I-538 | 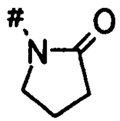 | N | CH | CH | CH | $CH_3$ | $CH_3$ | H | Z | 2,620 / 419,15 $[M+H]^+$ |
| I-539 | Br | N | CH | CH | CH | $CH_3$ | $CH_3$ | H | Z | 3,300/440,0 $[M+Na]^+$ |
| I-540 | 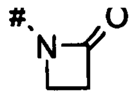 | N | CH | CH | CH | $CH_3$ | $CH_3$ | H | Z | 2,556 / 405,1 $[M+H]^+$ |
| I-541 | CN | N | CH | CH | CH | $(CH_2)_3CH_3$ | $CH_3$ | H | Z | 3,480/403,1 $[M+H]^+$ |
| I-542 | $COCH_3$ | N | CH | CH | CH | $CH_2CH=CH_2$ | $CH_3$ | H | Z | 2.992 / 404,1 $[M+H]^+$ |
| I-543 | $COCH_3$ | N | CH | CH | CH | $CH_2C{\equiv}CH$ | $CH_3$ | H | Z | 3,036/402,15 $[M+H]^+$ |

(fortgesetzt)

| Nr. | $R^a$ | V | W | X | Y | $R^1$ | $R^3$ | $R^9/R^{10}$ | Isomer*) | phys. Daten (Fp.[°C]; HPLC: RT[min] /m/z) |
|---|---|---|---|---|---|---|---|---|---|---|
| I-544 | $OCH_2CH_3$ | N | CH | CH | CH | $CH_2CHF_2$ | $CH_3$ | H | Z | 3,447 / 430,1 $[M+H]^+$ |
| I-545 | $C(OCH_3)_2CH_2OC\ H_3$ | N | CH | CH | CH | $CH_3$ | $CH_3$ | H | Z | 159 |
| I-546 | $C(OCH_3)_2CH_2Br$ | N | CH | CH | CH | $CH_3$ | $CH_3$ | H | Z | 2,489 / 502,1 $[M+H]^+$ |
| I-547 | CHO | N | CH | CH | CH | $CH_3$ | $CH_3$ | H | Z | 183 |
| I-548 | $CH_2OH$ | N | CH | CH | CH | $CH_3$ | $CH_3$ | H | Z | 117 |
| I-549 | $COCH_2Cl$ | N | CH | CH | CH | $CH_3$ | $CH_3$ | H | Z | 161 |
| I-550 | $COCH_2OCH_3$ | N | CH | CH | CH | $CH_3$ | $CH_3$ | H | Z | 2,634 / 408,1 $[M+H]^+$ |
| I-551 | CN | N | CH | CH | CH | $CH_2F$ | $CH_3$ | H | Z | 3,323 / 378,8 $[M]^+$ |
| I-552 | CN | N | CH | CH | CH | $CH_2C{\equiv}CCH_3$ | $CH_3$ | H | Z | 3,152 / 399,2 $[M+H]^+$ |
| I-553 | CN | N | CH | CH | CH | $CH_2$-CH=CHCH$_3$ | $CH_3$ | H | Z | 3,215 / 400,8 $[M+H]^+$ |
| I-554 | CN | N | CH | CH | CH | $CH_2$-C(CH$_3$)=CH$_2$ | $CH_3$ | H | Z | 3,108 / 400,8 $[M+H]^+$ |
| I-555 | CN | N | CH | CH | CH | $(CH_2)_5CH_3$ | $CH_3$ | H | Z | 4,024 / 431,4 $[M+H]^+$ |
| I-556 | CN | N | CH | CH | CH | $(CH_2)_4CH_3$ | $CH_3$ | H | Z | 4,139 / 417,5 $[M+H]^+$ |

** kennzeichnet die nicht erfindungegemäßen Beispiele
# kennzeichnet die Bindung zu dem C-Atom, an welches $R^a$ gebunden ist
*) Diese Angabe bezieht sich auf die Stereochemie der Doppelbindung am Piperazingerüst. Bei den hergestellten Verbindungen handelt es sich jeweils um das Racemat.
1) kennzeichnet das Ring-Atom V 2) kennzeichnet das Ring-Atom W 3) kennzeichnet das Ring-Atom X x) Verbindung lag als Trifluoracetat vor

Tabelle II: Verbindungen der Formel welches der Formel I.B' entsprechen:

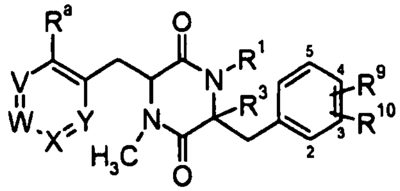

**I.B'** $R^3 = CH_3$ ; $R^9$ / $R^{10}$ = H

| Nr. | $R^a$ | V | W | X | Y | $R^1$ | Isomer | phys. Daten (Fp.[°C]; HPLC: RT [min]/m/z) |
|---|---|---|---|---|---|---|---|---|
| II-1 | Br | N | CH | CH | CH | $CH_3$ | cis | 2,808 / 412,8 $[M+2]^+$ |
| II-2 ** | $CH_2Br$ | N | $C^{2)}$-CH=CH-CH=CH-$C^{3)}$ | | N | $CH_3$ | cis | 2,967 / 482,8 $[M+2]^+$ |
| II-3 ** | OH | CH | CH | CH | N | $CH_3$ | cis | 2,043 / 355,2 $[M+2]^+$ |
| II-4 ** | $CH_3$ | $C^{1)}$-$OCH_2CF_3$ | CH | CH | N | $CH_3$ | cis | 2,355 / 448,2 $[M]^+$ |
| II-5 ** | Cl | N | $C^{2)}$-CH=CH-CH=CH-$C^{3)}$ | | CH | $CH_3$ | cis | 3,241 / 423,3 $[M+H]^+$ |
| II-6 | CN | N | CH | CH | CH | $CH_3$ | cis | 2,646 / 364,2 $[M+2]^+$ |
| II-7 ** | $OCH_3$ | $C^{1)}$-$OCH_3$ | CH | CH | N | $CH_3$ | cis | 2,111 / 398,3 $[M+H]^+$ |
| II-8 ** | $CH_3$ | $C^{1)}$-$O(CH_2)_3OCH_3$ | CH | CH | N | $CH_3$ | cis | 2,381 / 440,4 $[M-HCl]^+$ |
| II-9 ** | Cl | N | $C^{2)}$-$CH_3$ | CH | C-Cl | $CH_3$ | cis | 3,076 / 421,1 [M+H], |
| II-10 | $OCH_2CH_3$ | N | CH | CH | CH | $CH_3$ | cis | 129 |

(fortgesetzt)

| Nr. | $R^a$ | V | W | X | Y | $R^1$ | Isomer | phys. Daten (Fp.[°C]; HPLC: RT [min]/m/z) |
|---|---|---|---|---|---|---|---|---|
| II-11 ** | CN | N | $C^{2)}$-CN | CH | CH | $CH_3$ | cis | 2,821/409,8 $[M+Na]^+$ |
| II-12 | $OCH_2CH_3$ | N | CH | CH | CH | $CH_2CH_2CH_3$ | cis | 118 |
| II-13 ** | Cl | CH | $C^{2)}$-$CF_3$ | CH | N | $CH_3$ | cis | 3,382/441,1 [M+H]+ |
| II-14 ** | $CH_3$ | $C^{l)}$-$OCH_3$ | $C^{2)}$-$CH_3$ | CH | N | $CH_3$ | cis | 2,291 / 396,4 $[M+H]^+$ |
| II-15 ** | Cl | N | $C^{2)}$-CH=C($OCH_3$)-CH=CH-$C^{3)}$ | | CH | $CH_3$ | cis | 3,271 / 453,3 $[M+H]^+$ |
| II-16 | $OCH_2CH_3$ | N | CH | CH | CH | $CH_3$ | trans | 99 |
| II-17 | $OCH_2CH_3$ | N | CH | CH | CH | H | cis | 162 |
| II-18 | $OCH_3$ | N | CH | CH | CH | $CH_3$ | trans | 2,583 / 368,3 $[M+H]^+$ |
| II-19 | $OCH_3$ | N | CH | CH | CH | $CH_3$ | cis | 2,460/368,3 $[M+H]^+$ |
| II-20 ** | $CH_3$ | CH | CH | CH | N | $CH_3$ | cis | 2,106 / 352,3 [M+H]+ |
| II-21 | $OCH(CH_3)_2$ | N | CH | CH | CH | $CH_3$ | trans | 134 |
| II-22 | $OCH(CH_3)_2$ | N | CH | CH | CH | $CH_3$ | cis | 153 |
| II-23 | $OCH_2CH_3$ | N | CH | CH | CH | $CH_2CH=CH_2$ | cis | 122 |
| II-24 | $OCH_2CF_3$ | N | CH | CH | CH | $CH_3$ | trans | 3,141 / 435,7 [M+H]+ |
| II-25 ** | $CH_3$ | N | $C^{2)}$-$CH_3$ | CH | CH | $CH_3$ | cis | 1,850 / 370,2 $[M+H]^+$ |
| II-26 ** | $CH_3$ | N | $C^{2)}$-F | CH | CH | $CH_3$ | cis + trans | 2,624 / 370,2 $[M+H]^+$ |

(fortgesetzt)

| Nr. | Rª | V | W | X | Y | R¹ | Isomer | phys. Daten (Fp.[°C]; HPLC: RT [min]/m/z) |
|---|---|---|---|---|---|---|---|---|
| II-27 | $OCH_2CF_3$ | N | CH | CH | CH | $CH_3$ | cis | 3,141 / 435,7 [M+H]⁺ |
| II-28 ** | $OCH_2CH_3$ | N | CH | CH | $C^{4)}$-$OCH_2CH_3$ | $CH_3$ | trans | 132 |
| II-29 ** | $OCH_2CH_3$ | N | CH | CH | $C^{4)}$-$OCH_2CH_3$ | $CH_3$ | cis | 124 |
| II-30 ** | $OCH_3$ | N | CH | C-$CH_2OCH_3$ | CH | $CH_3$ | cis | 2,665/412,35 [M+H]⁺ |
| II-31 ** | $OCH_3$ | N | CH | $C^{3)}$-$CH_2CH_3$ | CH | $CH_3$ | cis | 2,869/396,35 [M+H]⁺ |
| II-32 | $OCH_2CH_3$ | N | CH | CH | CH | $CH_2C{\equiv}CH$ | cis | 123 |
| II-33 ** | $OCH_3$ | N | CH | $C^{3)}$-$CH_2CH_3$ | CH | $CH_3$ | trans | 2,836 / 396,35 [M+H]⁺ |
| II-34 ** | $OCH_3$ | N | $C^{2)}$-OH | CH | CH | $CH_3$ | cis | 2,026/368,25 [M+H]⁺ |
| II-35 ** | $CH_3$ | N | $C^{2)}$-$NH_2$ | CH | CH | $CH_3$ | cis + trans | 112 |
| II-36 ** | $OCH_2CH_3$ | N | $C^{2)}$-$OCH_3$ | CH | CH | $CH_3$ | trans | 2,911/396,35 [M+H]⁺ |
| II-37 | $OCH_2CH_2C\ H_3$ | N | CH | CH | CH | $CH_3$ | cis | 2,995 / 395,9 [M+H]⁺ |
| II-38 | $OCH_2CH_3$ | N | CH | CH | CH | $CH_2CH_3$ | cis | 136 |
| II-39 | $OCH_2CH_3$ | N | CH | CH | CH | $CH_2CH{=}CHCH_3$ | cis | 87 |
| II-40 | $OCH_2CH_3$ | N | CH | CH | CH | $(CH_2)_3CH_3$ | cis | 85 |
| II-41 ** | $OCH_2CH_3$ | N | CH | C-$CH_3$ | CH | $CH_3$ | cis | 2,605 / 395,9 [M+H]⁺ |
| II-42 | 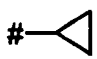 | N | CH | CH | CH | H | trans | 1,738/ 363,8 [M+H]⁺ |

(fortgesetzt)

| Nr. | $R^a$ | V | W | X | Y | $R^1$ | Isomer | phys. Daten (Fp.[°C]; HPLC: RT [min]/m/z) |
|---|---|---|---|---|---|---|---|---|
| II-43 | 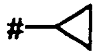 | N | CH | CH | CH | H | cis | 1,770 / 364,4 $[M+H]^+$ |
| II-44 ** | $CO_2CH_2CH$ 3 | CH | N | $C^{3)}$-$N(CH_3)_2$ | N | $CH_3$ | cis | 3,305 / 454,4 $[M+H]^+$ |
| II-45 | $OCH_2CHF_2$ | N | CH | CH | CH | $CH_3$ | trans | 2,956 / 418,2 $[M+H]^+$ |
| II-46 | $OCHF_2$ | N | CH | CH | CH | $CH_3$ | trans | 121 |
| II-47 ** | $OCH_3$ | N | CH | C-F | CH | $CH_3$ | trans | 110 |
| II-48 ** | $OCH_3$ | N | $C^{2)}$-CH=C($OCH_3$)-CH=CH-$C^{3)}$ | | CH | $CH_3$ | trans | 3,065 / 448,4 $[M+H]^+$ |
| II-49 | $OCHF_2$ | N | CH | CH | CH | $CH_3$ | cis | 183 |
| II-50 ** | $OCH_3$ | N | CH | C-F | CH | $CH_3$ | cis | 169 |
| II-51 ** | $OCH_3$ | N | $C^{2)}$-CH=C($OCH_3$)-CH=CH-$C^{3)}$ | | CH | $CH_3$ | cis | 84 |
| II-52 ** | $OCH_3$ | N | $C^{2)}$-CH=CH-CH=CH-$C^{3)}$ | | CH | $CH_3$ | cis | 3,134 / 417,8 $[M+H]^+$ |
| II-53 ** | $OCH_3$ | N | CH | C-$CH_3$ | CH | $CH_3$ | cis | 2,482 / 381,8 $[M+H]^+$ |
| II-54 | 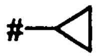 | N | CH | CH | CH | $CH_3$ | cis | 109 |
| II-55 | $CH_2CH_3$ | N | CH | CH | CH | $CH_3$ | cis | 121 |
| II-56 | $CH_2CH_2CH$ 3 | N | CH | CH | CH | $CH_3$ | cis | 116 |
| II-57 ** | $OCH_3$ | N | CH | C-$CH_3$ | CH | $CH_3$ | trans | 2,430 / 381,8 [M+H]+ |
| II-58 | $CH_2CH_3$ | N | CH | CH | CH | $CH_3$ | trans | 1,906 / 366,1 $[M+H]^+$ |

(fortgesetzt)

| Nr. | $R^a$ | V | W | X | Y | $R^1$ | Isomer | phys. Daten (Fp.[°C]; HPLC: RT [min]/m/z) |
|---|---|---|---|---|---|---|---|---|
| II-59 | $CH_2CH_2CH$ 3 | N | CH | CH | CH | $CH_3$ | trans | 2,042 / 379,9 $[M+H]^+$ |
| II-60 ** | $OCH_2CH_3$ | N | CH | $C-CH_2CH_3$ | CH | $CH_3$ | trans | 80 |
| II-61 ** | $OCH_2CH_3$ | N | $C^{2)}-N(CH_3)-N=C(CH_3)-C^{3)}$ | | CH | $CH_3$ | cis | 167 |
| II-62 | 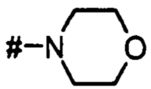 | N | CH | CH | CH | $CH_2CH_2CH_3$ | cis + trans | 47 |
| II-63 ** | $OCH_2CH_3$ | N | CH | $C^{3)}-CH_2CH_3$ | CH | $CH_3$ | cis | 127 |
| II-64 | CN | N | CH | CH | CH | H | cis | b) 3,730/349 $[M+H]^+$ |
| II-65 | OH | N | CH | CH | CH | $CH_3$ | cis | 2,130/354,15 $[M+H]^+$ |
| II-66 ** | $OCH_3$ | N | $C^{2)}-OCH_3$ | CH | CH | $CH_3$ | cis | 3,012 / 397,8 $[M+H]^+$ |
| II-67 | 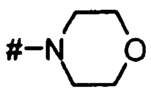 | N | CH | CH | CH | $CH_3$ | cis | 200 |
| II-68 ** | $OCH_2CH_2C\ H_3$ | N | $C^{2)}-OCH_2-CH_2CH_3$ | CH | CH | $CH_3$ | cis | 4,029 / 454,2 $[M+H]^+$ |
| II-69 ** | $OCH_3$ | N | $C^{2)}-C(CH_3)=CH-CH=C(CH_3)-C^{3)}$ | | CH | $CH_3$ | cis | 4,008 / 445,9 $[M+H]^+$ |
| 11-70 ** | $OCH_2CH_3$ | N | $C^{2)}-OC_2H_5$ | CH | CH | $CH_3$ | cis | 115-117 |
| II-71 ** | $OCH_3$ | N | $C^{2)}-CH=CH-C(OCH_3)=CH-C^{3)}$ | | CH | $CH_3$ | cis | 164-166 |
| II-72 | $OCH_2OCH$ 3 | N | CH | CH | CH | $CH_3$ | cis | 159-161 |
| II-73 | $OCH_3$ | N | CH | CH | CH | $CH_2CH_2CH_3$ | trans | 125 |
| II-74 | $OCH_3$ | N | CH | CH | CH | $CH_2CH_3$ | trans | 154 |

(fortgesetzt)

| Nr. | $R^a$ | V | W | X | Y | $R^1$ | Isomer | phys. Daten (Fp.[°C]; HPLC: RT [min]/m/z) |
|---|---|---|---|---|---|---|---|---|
| II-75 | $OCH_3$ | N | CH | CH | CH | $CH_2CH_3$ | cis | 150 |
| II-76 | $OCH_3$ | N | CH | CH | CH | $CH_2CH_2CH_3$ | cis | 180 |
| II-77 | $OSO_2CF_3$ | N | CH | CH | CH | $CH_3$ | cis | 3,264 / 485,7 $[M+H]^+$ |
| II-78 | $CF_3$ | N | CH | CH | CH | $CH_3$ | cis | 2,718 / 405,8 $[M+H]^+$ |
| II-79 | $CF_3$ | N | CH | CH | CH | $CH_3$ | trans | 2,775 / 405,8 $[M+H]^+$ |
| II-80 | $CH_3$ | N | CH | CH | CH | $CH_3$ | cis | 1,812/351,9 $[M+H]^+$ |
| II-81 | $CHF_2$ | N | CH | CH | CH | $CH_3$ | cis | 2,618 / 387,9 $[M+H]^+$ |
| II-82 | $OSO_2CF_3$ | N | CH | CH | CH | H | cis | 3,072 / 493,6 $[M+H]^+$ |
| II-83 | OH | N | CH | CH | CH | H | cis | 1,898/339,8 $[M+H]^+$ |
| II-84 | $(CH_2)_3CH_3$ | N | CH | CH | CH | H | cis | 2,159/380,2 $[M+H]^+$ |
| II-85 | $(CH_2)_3CH_3$ | N | CH | CH | CH | $CH_3$ | cis | 108 - 109 |
| II-86 | $(CH_2)_3CH_3$ | N | CH | CH | CH | $CH_2CH_2CH_3$ | cis | 2,688/421,9 $[M+H]^+$ |
| II-87 | $(CH_2)_3CH_3$ | N | CH | CH | CH | H | trans | 2,028 / 380,2 $[M+H]^+$ |
| II-88 | $(CH_2)_3CH_3$ | N | CH | CH | CH | $CH_3$ | trans | 108 -109 |
| II-89 | $OCH_2CHF_2$ | N | CH | CH | CH | $CH_3$ | cis | 2,926/417,8 $[M+H]^+$ |

(fortgesetzt)

| Nr. | $R^a$ | V | W | X | Y | $R^1$ | Isomer | phys. Daten (Fp.[°C]; HPLC: RT [min]/m/z) |
|---|---|---|---|---|---|---|---|---|
| II-90 | $OCH_2$-$CH_2OCH_3$ | N | CH | CH | CH | $CH_3$ | trans | 2,498 / 411,8 $[M+H]^+$ |
| II-91 | $OCH_2$-$CH_2OCH_3$ | N | CH | CH | CH | $CH_3$ | cis | 2,684 / 411,8 $[M+H]^+$ |
| II-92 | $OCH_2CH_2$ O-$CH_2CH_3$ | N | CH | CH | CH | $CH_3$ | cis | 2,846 / 425,8 $[M+H]^+$ |
| II-93 | $OCH_2CH_3$ | N | CH | CH | CH | $CH_2CHF_2$ | cis | 3,557/430,1 $[M+H]^+$ |
| II-94 | $OCH_2CH_3$ | N | CH | CH | CH | $CH_2CHF_2$ | trans | 3,401/430,1 $[M+H]^+$ |
| II-95 | $OCH_3$ | N | CH | CH | CH | $CH_2CH=CH_2$ | cis | 2,750/394,1 $[M+H]^+$ |

** kennzeichnet die nicht erfindungegemäßen Beispiele
# kennzeichnet die Bindung zu dem C-Atom, an welches $R^a$ gebunden ist
*) Diese Angabe bezieht sich auf die Stereochemie der Doppelbindung am Piperazingerüst. Bei den hergestellten Verbindungen handelt es sich jeweils um das Racemat.
1) kennzeichnet das Ring-Atom V 2) kennzeichnet das Ring-Atom W 3) kennzeichnet das Ring-Atom X
4) kennzeichnet das Ring-Atom Y

Tabelle III: Weitere Verbindungen der Formel I

| Nr. | Struktur | Isomer | phys. Daten (Fp.[°C]; HPLC: RT[min]/m/z) |
|---|---|---|---|
| III-1 | 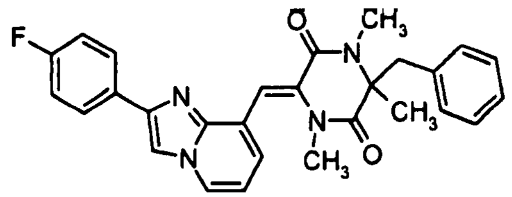 | Z | 2,677 / 468,7 [M+H]$^+$ |
| III-2 | 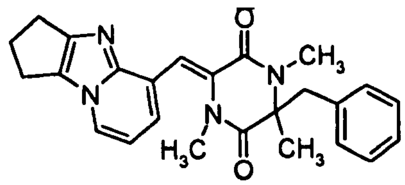 | Z | 2,272 / 414,8 [M+H]$^+$ |
| III-3 | 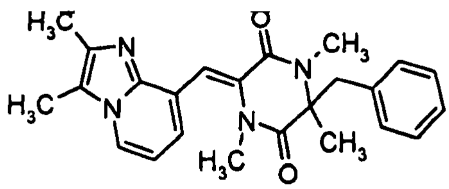 | Z | 2,276/ 402,8 [M+H]$^+$ |
| III-4 | 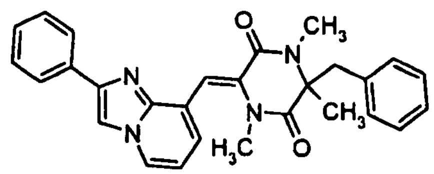 | Z | 2,579 / 450,8 [M+H]$^+$ |
| III-5 | 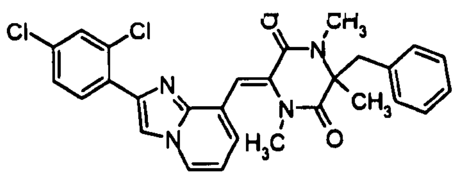 | Z | 3,007 / 520,6 [M+H]$^+$ |
| III-6 | 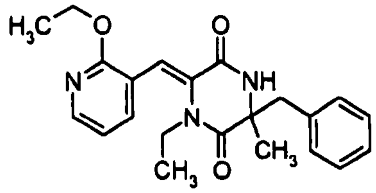 | Z | 148 |

Anwendungsbeispiele

Die herbizide Wirkung der Verbindungen der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst

als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Eine gute herbizide Aktivität ist bei Werten von wenigstens 70 und eine sehr gute herbizide Aktivität ist bei Werten von wenigstens 85 gegeben.

Die in den Gewächshausversuchen verwendeten Pflanzen setzten sich aus folgenden Arten zusammen:

| Bayercode | Lateinischer Name | Deutscher Name | Englischer Name |
|---|---|---|---|
| ABUTH | Abutilon theophrasti | Chinesischer Hanf | China jute |
| ALOMY | Alopecurus agrestis | Acker-Fuchsschwanz | black twitch |
| AMARE | Amaranthus retoflexus | Bogen-Fuchsschwanz | redroot pigweed |
| AVEFA | Avena fatua | Flughafer | spring wild-oat |
| APESV | Apera spica-venti | Gemeiner Windhalm | windgrass |
| BRAPL | Brachiaria plantaginea | - | alexandergrass |
| BROIN | Bromus inermis | Wehrlose Trespe | Awnless brome |
| CHEAL | Chenopodium album | Weisser Gänsefuss | White goosefoot |
| ECHCG | Echinochloa crus-galli | Hühnerhirse | barnyardgrass |
| LAMPU | Lamium purpureum | Taubnessel | deadnettle |
| LOLMU | Lolium multiflorum | Italienisches Raygras | Italian ryegrass |
| POLCO | Fallopia convolvulus | Windenknöterich | Bearbine |
| SETVI | Setaria viridis | Grüne Borstenhirse | green foxtail |
| SETFA | Setaria faberi | Fabers Borstenhirse | giant foxtail |
| VIOAR | Viola arvensis | Feld-Stiefmütterchen | Field pansy |

Beispiele 1-10: Einzelwirkstoffe

1) Die Wirkstoffe I-1, I-8, I-9, I-22, I-23, I-50, I-51, I-53, I-78, I-85 und I-132 zeigten bei einer Aufwandmenge von 1,0 kg/ha im Vorauflauf gegen ALOMY eine sehr gute herbizide Wirkung.
2) Die Wirkstoffe I-23, I-52 und I-85 zeigten bei einer Aufwandmenge von 1,0 kg/ha, sowie die Wirkstoffe I-404 bei einer Aufwandmenge von 0,5 kg/ha im Vorauflauf gegen AMARE eine sehr gute herbizide Wirkung.
3) Die Wirkstoffe I-1 und I-50 zeigten bei einer Aufwandmenge von 1,0 kg/ha im Nachauflauf gegen AMARE eine sehr gute herbizide Wirkung.
4) Die Wirkstoffe I-1, I-2, I-7, I-8, I-9, I-10, I-22, I-27, I-38, I-50, I-51, I-53, I-78 und 1-85, I-127, I-129, I-130, I-132 und I-133 zeigten bei einer Aufwandmenge von 1,0 kg/ha, sowie die Wirkstoffe I-5, I-36, I-203, I-324, I-419, I-427, I-447, I-463, I-487, I-489, I-497, I-512, I-521, I-526, II-10, II-24, II-38, II-49 und II-76 bei einer Aufwandmenge von 0,5 kg/ha im Vorauflauf gegen APESV eine sehr gute herbizide Wirkung.
5) Der Wirkstoff I-1 zeigte bei einer Aufwandmenge von 1,0 kg/ha im Nachauflauf gegen AVEFA eine sehr gute herbizide Wirkung.
6) Die Wirkstoffe I-1, I-2, I-3, I-7, I-8, I-9, I-10, I-22, I-27, I-38, I-50, I-52, I-85, I-90, I-127, I-129, I-130, I-132 und I-133 zeigten bei einer Aufwandmenge von 1,0 kg/ha, sowie die Wirkstoffe I-36, I-203, I-324, I-419, I-427, I-447, I-489, I-497, I-512, I-521, I-526, II-10, II-24, II-38, II-49 und II-76 bei einer Aufwandmenge von 0,5 kg/ha im Vorauflauf gegen ECHCG eine sehr gute und der Wirkstoff I-463 eine gute herbizide Wirkung.
7) Der Wirkstoff I-1 zeigte bei einer Aufwandmenge von 1,0 kg/ha im Nachauflauf gegen ECHCG eine sehr gute herbizide Wirkung.

8) Der Wirkstoff I-1 zeigte bei einer Aufwandmenge von 1,0 kg/ha im Nachauflauf gegen LOLMU eine sehr gute herbizide Wirkung.

9) Die Wirkstoffe I-1, I-2, I-3, I-7, 1-8, I-9, I-10, I-22, I-27, I-38, I-50, I-52, I-78, I-85, I-127, I-129, I-130, I-132 und 1-133 zeigten bei einer Aufwandmenge von 1,0 kg/ha, sowie die Wirkstoffe I-36, I-203, I-324, I-419, I-427, I-447, I-463, I-487, I-489, I-497, I-512, I-521, I-526, II-10, II-24, II-38, II-49 und II-76 bei einer Aufwandmenge von 0,5 kg/ha im Vorauflauf gegen SETFA eine sehr gute herbizide Wirkung.

10) Die Wirkstoffe I-1 und I-50 zeigten bei einer Aufwandmenge von 1,0 kg/ha im Nachauflauf gegen SETVI eine sehr gute herbizide Wirkung.

Beispiel 11: Vergleichsversuche gegenüber WO 2007/077247

Die vorteilhafte herbizide Wirkung der erfindungsgemäßen Wirkstoffe gegenüber den aus WO 2007/077247 bekannten Verbindungen konnte durch die folgenden Vergleichsversuche gezeigt werden:

Getestete Verbindungen:

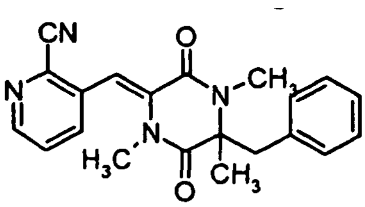

I-2

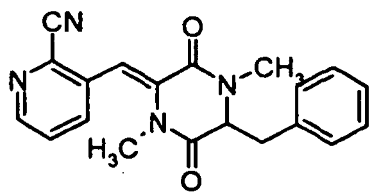

Bsp. 14 aus WO 2007/077247

a) Der Wirkstoff I-2 zeigte bei einer Aufwandmenge von 0,125 kg/ha im Vorauflauf gegen APESV 100% herbizide Wirkung, während die Verbindung Bsp. 14 nur 90% herbizide Wirkung zeigte.

b) Der Wirkstoff I-2 zeigte bei einer Aufwandmenge von 0,125 kg/ha im Vorauflauf gegen ALOMY 75% herbizide Wirkung, während die Verbindung Bsp. 14 keine herbizide Wirkung zeigte.

c) Der Wirkstoff I-2 zeigte bei einer Aufwandmenge von 0,125 kg/ha im Vorauflauf gegen ECHCG 80% herbizide Wirkung, während die Verbindung Bsp. 14 keine herbizide Wirkung zeigte.

Beispiele 12-16: Wirkstoffkombinationen

Die jeweils angegebenen Komponenten A und B wurde als 5 gew.%iges oder10 gew.%iges Emulsionskonzentrat formuliert oder es wurde eine handelsübliche Formulierung der Komponente B eingesetzt und unter Zugabe von derjenigen Menge an Lösungsmittelsystem in die Spritzbrühe eingesetzt, mit welcher der Wirkstoff ausgebracht wurde. Als Lösungsmittel diente in den Beispielen Wasser.

Die Versuchsperiode erstreckte sich über 20 bzw. 21 Tage. Während dieser Zeit wurden die Pflanzen gepflegt, wobei ihre Reaktionen auf die Wirkstoff- Behandlungen erfasst wurden.

Bei den folgenden Beispielen wurde nach der Methode von S. R. Colby (1967) "Calculating synergistic and antagonistic responses of herbicide combinations", Weeds 15, S. 22ff. derjenige Wert E errechnet, der bei einer nur additiven Wirkung der Einzelwirkstoffe zu erwarten ist.

$$E = X + Y - (X \cdot Y/100)$$

wobei

X = Prozentsatz Wirkung mit Wirkstoff A bei einer Aufwandmenge a;

Y = Prozentsatz Wirkung mit Wirkstoff B bei einer Aufwandmenge b;

E =zu erwartende Wirkung (in %) durch A + B bei Aufwandmengen a + b bedeuten.

Ist der experimentell gefundene Wert höher als der nach Colby errechnete Wert E, so liegt eine synergistische Wirkung vor.

Die Ergebnisse dieser Tests sind in den nachfolgenden Tabellen der Anwendungsbeispiele 11 bis 15 angegeben und belegen die synergistische Wirkung von Mischungen, die wenigstens eine Piperazindion-Verbindung der Formel I und wenigstens ein weiteres Herbizid enthalten.

Hierbei bedeutet a.S. = aktive Substanz, bezogen auf 100 % Wirkstoff. Die nach Colby errechneten Werte E

sind in Klammern () in den Anwendungsbeispielen 11 bis 15 angegeben.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

12) Synergistische herbizide Wirkung des Wirkstoffs I-2 mit Pendimethalin (Stomp®; SC, 400g/l) im Nachauflaufverfahren:

| Wirkstoff | Aufwandmenge a.S. in g/ha | Herbizide Wirkung in % nach 20 Tagen gegen | |
|---|---|---|---|
| | | BROIN | CH EAL |
| I-2 | 125 | 45 | 70 |
| Pendimethalin | 250 | 0 | 75 |
| I-2+ Pendimethalin | 125 + 250 | 65 (45) | 95 (93) |

13) Synergistische herbizide Wirkung des Wirkstoffs I-2 mit Pendimethalin (Stomp®; SC, 400g/l) im Vorauflaufverfahren:

| Wirkstoff | Aufwandmenge a.S. in g/ha | Herbizide Wirkung in % nach 20 Tagen gegen | | |
|---|---|---|---|---|
| | | APESV | BROIN | POLCO |
| I-2 | 125 | 95 | 80 | 80 |
| Pendimethalin | 250 | 95 | 40 | 70 |
| I-2 + Pendimethalin | 125 + 250 | 100 (100) | 95 (88) | 95 (94) |

14) Synergistische herbizide Wirkung des Wirkstoffs I-2 mit Saflufenacil (WG, 70%) im Vorauflaufverfahren:

| Wirkstoff | Aufwandmenge a.S. in g/ha | Herbizide Wirkung in % nach 20 Tagen gegen | | | |
|---|---|---|---|---|---|
| | | BRAPL | ECHCG | ABUTH | VIOAR |
| I-2 | 62,5 | 65 | 40 | 0 | 60 |
| Saflufenacil | 6,25 | 35 | 0 | 90 | 80 |
| I-2 + Saflufenacil | 62,5 + 6,25 | 80 (77) | 50 (40) | 100 (90) | 98 (92) |

15) Synergistische herbizide Wirkung des Wirkstoffs I-2 mit Pyroxasulfone (SC, 100 g/l) im Vorauflaufverfahren:

| Wirkstoff | Aufwandmenge a.S. in g/ha | Herbizide Wirkung in % nach 20 Tagen gegen | | | |
|---|---|---|---|---|---|
| | | ALOMY | SETVI | BROIN | LAMPU |
| I-2 | 62,5 | 65 | 90 | 20 | 40 |
| Pyroxasulfone | 12,5 | 90 | 90 | 65 | 80 |
| I-2 + Pyroxasulfone | 62,5 + 12,5 | 100 (97) | 100 (99) | 90 (72) | 100 (88) |

16) Synergistische herbizide Wirkung des Wirkstoffs I-2 mit Pyroxasulfone (SC, 100 g/l) im im Nachauflaufverfahren:

| Wirkstoff | Aufwandmenge a.S. in g/ha | Herbizide Wirkung in % nach 20 Tagen gegen | | |
|---|---|---|---|---|
| | | ALOMY | APESV | POLCO |
| I-2 | 62,5 | 45 | 40 | 40 |
| Pyroxasulfone | 12,5 | 85 | 80 | 40 |
| I-2+ Pyroxasulfone | 62,5 + 12,5 | 98 (92) | 95 (88) | 70 (64) |

## Patentansprüche

1. Piperazinverbindungen der Formel I.1

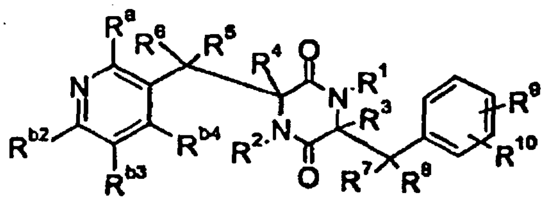

I.1

worin die Variablen folgende Bedeutung haben

$R^a$ Halogen, CN, $NO_2$, $C_1$-$C_4$-Alkyl, $C_3$-$C_6$-Cycloalkyl, $C_1$-$C_4$-Haloalkyl, $C_1$-$C_4$-Alkoxy, $C_1$-$C_4$-Thioalkyl, $C_1$-$C_4$-Haloalkoxy, $C_1$-$C_4$-Halogenthioalkyl, $S(O)_nR^y$, $C_2$-$C_6$-Alkenyl, $C_3$-$C_6$-Cycloalkenyl, $C_3$-$C_6$-Alkenyloxy, $C_3$-$C_6$-Thioalkenyl, $C_2$-$C_6$-Alkinyl, $C_3$-$C_6$-Akinyloxy, $C_3$-$C_6$-Thioalkinyl, $NR^AR^B$, Tri-$C_1$-$C_4$-alkylsilyl, Z-C(=O)-$R^{a1}$, Z-C(=S)-$R^{a1}$, Z-C(=N-$OR^A$)-$R^{a1}$, Z-C[=N(O)-$R^A$]-$R^{a1}$, Z-P(=O)$(R^{a1})_2$, über C oder N gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen $R^{aa}$ und/oder $R^{a1}$ substituiert und/oder an einen einen weiteren gesättigten, ungesättigten oder aromatischen carbo- oder heterocyclischen Ring anneliert sein kann,

$R^y$ $C_1$-$C_4$-Alkyl und $C_1$-$C_4$-Haloalkyl bedeutet und n für 0, 1 oder 2 steht;

$R^A$, $R^B$ unabhängig voneinander Wasserstoff, $C_1$-$C_6$-Akyl, $C_3$-$C_6$-Alkenyl, $C_3$-$C_6$-Alkinyl, $C_3$-$C_6$-Cycloalkyl, $C_1$-$C_6$-Alkylcarbonyl, $C_3$-$C_6$-Cycloalkylcarbonyl, $C_3$-$C_6$-Alkenylcarbonyl, $C_3$-$C_6$-Cycloalkenyl-carbonyl und $C_3$-$C_6$-Alkinylcarbonyl;

Z eine kovalente Bindung oder $C_1$-$C_4$-Alkylen;

$R^{a1}$ Wasserstoff, OH, $C_1$-$C_8$-Alkyl, $C_1$-$C_4$-Haloalkyl, $C_3$-$C_6$-Cycloalkyl, $C_2$-$C_8$-Alkenyl, $C_5$-$C_6$-Cycloalkenyl, $C_2$-$C_8$-Alkinyl, $C_1$-$C_6$-Alkoxy, $C_1$-$C_4$-Haloalkoxy, $C_3$-$C_8$-Alkenyloxy, $C_3$-$C_8$-Alkinyloxy, $NH_2$, $C_1$-$C_6$-Alkylamino, [Di-($C_1$-$C_6$)-alkyl]amino, $C_1$-$C_5$-Alkoxyamino, $C_1$-$C_6$-Alkylsulfonylamino, $C_1$-$C_6$-Alkylaminosulfonylamino, [Di-($C_1$-$C_6$)-alkylamino] sulfonylamino, $C_3$-$C_6$-Alkenylamino, $C_3$-$C_6$-Alkinylamino, N-($C_2$-$C_6$-Alkenyl)-N-($C_1$-$C_6$-alkyl)-amino, N-($C_2$-$C_6$-Alkinyl)-N-($C_1$-$C_6$-alkyl)-amino, N-($C_1$-$C_6$-Alkoxy)-N-($C_1$-$C_6$-alkyl)-amino, N-(C2-$C_6$-Alkenyl)-N-($C_1$-$C_6$-alkoxy)-amino, N-($C_2$-$C_9$-Alkinyl)-N-($C_1$-$C_6$-alkoxy)-amino, $C_1$-$C_6$-Alkylsulfonyl, Tri-$C_1$-$C_4$-alkylsilyl, Phenyl, Phenoxy, Phenylamino und 5- oder 6-gliedriger monocyclischer oder oder 10-gliedriger bicyclischer Heterocyclus, enthaltend 1,2,3 oder 4 Heteroatome ausgewählt aus O, N und S, wobei die cyclischen Gruppen unsubstituiert oder durch 1, 2, 3 oder 4 Gruppen $R^{aa}$ substituiert sind, bedeutet;

$R^{aa}$ Halogen, CN, $NO_2$, $C_1$-$C_4$-Alkyl, $C_1$-$C_4$-Haloalkyl, $C_1$-$C_4$-Alkoxy, $C_1$-$C_4$-Haloalkoxy, Z-C(=O)-$R^{a1}$, Z-C(=S)-$R^{a1}$, Z-C(=N-$OR^A$)-$R^{a1}$, Z-C(=N(O)-$R^A$]-$R^{a1}$, Oxo (=O) und Tri-$C_1$-$C_4$-alkylsilyl:

wobei in Gruppen $R^a$ und deren Untersubstituenten die Kohlenstoffketten und/oder die cyclischen Gruppen 1,2,3 oder 4 Substituenten $R^{aa}$ und/oder $R^{a1}$ tragen können;

$R^{b2}$, $R^{b3}$ und $R^{b4}$ jeweils für Wasserstoff stehen;

$R^1$ Wasserstoff, $C_1$-$C_6$-Alkyl, $C_1$-$C_4$-Haloalkyl, $C_3$-$C_4$-Alkenyl, $C_3$-$C_4$-Alkinyl und C(=O)$R^{11}$,

$R^{11}$ Wasserstoff, $C_1$-$C_4$-Alkyl, $C_1$-$C_4$-Haloalkyl, $C_1$-$C_4$-Alkoxy und $C_1$-$C_4$-Haloalkoxy;

$R^2$ $C_1$-$C_4$-Alkyl, $C_3$-$C_4$-Alkenyl und $C_3$-$C_4$-Alkinyl;

$R^3$ OH, $NH_2$, $C_1$-$C_4$-Alkyl, $C_3$-$C_6$-Cycloalkyl, $C_3$-$C_6$-Alkenyl, $C_3$-$C_6$-Alkinyl, $C_1$-$C_4$-Hydroxyalkyl, $C_1$-$C_4$-Cyanoalkyl, $C_1$-$C_4$-Haloalkyl, $C_1$-$C_4$-Alkoxy-$C_1$-$C_4$-alkyl und C(=O)$R^{11}$;

$R^4$ und $R^5$ gemeinsam für eine kovalente Bindung oder für Wasserstoff und $R^6$, $R^7$ und $R^8$ für Wasserstoff stehen

$R^9$,$R^{10}$ unabhängig voneinander Wasserstoff, Halogen, OH, Haloalkyl, $NR^AR^B$, $NR^AC(O)R^{91}$, CN, $NO_2$, $C_1$-$C_4$-Alkyl, $C_1$-$C_4$-Haloalkyl, $C_2$-$C_4$-Alkenyl, $C_3$-$C_6$-Alkinyl, $C_1$-$C_4$-Alkoxy, $C_1$-$C_4$-Haloalkoxy, O-C(O)$R^{91}$, Phenoxy und Benzyloxy, wobei in Gruppen $R^9$ und $R^{10}$ die Kohlenstoffketten und/oder die cyclischen Gruppen 1, 2, 3 oder 4 Substituenten $R^{aa}$ tragen können; $R^{91}$ $C_1$-$C_4$-Alkyl oder $NR^AR^B$;

sowie deren landwirtschaftlich geeignete Salze.

2. Verbindungen nach Anspruch 1 und deren landwirtschaftlich geeignete Salze, worin

$R^a$ Halogen, CN, $NO_2$, $C_1$-$C_4$-Alkyl, $C_3$-$C_6$-Cycloalkyl, $C_1$-$C_4$-Haloalkyl, $C_1$-$C_4$-Alkoxy, $C_1$-$C_4$-Haloalkoxy, $S(O)_nR^y$, $C_2$-$C_6$-Alkenyl, $C_3$-$C_6$-Cycloalkenyl, $C_3$-$C_6$-Alkenyloxy, $C_2$-$C_6$-Alkinyl, $C_3$-$C_8$-Alkinyloxy, $NR^AR^B$, Tri-$C_1$-$C_4$-alkylsilyl, Z-C(=O)-$R^{a1}$, Z-P(=O)$(R^{a1})_2$, über C oder N gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen $R^{aa}$ und/oder $R^{a1}$ substituiert sein kann,

$R^A$,$R^B$ unabhängig voneinander Wasserstoff und $C_1$-$C_6$-Akyl, $C_3$-$C_6$-Alkenyl und $C_3$-$C_6$-Alkinyl;
$R^{aa}$ Halogen, CN, $NO_2$, $C_1$-$C_4$-Alkyl, $C_1$-$C_4$-Haloalkyl, $C_1$-$C_4$-Alkoxy, $C_1$-$C_4$-Haloalkoxy, Z-C(=O)-$R^{a1}$ und Tri-$C_1$-$C_4$-alkylsilyl;
$R^1$ Wasserstoff, $C_1$-$C_6$-Alkyl, $C_3$-$C_4$-Alkenyl, $C_3$-$C_4$-Alkinyl und C(=O)$R^{11}$.

3. Verbindungen nach Anspruch 1 oder 2, worin $R^a$ für Halogen, Cyano oder Nitro steht.

4. Verbindungen nach Anspruch 1 oder 2, worin $R^a$ für $C_1$-$C_4$-Alky, $C_1$-$C_4$-Alkoxy, $C_1$-$C_4$-Haloalkyl, $C_1$-$C_4$-Haloalkoxy, $S(O)_nR^y$, $C_1$-$C_4$-Haloalkylthio oder ein über N gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen $R^{aa}$ und/oder $R^{a1}$ substituiert sein kann.

5. Verbindungen nach Anspruch 1 oder 2, worin $R^a$ für Chlor, Cyano, itro, $C_1$-$C_4$-Alkoxy, $C_1$-$C_4$-Thioalkyl, Trifluormethyl, oder über N gebundener ggf. subst. 6-gliedriger gesättigter Heterocyclus, enthaltend 1 oder 2 Heteroatome ausgewählt aus O und N, steht.

6. Verbindungen nach einem der Ansprüche 1 bis 5, worin $R^4$ und $R^5$ gemeinsam für eine kovalente Bindung stehen.

7. Verbindungen nach Anspruch 6, wobei die exo-Doppelbindung am Piperazinring die (Z)-Konfiguration aufweist.

8. Verbindungen nach einem der vorhergehenden Ansprüche, worin $R^1$ für Wasserstoff, Methyl, Ethyl, Propyl, Butyl, Allyl oder Propargyl steht.

9. Verbindungen nach einem der vorhergehenden Ansprüche, worin $R^2$ für Methyl oder Ethyl steht.

10. Verbindungen nach einem der vorhergehenden Ansprüche, worin $R^3$ für Methyl oder Ethyl steht.

11. Mittel, enthaltend eine herbizid wirksame Menge mindestens einer Piperazinverbindung oder eines landwirtschaftlich geeigneten Salzes davon nach einem der Ansprüche 1 bis 10 und für die Formulierung von Pflanzenschutzmitteln übliche Hilfsmittel.

12. Mittel gemäß Anspruch 11, enthaltend mindestens einen weiteren Wirkstoff.

13. Verfahren zur Bekämpfung von unerwünschtem Pflanzenwuchs, **dadurch gekennzeichnet, dass** man eine herbizid wirksame Menge mindestens einer Piperazinverbindung oder eines landwirtschaftlich brauchbaren Salzes davon nach einem der Ansprüche 1 bis 10 auf Pflanzen, deren Samen und/oder deren Lebensraum einwirken lässt.

## Claims

1. A piperazine compound of the formula I.1

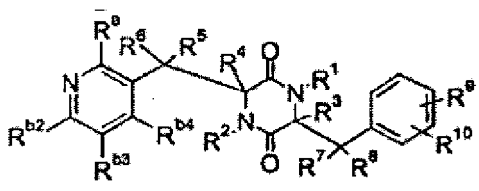

I.1

in which the variables are as defined below:

$R^a$ is halogen, CN, $NO_2$, $C_1$-$C_4$-alkyl, $C_3$-$C_6$-cycloalkyl, $C_1$-$C_4$-haloalkyl, $C_1$-$C_4$-alkoxy, $C_1$-$C_4$-thioalkyl, $C_1$-$C_4$-haloalkoxy, $C_1$-$C_4$-halothioalkyl, $S(O)_nR^y$, $C_2$-$C_6$-alkenyl, $C_3$-$C_6$-cycloalkenyl, $C_3$-$C_6$-alkenyloxy, $C_3$-$C_6$-thioalkenyl, $C_2$-$C_6$-alkynyl, $C_3$-$C_6$-alkynyloxy, $C_3$-$C_6$-thioalkynyl, $NR^AR^B$, tri-$C_1$-$C_4$-alkylsilyl, Z-C(=O)-$R^{a1}$, Z-C(=S)-$R^{a1}$, Z-C(=N-$OR^A$)-$R^{a1}$, Z-C[=N(O)-$R^A$]-$R^{a1}$, Z-P(=O)$(R^{a1})_2$, a 3- to 7-membered monocyclic or 9-or 10-membered bicyclic saturated, unsaturated or aromatic heterocycle which is attached via carbon or nitrogen, which contains 1, 2, 3 or 4 heteroatoms selected from the group consisting of O, N and S and which

may be partially or fully substituted by groups $R^{aa}$ and/or $R^{a1}$ and/or fused to a further saturated, unsaturated or aromatic carbo- or heterocyclic ring,

$R^y$ is $C_1$-$C_4$-alkyl or $C_1$-$C_4$-haloalkyl and n is 0, 1 or 2;

$R^A$, $R^B$ independently of one another are hydrogen, $C_1$-$C_6$-alkyl, $C_3$-$C_6$-alkenyl, $C_3$-$C_6$-alkynyl, $C_3$-$C_6$-cycloalkyl, $C_1$-$C_6$-alkylcarbonyl, $C_3$-$C_6$-cycloalkylcarbonyl, $C_3$-$C_6$-alkenylcarbonyl, $C_3$-$C_6$-cycloalkenylcarbonyl or $C_3$-$C_6$-alkynylcarbonyl;

Z is a covalent bond or $C_1$-$C_4$-alkylene;

$R^{a1}$ is hydrogen, OH, $C_1$-$C_8$-alkyl, $C_1$-$C_4$-haloalkyl, $C_3$-$C_6$-cycloalkyl, $C_2$-$C_8$-alkenyl, $C_5$-$C_6$-cycloalkenyl, $C_2$-$C_8$-alkynyl, $C_1$-$C_6$-alkoxy, $C_1$-$C_4$-haloalkoxy, $C_3$-$C_8$-alkenyloxy, $C_3$-$C_8$-alkynyloxy, $NH_2$, $C_1$-$C_6$-alkylamino, [di-($C_1$-$C_6$)-alkyl]amino, $C_1$-$C_6$-alkoxyamino, $C_1$-$C_6$-alkylsulfonylamino, $C_1$-$C_6$-alkylaminosulfonylamino, [di-($C_1$-$C_6$)-alkylamino]sulfonylamino, $C_3$-$C_6$-alkenylamino, $C_3$-$C_6$-alkynylamino, N-($C_2$-$C_6$-alkenyl)-N-($C_1$-$C_6$-alkyl)amino, N-($C_2$-$C_6$-alkynyl)-N-($C_1$-$C_6$-alkyl)amino, N-($C_1$-$C_6$-alkoxy)-N-($C_1$-$C_6$-alkyl)amino, N-($C_2$-$C_6$-alkenyl)-N-($C_1$-$C_6$-alkoxy)amino, N-($C_2$-$C_6$-alkynyl)-N-($C_1$-$C_6$-alkoxy)amino, $C_1$-$C_6$-alkylsulfonyl, tri-$C_1$-$C_4$-alkylsilyl, phenyl, phenoxy, phenylamino or a 5- or 6-membered monocyclic or 9- or 10-membered bicyclic heterocycle which contains 1, 2, 3 or 4 heteroatoms selected from the group consisting of O, N and S, where the cyclic groups are unsubstituted or substituted by 1, 2, 3 or 4 groups $R^{aa}$;

$R^{aa}$ is halogen, CN, $NO_2$, $C_1$-$C_4$-alkyl, $C_1$-$C_4$-haloalkyl, $C_1$-$C_4$-alkoxy, $C_1$-$C_4$-haloalkoxy, Z-C(=O)-$R^{a1}$, Z-C(=S)-$R^{a1}$, Z-C(=N-$OR^A$)-$R^{a1}$, Z-C[=N(O)-$R^A$]-$R^{a1}$, oxo(=O), and tri-$C_1$-$C_4$-alkylsilyl;

where in groups $R^a$ and their subsubstituents the carbon chains and/or the cyclic groups may carry 1, 2, 3 or 4 substituents $R^{aa}$ and/or $R^{a1}$;

$R^{b2}$, $R^{b3}$ and $R^{b4}$ are each hydrogen;

$R^1$ is hydrogen, $C_1$-$C_6$-alkyl, $C_1$-$C_4$-haloalkyl, $C_3$-$C_4$-alkenyl, $C_3$-$C_4$-alkynyl or C(=O)$R^{11}$,

$R^{11}$ is hydrogen, $C_1$-$C_4$-alkyl, $C_1$-$C_4$-haloalkyl, $C_1$-$C_4$-alkoxy or $C_1$-$C_4$-haloalkoxy;

$R^2$ is $C_1$-$C_4$-alkyl, $C_3$-$C_4$-alkenyl or $C_3$-$C_4$-alkynyl;

$R^3$ is OH, $NH_2$, $C_1$-$C_4$-alkyl, $C_3$-$C_6$-cycloalkyl, $C_3$-$C_6$-alkenyl, $C_3$-$C_6$-alkynyl, $C_1$-$C_4$-hydroxyalkyl, $C_1$-$C_4$-cyanoalkyl, $C_1$-$C_4$-haloalkyl, $C_1$-$C_4$-alkoxy-$C_1$-$C_4$-alkyl or C(=O)$R^{11}$;

$R^4$ and $R^5$ together are a covalent bond or are hydrogen and $R^6$, $R^7$ and $R^8$ are hydrogen

$R^9$,$R^{10}$ independently of one another are hydrogen, halogen, OH, haloalkyl, $NR^AR^B$, $NR^AC(O)R^{91}$, CN, $NO_2$, $C_1$-$C_4$-alkyl, $C_1$-$C_4$-haloalkyl, $C_2$-$C_4$-alkenyl, $C_3$-$C_6$-alkynyl, $C_1$-$C_4$-alkoxy, $C_1$-$C_4$-haloalkoxy, O-C(O)$R^{91}$, phenoxy or benzyloxy, where in groups $R^9$ and $R^{10}$ the carbon chains and/or the cyclic groups may carry 1, 2, 3 or 4 substituents $R^{aa}$;

$R^{91}$ is $C_1$-$C_4$-alkyl or $NR^AR^B$;

and the agriculturally suitable salts thereof.

**2.** The compound according to claim 1 or an agriculturally suitable salt thereof, in which

$R^a$ is halogen, CN, N02, C1-C4-alkyl, C3-C6-cycloalkyl, C1-C4-haloalkyl, C1-C4-alkoxy, C1-C4-haloalkoxy, S(O)nRy, C2-C6-alkenyl, C3-C6-cycloalkenyl, C3-C6-alkenyloxy, C2-C6-alkynyl, C3-C6-alkynyloxy, NRARB, tri-C1-C4-alkylsilyl, Z-C(=O)-Ra1, Z-P(=O)(Ra1)2, a 3- to 7-membered monocyclic or 9- or 10-membered bicyclic saturated, unsaturated or aromatic heterocycle which is attached via carbon or nitrogen, which contains 1, 2, 3 or 4 heteroatoms selected from the group consisting of O, N and S and which may be partially or fully substituted by groups Raa and/or Ra1,

$R^A$, $R^B$ independently of one another are hydrogen, C1-C6-alkyl, C3-C6-alkenyl or C3-C6-alkynyl;

$R^{aa}$ is halogen, CN, N02, C1-C4-alkyl, C1-C4-haloalkyl, C1-C4-alkoxy, C1-C4-haloalkoxy, Z-C(=O)-Ra1 or tri-C1-C4-alkylsilyl;

$R^1$ is hydrogen, C1-C6-alkyl, C3-C4-alkenyl, C3-C4-alkynyl or C(=O)R11.

**3.** The compound according to claim 1 or 2 in which $R^a$ is halogen, cyano or nitro.

**4.** The compound according to claim 1 or 2 in which Ra is C1-C4-alkyl, C1-C4-alkoxy, C1-C4-haloalkyl, C1-C4-haloalkoxy, S(O)nRy, C1-C4-haloalkylthio or a 3- to 7-membered monocyclic or 9- or 10-membered bicyclic saturated, unsaturated or aromatic heterocycle which is attached via nitrogen, which contains 1, 2, 3 or 4 heteroatoms selected from the group consisting of O, N and S and which may be partially or fully substituted by groups Raa and/or Ra1.

**5.** The compound according to claim 1 or 2 in which Ra is chlorine, cyano, nitro, C1-C4-alkoxy, C1-C4-thioalkyl, trifluoromethyl or an optionally substituted 6-membered saturated heterocycle which is attached via nitrogen and contains 1 or 2 heteroatoms selected from the group consisting of O and N.

6. The compound according to any of claims 1 to 5 in which R4 and R5 together are a covalent bond.

7. The compound according to claim 6 where the exo double bond at the piperazine ring has the (Z) configuration.

8. The compound according to any of the preceding claims in which R1 is hydrogen, methyl, ethyl, propyl, butyl, allyl or propargyl.

9. The compound according to any of the preceding claims in which R2 is methyl or ethyl.

10. The compound according to any of the preceding claims in which R3 is methyl or ethyl.

11. A composition comprising a herbicidally effective amount of at least one piperazine compound or an agriculturally suitable salt thereof according to any of claims 1 to 10 and auxiliaries customary for formulating crop protection agents.

12. The composition according to claim 11 which comprises at least one further active compound.

13. A method for controlling unwanted vegetation which comprises allowing a herbicidally effective amount of at least one piperazine compound or of an agriculturally suitable salt thereof according to any of claims 1 to 10 to act on plants, their seed and/or their habitat.

**Revendications**

1. Composés de type pipérazine de formule I.1

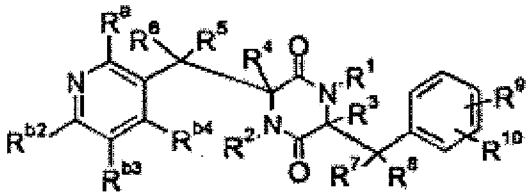

I.1

dans laquelle les variables ont les significations suivantes
$R^a$ représente un atome d'halogène, CN, $NO_2$, un groupe alkyle en $C_1$-$C_4$, cycloalkyle en $C_3$-$C_6$, halogénoalkyle en $C_1$-$C_4$, alcoxy en $C_1$-$C_4$, thioalkyle en $C_1$-$C_4$, halogénoalcoxy en $C_1$-$C_4$, halogénothioalkyle en $C_1$-$C_4$, $S(O)_nR^y$, alcényle en $C_2$-$C_6$, cycloalcényle en $C_3$-$C_6$, alcényloxy en $C_3$-$C_6$, thioalcényle en $C_3$-$C_6$, alcynyle en $C_2$-$C_6$, alcynyloxy en $C_3$-$C_6$, thioalcynyle en $C_3$-$C_6$, $NR^AR^B$, trialkyl ($C_1$-$C_4$) silyle, Z-C (=O) -$R^{a1}$, Z-C(=S)-$R^{a1}$, Z-C(=N-OR)-$R^{a1}$, Z-C [=N(O)-$R^A$]-$R^{a1}$, Z-P(=O)$(R^{a1})_2$, un hétérocycle monocyclique à 3-7 chaînons ou bicyclique à 9 ou 10 chaînons, saturé, insaturé ou aromatique, lié par C ou N, contenant 1, 2, 3 ou 4 hétéroatomes choisis parmi O, N et S, qui peut être partiellement ou totalement substitué par des groupes $R^{aa}$ et/ou $R^{a1}$ et/ou soudé à un autre cycle carbocyclique ou hétérocyclique saturé, insaturé ou aromatique,
$R^y$ représente des groupes alkyle en $C_1$-$C_4$ et halogénoalkyle en $C_1$-$C_4$ et n représente 0, 1 ou 2 ;
$R^A$, $R^B$ représentent indépendamment l'un de l'autre un atome d'hydrogène, un groupe alkyle en $C_1$-$C_6$, alcényle en $C_3$-$C_6$, alcynyle en $C_3$-$C_6$, cycloalkyle en $C_3$-$C_6$, alkyl($C_1$-$C_6$)-carbonyle, cycloalkyl($C_3$-$C_6$)carbonyle, alcényl ($C_3$-$C_6$) carbonyle, cycloalcényl ($C_3$-$C_6$) - carbonyle et alcynyl($C_3$-$C_6$)carbonyle ;
Z représente une liaison covalente ou un groupe alkylène en $C_1$-$C_4$ ;
$R^{a1}$ représente un atome d'hydrogène, OH, un groupe alkyle en $C_1$-$C_8$, halogénoalkyle en $C_1$-$C_4$, cycloalkyle en $C_3$-$C_6$, alcényle en $C_2$-$C_8$, cycloalcényle en $C_5$-$C_6$, alcynyle en $C_2$-$C_8$, alcoxy en $C_1$-$C_6$, halogénoalcoxy en $C_1$-$C_4$, alcényloxy en $C_3$-$C_8$, alcynyloxy en $C_3$-$C_8$, $NH_2$, alkyl ($C_1$-$C_6$) amino, [dialkyl($C_1$-$C_6$)]amino, alcoxy($C_1$-$C_6$)amino, alkyl($C_1$-$C_6$sulfonylamino, alkyl ($C_1$-$C_6$) amino-sulfonylamino, [dialkyl($C_1$-$C_6$)amino]-sulfonylamino, alcényl ($C_3$-$C_6$) amino, alcynyl ($C_3$-$C_6$) amino, N-[alcényl($C_2$-$C_6$)]-N-[alkyl($C_1$-$C_6$) amino, N-[alcynyl($C_2$-$C_6$)]-N-[alkyl($C_1$-$C_6$)]amino, N-[alcoxy($C_1$-$C_6$)]-N-[alkyl ($C_1$-$C_6$)]amino, N-[alcényl($C_2$-$C_6$)]-N-[alcoxy ($C_1$-$C_6$)]amino, N- [alcynyl ($C_2$-$C_6$) -N-alcoxy ($C_1$-$C_6$)]amino, alkyl ($C_1$-$C_6$) sulfonyle, trialkyl($C_1$-$C_4$)silyle, phényle, phénoxy, phénylamino et un hétérocycle monocyclique à 5 ou 6 chaînons ou bicyclique à 9 ou 10 chaînons, contenant 1, 2, 3 ou 4 hétéroatomes choisis parmi O, N et S, les groupes cycliques étant non substitués ou substitués par 1, 2, 3 ou 4 groupes $R^{aa}$ ;
$R^{aa}$ représente un atome d'halogène, CN, $NO_2$, un groupe alkyle en $C_1$-$C_4$, halogénoalkyle en $C_1$-$C_4$, alcoxy en $C_1$-$C_4$, halogénoalcoxy en $C_1$-$C_4$, Z-C(=O)-$R^{a1}$, Z-C(=S)-$R^{a1}$, Z-C(=N-$OR^A$)-$R^{a1}$, Z-C[=N(O)-$R^A$) -$R^{a1}$, Oxo (=O) et

trialkyl($C_1$-$C_4$)silyle ;
dans les groupes $R^a$ et leurs substituants secondaires les chaînes carbonées et/ou les groupes cycliques pouvant porter 1, 2, 3 ou 4 substituants $R^{aa}$ et/ou $R^{a1}$ ;
$R^{b2}$, $R^{b3}$ et $R^{b4}$ représentent chacun un atome d'hydrogène ;
$R^1$ représente un atome d'hydrogène, un groupe alkyle en $C_1$-$C_6$, halogénoalkyle en $C_1$-$C_4$, alcényle en $C_3$-$C_4$, alcynyle en $C_3$-$C_4$ et C(=O)$R^{11}$,
$R^{11}$ représente un atome d'hydrogène, un groupe alkyle en $C_1$-$C_4$, halogénoalkyle en $C_1$-$C_4$, alcoxy en $C_1$-$C_4$ et halogénoalcoxy en $C_1$-$C_4$ ;
$R^2$ représente un groupe alkyle en $C_1$-$C_4$, alcényle en $C_3$-$C_4$ et alcynyle en $C_3$-$C_4$ ;
$R^3$ représente OH, $NH_2$, un groupe alkyle en $C_1$-$C_4$, cycloalkyle en $C_3$-$C_6$, alcényle en $C_3$-$C_6$, alcynyle en $C_3$-$C_6$, hydroxyalkyle en $C_1$-$C_4$, cyanoalkyle($C_1$-$C_4$) halogénoalkyle en $C_1$-$C_4$, alcoxy($C_1$-$C_4$)-alkyle ($C_1$-$C_4$) et C(=O)$R^{11}$ ;
$R^4$ et $R^5$ représentent ensemble une liaison covalente ou un atome d'hydrogène et $R^6$, $R^7$ et R $^8$ représentent des atomes d'hydrogène
$R^9$ et $R^{10}$ représentent indépendamment l'un de l'autre un atome d'hydrogène ou d'halogène, OH, un groupe halogénoalkyle, $NR^AR^B$, $NR^AC(O)R^{91}$, CN, $NO_2$, alkyle en $C_1$-$C_4$, halogénoalkyle en $C_1$-$C_4$, alcényle en $C_2$-$C_4$, alcynyle en $C_3$-$C_6$, alcoxy en $C_1$-$C_4$, halogénoalcoxy en $C_1$-$C_4$, O-C(O)$R^{91}$, phénoxy et benzyloxy, dans les groupes $R^9$ et $R^{10}$ les chaînes carbonées et/ou les groupes cycliques pouvant porter 1, 2, 3 ou 4 substituants $R^{aa}$ ;
$R^{91}$ représente un groupe alkyle en $C_1$-$C_4$ ou $NR^AR^B$ ;
ainsi que leurs sels appropriés en agriculture.

2. Composés selon la revendication 1 et leurs sels appropriés en agriculture, dans lesquels
$R^a$ représente un atome d'halogène, CN, $NO_2$, un groupe alkyle en $C_1$-$C_4$, cycloalkyle en $C_3$-$C_6$, halogénoalkyle en $C_1$-$C_4$, alcoxy en $C_1$-$C_4$, halogénoalcoxy en $C_1$-$C_4$, $S(O)_nR^y$, alcényle en $C_2$-$C_6$, cycloalcényle en $C_3$-$C_6$, alcényloxy en $C_3$-$C_6$, alcynyle en $C_2$-$C_6$, alcynyloxy en $C_3$-$C_6$, $NR^AR^B$, trialkyl($C_1$-$C_4$)silyle, Z-C(=O)-$R^{a1}$, Z-P(=O)$(R^{a1})_2$, un hétérocycle monocyclique à 3-7 chaînons ou bicyclique à 9 ou 10 chaînons, saturé, insaturé ou aromatique, lié par C ou N, contenant 1, 2, 3 ou 4 hétéroatomes choisis parmi 0, N et S, qui peut être partiellement ou totalement substitué par des groupes $R^{aa}$ et/ou $R^{a1}$,
$R^A$, $R^B$ représentent indépendamment l'un de l'autre un atome d'hydrogène et un groupe alkyle en $C_1$-$C_6$, alcényle en $C_3$-$C_6$ et alcynyle en $C_3$-$C_6$ ;
$R^{aa}$ représente un atome d'halogène, CN, $NO_2$, un groupe alkyle en $C_1$-$C_4$, halogénoalkyle en $C_1$-$C_4$, alcoxy en $C_1$-$C_4$, halogénoalcoxy en $C_1$-$C_4$, Z-C(=O)-$R^{a1}$ et trialkyl($C_1$-$C_4$)silyle ;
$R^2$ représente un atome d'hydrogène, un groupe alkyle en $C_1$-$C_6$, alcényle en $C_3$-$C_4$, alcynyle en $C_3$-$C_4$ et C(=O)$R^{11}$.

3. Composés selon la revendication 1 ou 2, dans lesquels $R^a$ représente un atome d'halogène, le groupe cyano ou nitro.

4. Composés selon la revendication 1 ou 2, dans lesquels $R^a$ représente un groupe alkyle en $C_1$-$C_4$, alcoxy en $C_1$-$C_4$, halogénoalkyle en $C_1$-$C_4$, halogénoalcoxy en $C_1$-$C_4$, $S(O)_nR^y$, halogénoalkyl($C_1$-$C_4$)thio ou un hétérocycle monocyclique à 3-7 chaînons ou bicyclique à 9 ou 10 chaînons, saturé, insaturé ou aromatique, lié par N, contenant 1, 2, 3 ou 4 hétéroatomes choisis parmi 0, N et S, qui peut être partiellement ou totalement substitué par des groupes $R^{aa}$ et/ou $R^{a1}$.

5. Composés selon la revendication 1 ou 2, dans lesquels $R^a$ représente un atome de chlore, un groupe cyano, nitro, alcoxy en $C_1$-$C_4$, thioalkyle en $C_1$-$C_4$, trifluorométhyle ou un hétérocycle saturé à 6 chaînons, éventuellement substitué, lié par N, contenant 1 ou 2 hétéroatomes choisis parmi O et N.

6. Composés selon l'une quelconque des revendications 1 à 5, dans lesquels $R^4$ et $R^5$ représentent ensemble une liaison covalente.

7. Composés selon la revendication 6, dans lesquels la double liaison exo sur le cycle pipérazine présente la configuration (Z).

8. Composés selon l'une quelconque des revendications précédentes, dans lesquels $R^1$ représente un atome d'hydrogène, le groupe méthyle, éthyle, propyle, butyle, allyle ou propargyle.

9. Composés selon l'une quelconque des revendications précédentes, dans lesquels $R^2$ représente le groupe méthyle ou éthyle.

10. Composés selon l'une quelconque des revendications précédentes, dans lesquels $R^3$ représente le groupe méthyle

ou éthyle.

11. Composition, contenant une quantité à activité herbicide d'au moins un composé de type pipérazine ou d'un sel approprié en agriculture d'un tel composé, selon l'une quelconque des revendications 1 à 10, et des adjuvants usuels pour la formulation de produits phytosanitaires.

12. Composition selon la revendication 11, contenant au moins une autre substance active.

13. Procédé pour la lutte contre la croissance indésirable de plantes, **caractérisé en ce qu'**on fait agir sur des plantes, leurs semences et/ou leur habitat une quantité à activité herbicide d'au moins un composé de type pipérazine ou d'un sel approprié en agriculture d'un tel composé, selon l'une quelconque des revendications 1 à 10.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007077201 A
- WO 2007077247 A
- EP 257993 A
- US 5013659 A
- US 6222100 B
- WO 0182685 A
- WO 0026390 A
- WO 9741218 A
- WO 9802526 A
- WO 9802527 A
- WO 04106529 A
- WO 0520673 A
- WO 0314357 A
- WO 0313225 A
- WO 0314356 A
- WO 0416073 A
- WO 9200377 A
- EP 242236 A
- EP 242246 A
- US 5559024 A
- WO 2002015701 A
- EP 374753 A
- WO 93007278 A
- WO 9534656 A
- EP 427529 A
- EP 451878 A
- WO 03018810 A
- WO 03052073 A
- EP 0392225 A
- WO 9626202 A
- WO 9741116 A
- WO 9741117 A
- WO 9741118 A
- WO 0183459 A
- WO 2008074991 A
- EP 2008057329 W
- WO 2008152073 A


### In der Beschreibung aufgeführte Nicht-Patentliteratur


- **KING R. R. et al.** *J. Agric. Food Chem.,* 1992, vol. 40, 834-837
- **KING R. R. et al.** *J. Agric. Food Chem.,* 2001, vol. 49, 2298-2301
- **I.O. DONKOR et al.** *Bioorg. Med. Chem. Lett.,* 2001, vol. 11 (19), 2647-2649
- **B.B. SNIDER et al.** *Tetrahedron,* 2001, vol. 57 (16), 3301-3307
- **I. YASUHIRO et al.** *J. Am. Chem. Soc.,* 2002, vol. 124 (47), 14017-14019
- **M. FALOMI et al.** *Europ. J. Org. Chem.,* 2000, 1669-1675
- **J. TSUJI.** *Top. Organomet. Chem.,* 2005, 332
- **J. TSUJI.** *Organic Synthesis with Palladium Compounds,* 1980, 207
- *Accts. Chem. Res.,* 2008, vol. 41 (11), 1439-1564
- *Angew. Chem. Int. Ed. Engl.,* 2009, vol. 48, 4114-4133
- Organikum. Wiley, 2001, 404
- *Tetrahedron Lett.,* 2001, vol. 42, 7473
- *Org. Lett.,* 2003, vol. 5, 1785
- **G. PORZI et al.** *Tetrahedron,* 1998, vol. 9 (19), 3411-3420
- **C. I. HARDING et al.** *Tetrahedron,* 2004, vol. 60 (35), 7679-7692
- **GLENN L. STAHL et al.** *J. Org. Chem.,* 1978, vol. 43 (11), 2285-6
- **A. K. GHOSH et al.** *Org. Lett.,* 2001, vol. 3 (4), 635-638
- **WILFORD L. MENDELSON et al.** *Int. J.Peptide & Protein Research,* 1990, vol. 35 (3), 249-57
- **I. OJIMA et al.** *J. Am. Chem. Soc.,* 1987, vol. 109 (21), 6537-6538
- **J. M. MCLNTOSH et al.** *Tetrahedron,* 1992, vol. 48 (30), 6219-6224
- **M. FALORNI et al.** *Europ. J. Org. Chem.,* 2000, 1669-1675
- **T. KAWASAKI et al.** *Org. Lett.,* 2000, vol. 2 (19), 3027-3029
- *J. Org. Chem.,* 2000, vol. 65 (24), 8402-8405
- **B. HOCK ; C. FEDTKE ; R. R. SCHMIDT.** Herbizide. Georg Thieme Verlag, 1995
- Modern Crop Protection Compounds. Wiley VCH, 2007, vol. 1